# EUROPEAN PATENT SPECIFICATION

(11) **EP 3 610 036 B1**
(45) Date of publication and mention of the grant of the patent: **02.07.2025**
(21) Application number: 18784711.6
(22) Date of filing: 11.04.2018
(51) Int. Cl.: C12Q 1/6816

(54) **DETECTION CASCADES**
ERKENNUNGSKASKADEN
CASCADES DE DÉTECTION

(30) Priority: 11.04.2017 AU 2017901321
(43) Date of publication of application: 19.02.2020
(73) Proprietor: SpeeDx Pty Ltd, Eveleigh, NSW 2015 (AU)
(72) Inventor: TODD, Alison Velyian, Glebe, NSW 2037 (AU); HASICK, Nicole Jane, Randwick, NSW 2031 (AU)
(74) Representative: Boult Wade Tennant LLP
(86) International application number: PCT/AU2018/000052
(87) International publication number: WO 2018/187829

(56) References cited:
- WO-A1-96/27026
- WO-A1-96/27026
- WO-A2-2012/034130
- NICOLE HASICK ET AL: "Sensitive Detection of Nucleic Acids Using Subzyme Feedback Cascades", MOLECULES, vol. 25, no. 7, 10 April 2020 (2020-04-10), DE, pages 1755, XP055748254, ISSN: 1433-1373, DOI: 10.3390/molecules25071755
- GERASIMOVA, Y. ET AL.: "Nucleic acid detection using MNAzymes", CHEMISTRY & BIOLOGY, vol. 17, no. 2, 26 February 2010 (2010-02-26), pages 104 - 106, XP055540114, Retrieved from the Internet <URL:doi:10.1016/j.chembiol.2010.02.003>
- WANG, F. ET AL.: "From cascaded catalytic nucleic acids to enzyme-DNA nanostructures: controlling reactivity, sensing, logic operations, and assembly of complex structures", CHEMICAL REVIEWS, vol. 114, no. 5, 27 February 2014 (2014-02-27), pages 2881 - 2941, XP055540121, Retrieved from the Internet <URL:DOI:10.1021/cr400354z>

## Description

### Technical Field

The present invention relates to compositions and methods for the detection of target molecules, and the amplification of detectable signals generated by detection assays. More specifically, the present invention relates to methods utilizing catalytic nucleic acid enzymes to generate and/or amplify a signal indicative of the presence of target molecules (e.g. nucleic acids and proteins), and compositions for use in the methods.

### Background

A number of assays are currently available for the detection of target nucleic acids or other molecules in a sample. Most assays rely on the use of protein enzymes, whilst a few exploit nucleic acid enzymes which catalyse modifications to nucleic acids as discussed below.

### - Catalytic Nucleic Acid Enzymes

Catalytic nucleic acid enzymes are non-protein enzymes capable of modifying specific substrates. Catalytic nucleic acid enzymes include DNA molecules (also known in the art as a DNAzyme, deoxyribozyme, or DNA enzyme), RNA molecules (also known in the art as a ribozyme), and multi-component nucleic acid enzymes composed of multiple DNA and/or RNA molecules (also known in the art as an MNAzyme or PlexZyme). Catalytic nucleic acid enzymes can modify specific nucleic acid substrate sequences by, for example, cleavage or ligation. A unique class of catalytic nucleic acid enzymes (known in the art as a horseradish peroxidase-mimicking DNAzyme) can catalyse peroxidase reactions that convert specific chemical substrates into their oxidated products which can for example, produce a change in colour or emit a fluorescent or chemiluminescent signal.

DNAzymes and ribozymes, capable of cleaving or ligating RNA substrates, DNA substrates and/or chimeric DNA/RNA substrates, can generally only modify a target nucleic acid substrate that meets minimum sequence requirements. For example, the substrate should exhibit sufficient base pair complementarity to the substrate binding arms of the enzyme, and also needs a specific sequence at the site of catalytic modification. Examples of such sequence requirements at the catalytic cleavage site include the requirement for a purine:pyrmidine sequence for DNAzyme cleavage (10-23 type), the requirement for a dinucleotide junction (8-17 type) and the requirement for the sequence uridine:X where X can equal A, C or U but not G, for the hammerhead ribozymes. The 10-23 DNAzyme is a DNAzyme that is capable of cleaving nucleic acid substrates at specific RNA phosphodiester bonds. This DNAzyme has a catalytic domain of 15 deoxyribonucleotides flanked by two substrate-recognition domains (arms). The 8:17 DNAzyme differs from the 10:23 DNAzyme in both the catalytic core region and cleavage site preference. The 8-17 DNAzyme does not have any stringent substrate sequence requirements and is capable of cleaving nucleic acid substrates at all four types of 5' NG dinucleotide junctions (i.e 5' GG, AG, CG and UG). There is an overall hierarchy of reactivity between groups of related junctions that roughly follow the order 5' NG>NA>NC>NT where NG junctions are the most susceptible to cleavage and pyrimidine-pyrimidine junctions are the least susceptible. The 8-17 DNAyme has a catalytic domain of 14-15 deoxynucleotides and is characterized by a short intramolecular 3 base-pair stem-triloop and a 4-5 single stranded deoxunucleotide region.

Multi-component nucleic acid enzymes (MNAzymes, also known as PlexZymes) are another category of catalytic nucleic acid enzyme. These enzymes require an assembly facilitator (e.g. a target nucleic acid) for their assembly and catalytic activity. MNAzymes are composed of multiple part-enzymes, or partzymes, which self-assemble in the presence of one or more assembly facilitators and form catalytically active MNAzymes capable of modifying substrates. The partzymes have multiple domains including sensor arms which bind to the assembly facilitator (such as a target nucleic acid); substrate arms which bind the substrate, and partial catalytic core sequences which, upon assembly of multiple partzyme components, combine to provide a complete catalytic core. MNAzymes can be designed to recognize a broad range of assembly facilitators including, for example, different target nucleic acid sequences. In the presence of the assembly facilitator, a catalytically active MNAzyme can assemble from partzyme components, and then bind and catalytically modify a substrate to generate an output signal. The assembly facilitator may be a target nucleic acid present in a biological or environmental sample. In such cases, catalytic activity of the MNAzymes is indicative of the presence of the target. Several MNAzymes capable of cleaving nucleic acid substrates have been reported and additional MNAzymes which can ligate nucleic acid substrates are also known in the art (see, for example, WO/2007/041774, WO/2008/040095, WO2008/122084, and related US patent publication numbers 2007-0231810, 2010-0136536, and 2011-0143338)

Aptazymes are specific types of catalytic nucleic acids (DNAzymes, ribozymes or MNAzymes) which have been linked with an aptamer domain to allosterically regulate the nucleic acid enzymes such that their activity is dependent on the presence of the target analyte/ligand capable of binding to the aptamer domain. Complementary regulator oligonucleotides have been used to inhibit the activities of aptazymes in the absence of target analytes by binding to both the aptamer and part of the catalytic nucleic acid domains within the aptazymes. The inhibition of catalytic activity of aptazymes was reversible by the binding of target ligands to the aptamer portions thus promoting removal of the regulator oligonucleotide.

### - Signal Amplification Technologies

In order to increase the sensitivity of target detection, strategies for target amplification or signal amplification have been employed. Examples of existing methods which employ target amplification include the polymerase chain reaction (PCR), strand displacement amplification (SDA), loop-mediated isothermal amplification (LAMP), rolling circle amplification (RCA), recombinase polymerase amplification (RPA), helicase dependant amplification (HDA), strand invasion based amplification (SIBA), transcript-mediated amplification (TMA), self-sustained sequence replication (3SR), or nucleic acid sequence based amplification (NASBA). Those methods which are dependent on strand displacement for amplification, for example SDA, RCA and LAMP, require the use of polymerases which possess strand displacing activity. Signal amplification cascades that utilize nucleases including nicking endonucleases have also been described (e.g. NESA).

Many limitations are evident in existing signal amplification methods. For example, in a number of techniques the speed of the reaction is limited by the number of target DNA molecules initially present in the sample. These and other methods thus often lack the speed and sensitivity required for clinical application. Others suffer from false positive signal generation in the absence of target. Thus, there is an ongoing need for methods for detecting and quantifying nucleic acid sequences and other targets which incorporate signal amplification.

Signal amplification cascades that utilize catalytic nucleic acids have also been described. One example involves the use of an oligonucleotide consisting of two adjacent peroxidase-mimicking DNAzymes joined by a ribonucleotide junction. The ends of this oligonucleotide are linked together via a short linker DNA which hybridizes to each end, so as to form a quasi-circular structure. The formation of this structure temporarily inhibits the catalytic activity of the DNAzymes. An MNAzyme that assembles in the presence of its target assembly facilitator molecule hybridizes directly to the DNAzymes and cleaves the ribonucleotide junction between them. Cleavage of the oligonucleotide which contains the DNAzymes results in separation of the two DNAzymes from each other, and from the short linker DNA, resulting in activation of the DNAzymes. The limitations of this strategy are that amplification of signal is limited to only two DNAzymes activated for each MNAzyme cleavage event, and since these peroxidase-mimicking DNAzymes have no capacity to modify nucleic acid substrates, there is no mechanism for these DNAzymes to activate additional DNAzyme molecules. As such the strategy is unsuitable as a first step in a circular feedback cascade capable of exponential signal amplification. Further, the complementarity between the MNAzyme arms and the DNAzymes, which is necessary for the initial cleavage event to occur, may also result in the sequestering of DNAzyme molecules once they have been released from the quasi-circular structure, potentially limiting the sensitivity of the reaction.

Another approach, known as DoC, utilizes quasi-circular structures consisting of temporarily inactivated DNAzymes. The DNAzymes are inactivated by hybridization to Blocker Fragments. Cleavage of the Blocker by MNAzymes activates the DNAzymes thus initiating a signal amplification cascade. There are limitations to this approach, however, because the concentration of blocker needs to be higher than that of the DNAzymes making a substantive proportion of the MNAzyme cleavage futile. Further these quasi circular structures may be unstable and have the potential to initiate the cascade in the absence of target.

Signal amplification strategies using immobilised catalytic nucleic acid enzymes tethered to solid/fixed supports have also been described. The aim of tethering catalytic nucleic acid enzymes, their individual components, and/or their substrates to fixed/immobile supports in this manner is to promote spatial separation of the enzymes from their substrates and thereby attempt to promote reaction efficiency and/or specificity. However, signal amplification approaches relying on catalytic nucleic acid enzymes, substrates, and other components tethered to fixed/immobile solid supports are often difficult to develop and have in many cases proven sub-optimal due to inherent drawbacks. For example, there are significant difficulties in balancing (i) the need to tether substantial concentrations of catalytic nucleic enzymes, substrates, and other components to drive efficient reactions with (ii) the need to ensure that the density of these components is not too high such that molecular crowding inhibits accessibility of other reaction components due to steric hindrance. Other difficulties include (iii) undesirable conformational changes and/or potential surface interactions that may interfere with the activity of the nucleic acid catalytic domains and (iv) reduced reaction rates caused by diffusion restrictions and/or (v) the need to ensure that all unbound catalytic nucleic acids are removed to prevent cascade initiation in the absence of target.

A need exists for improved assays for the detection of target molecules. Preferably, the improved assays can facilitate increased sensitivity through improved signal amplification.
WO 96/27026 A1 relates to a method for detecting the presence of a catalytically active ribozyme in a medium. The detection is carried out in a catalytic system wherein the presence of the active ribozyme serves to produce other active ribozyme in a positive-feedback amplificatory manner.

### Summary of the Invention

The invention described herein alleviates at least some of the problems in the prior art associated with signal amplification assays utilising reaction components tethered to fixed/immobile supports. The present inventors have devised a system capable of maintaining spatial separation of nucleic acid enzymes and their components (e.g. partzyme oligonucleotide components of MNAzymes) from their substrates without the inherent drawbacks of assays that rely on fixed/immobilised supports. The assays described herein thus achieve the enhanced reaction efficiency and/or specificity that has been sought by signal amplification assays utilising reaction components tethered to fixed/immobile supports. In general, the assays of the present invention utilise a sectively permaeable membrane to separate nucleic acid enzymes and/or their components from their substrates. The components remain mobile in the assay mix which provides various advantages over assays relying on fixed/immobile supports. Upon undergoing a modification (e.g. cleavage of a mobile support component, alteration in charge and/or size, or other), which is triggered only when target molecule is present, the enzymes/enzyme components become capable of permeating the membrane and thereby coming into contact with their substrates to modify them and provide a signal.

More specifically, the present inventors have devised a rapid detection assay utilising subZyme oligonucleotides each comprising a catalytic nucleic acid substrate component, a catalytic nucleic acid component (either a full catalytic nucleic acid or a portion thereof such as a partzyme oligonucleotide), and a component and/or property preventing the subZyme oligonucleotide from moving through an otherwise permeable membrane utilised in the assay. The subZyme oligonucleotides are mobile in solution rather than, for example, being tethered to a planar surface. This provides a number of advantages including, but not limited to, improved accessibility of catalytic nucleic acids to their substrates and increased rates of diffusion to opposite sides of the membrane leading to faster detection. The catalytic nucleic acid component of the subZyme oligonucleotide exists in a catalytically active form without, for example, any conformational constraint/s (e.g. a hybridised inhibitory molecule/oligonucleotide) that would reduce or prevent its catalytic activity in the presence of its target substrate. Providing the catalytic nucleic acid component in an uninhibited form avoids the reduction in catalytic activity associated with utilising enzymes subjected to conformational constraints. In the assays described herein, the presence of a target molecule triggers cleavage of the subZyme oligonucleotide releasing the catalytic nucleic acid component from the subZyme oligonucleotide and allowing the released catalytic nucleic acid component to permeate the membrane. Once through the membrane the released catalytic nucleic acid component can cleave other subZyme oligonucleotides comprising its substrate and thereby release other catalytic nucleic acid components. These can in turn permeate to the other side of the membrane to cleave further subZyme oligonucleotides, and so on. The assays of the present invention thus rely on the transfer of catalytically active nucleic acid enzymes, and/or components thereof such as partzyme oligonucleotides, across the membrane following an initial target recognition event. The remainder of the cleaved subZyme which includes the substrate component remains on one side of the membrane and is still unable to permeate through it. Accordingly, the assays described herein do not rely on or require the transfer of nucleic acid enzyme substrates across the membrane. Rather, they rely on the provision of catalytically active nucleic acids, and/or components thereof, as components of subZyme oligonucleotides, and their subsequent release and transfer across the membrane upon a target recognition event.

The target recognition event of the assays described herein utilises an 'initiating' catalytic nucleic enzyme which specifically detects the target and upon doing so effects cleavage of a subZyme oligonuleotide. The present inventors have observed that the use of initiating enzymes in the manner disclosed herein can increase the sensitivity and/or specificity of the detection assay in comparison to other methods such as, for example, those relying on target molecules to remove inhibitory component/s from catalytic nucleic acids to facilitate their activity.

The assays of the present invention may incorporate feedback loops making them capable of signal amplification, independent of the number of target molecules present. A singletarget molecule can initiate a cascade in which an initial subZyme is cleaved in a target-dependent fashion by an initiating enzyme. This releases the catalytic nucleic acid component of the subZyme which is free to permeate across the membrane. Once across the membrane, the released catalytically active nucleic acid, and/or components thereof, can access and cleave its substrate, which exists as a component of another subZyme. This event in turn releases another catalytically active nucleic acid, or components thereof (e.g. partzyme oligonucleotides), from the cleaved subZyme which can move across the permeable membrane and cleave its substrate, which again is a component of another subZyme. SubZyme cleavage events can thus be perpetuated in this manner and a feedback amplifcation loop is established that is independent of additional target molecules being present. This provides a significant advantage over, for example, linear cascades in which the amount of signal generated is more restricted by the number of target molecules present.

The present invention is as defined in the appended claims.

### Brief Description of the Figures

Preferred embodiments of the present invention will now be described by way of example only, with reference to the accompanying figures wherein:

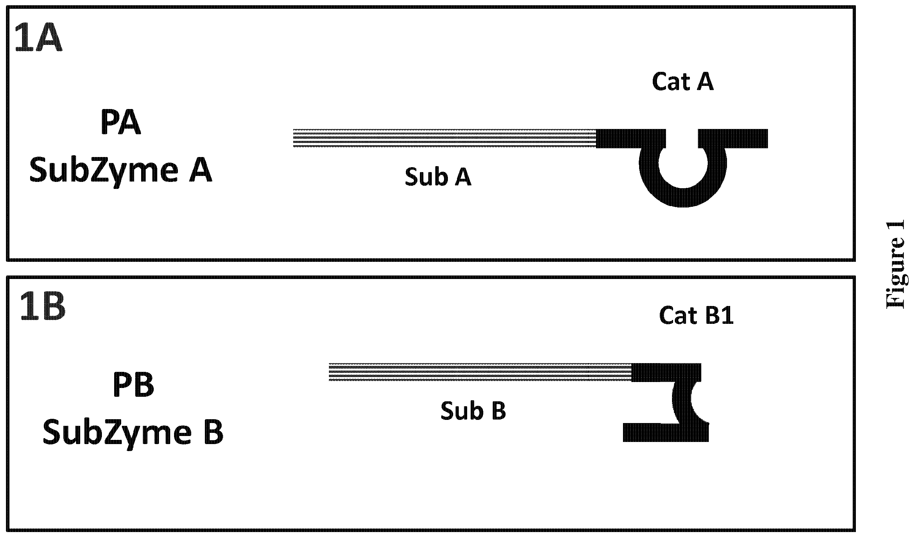

**Figure 1** depicts two exemplary SubZymes according to embodiments of the present invention. In this illustration SubZymes include a 'Sub' which is a substrate for an enzyme linked to a 'Cat' which is a Catalytic Nucleic Acid Molecule (as illustrated in **Figure 1A**) or a component of a Catalytic Nucleic Acid Molecule (as illustrated in **Figure 1B**)**.** By way of example, SubZyme A in **Figure 1A** could comprise a Cat A which is a 10-23 DNAzyme and a Sub A which is cleavable by a 8-17 DNAzyme. Alternatively, a SubZyme A could comprise a Cat A which is a 8-17 DNAzyme and a Sub A which is cleavable by a 10-23 DNAzyme. In other embodiments, illustrated as SubZyme B (**Figure 1B**), Subzymes could comprise a Cat B1 which is a partzyme B1 component for a multi compent nucleic acid enzyme (MNAzyme) linked to Sub B cleavable by a catalytic nucleic acid enzyme.

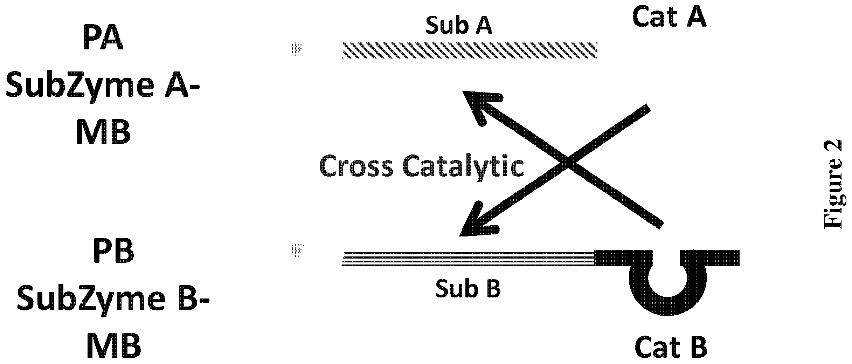

**Figure 2** depicts an exemplary pair of SubZymes according to embodiments of the present invention, which are capable of cross-catalysis. Both SubZymes are depicted as being attached to optional magnetic beads (MB) which can be used to manipulate the subZymes. By way of example, PA (SubZyme A-MB) comprises a Cat A and Sub A, and PB (SubZyme B-MB) comprises Cat B and Sub B. By way of example, PA comprises Cat A (a 10-23 DNAzyme A) linked to sub A which is cleavable by Cat B (an 8-17 DNAzyme B); PB comprises Cat B (an 8-17 DNAzyme B) linked to sub B which is cleavable by Cat A (10-23 DNAzyme A). Conversely, PA comprises Cat A (8-17 DNAzyme A) linked to substrate A which is cleavable by Cat B (10-23 DNAzyme B); PB comprises Cat B (10-23 DNAzyme B) linked to substrate B which is cleavable by Cat A (8-17 DNAzyme A). In addition Sub A and/or Sub B may be cleavable by an MNAzyme, for example an initiating MNAzyme;

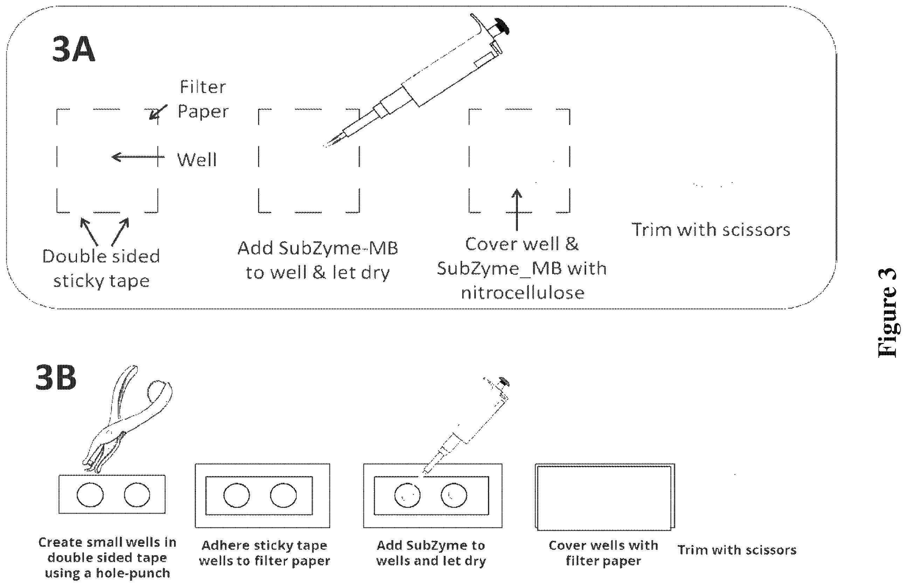

**Figure 3** depicts exemplary components and construction of a permeable barrier according to embodiments of the present invention also referred to herein as a 'T-bag'. T-bags can be constructed from inexpensive components including, for example, filter paper, sticky tape, glue and synthetic oligonucleotides. **Figure 3A** illustrates "Wells" made by placing double sided sticky tape on filter paper as indicated. Oligonucleotide (SubZymes) attached to bulky particles such as magnetic beads can then be spotted into the well and allowed to dry. A second layer of filter paper can be placed on top and the T-bag can be trimmed. **Figure 3B** illustrates T-bags made by folding double sided sticky tape in half so that the sticky sides are folded back on itself, exposing only the non-adhesive liner. "Wells' can be made using a hole punch and by placing the wells on strips of filter paper as indicated. Oligonucleotide (SubZymes) attached to bulky particles such as magnetic beads can then be spotted into the well and allowed to dry. A second layer of filter paper can be placed on top and the T-bag can be trimmed;

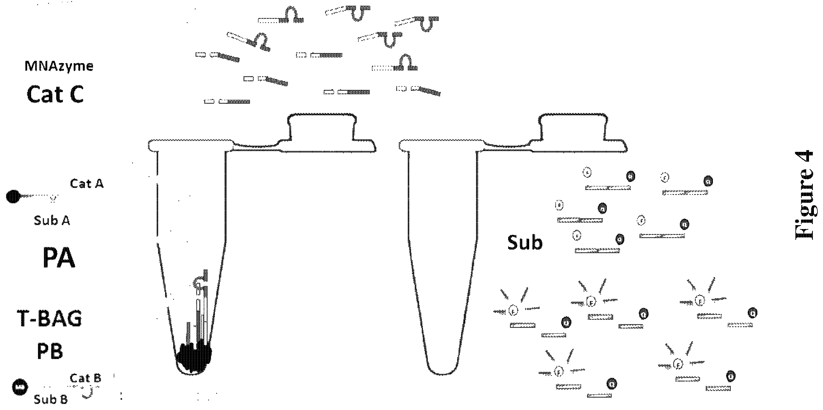

**Figure 4** depicts an exemplary scheme according to embodiments of the present invention in which permeable barriers (referred to as 'T-bags') and SubZymes are used to amplify a signal following detection of the target. The tube contains PA (SubZyme A - MB), PB (SubZyme B - MB inside a T-bag), and Partzymes for the chosen target. In the presence of the target, the partzymes align and form a Cat C (an MNAzyme). The MNAzyme cleaves Sub A (eg 10-23 Substrate) within PA separating the Cat A (eg 8-17 DNAzyme A) from the MB which allows it to migrate through the T-bag wall where it can cleave Sub B within PB thus separating the Cat B (eg 10-23 DNAzyme B) from the MB. The Cat B is now free to migrate from the T-bag into solution where it can cleave Sub A in PA and initiate the cascade. After a period of incubation a magnet can be used to retain uncleaved SubZyme-MB and cleaved MB fragments thus allowing the supernatant containing the free DNAzymes (Cat A and Cat B) to be transferred to another tube containing labelled Sub A and/or Sub B. The fluorescence which is generated following separation of the fluorophore and the quencher by DNAzyme cleavage can be monitored in real time;

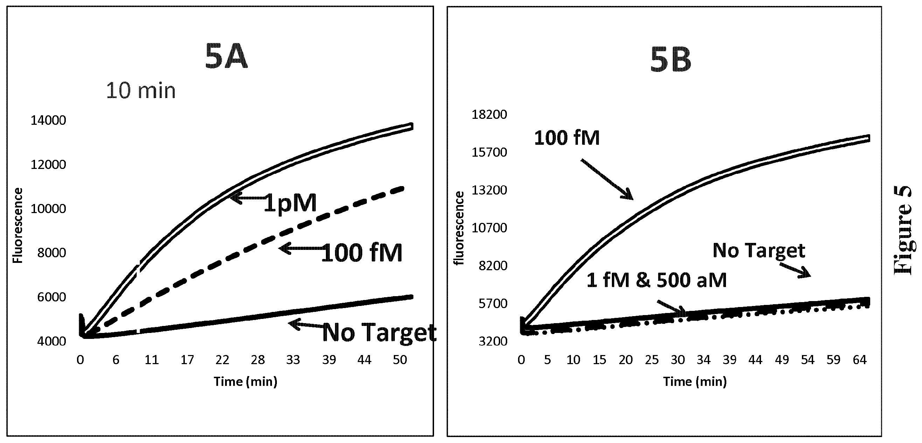

**Figure 5** depicts the results of an exemplary assay according to embodiments of the present invention in which a titration of oligonucleotide target homologous to the human TFRC gene was undertaken. Plots show monitoring of substrate cleavage following T-bag incubation reactions containing TFRC targets (1 pM, 100 fM, 1 fM and 500 aM) or no target. In this experiment, concentrations of 1 pM and 100 fM were easily detected above background signal generated in the absence of no target oligonucleotides. Graphs show real time monitoring of fluorescence substrate cleavage following incubation in a T-bag for 15 min (5A) or 20 mins (5B). Detection of 100 fM was evident after 10 min of real time monitoring. Under these experimental conditions 1 fM and 500 aM target concentrations did not generate a signal above background;

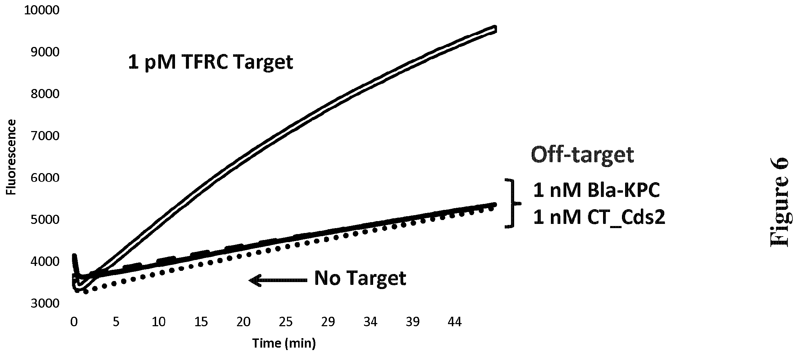

**Figure 6** depicts the results of an exemplary assay according to embodiments of the present invention in which successful detection of 1 pM of synthetic oligonucleotides target homologous to TFRC is demonstrated. In contrast 1 nM of off-target oligo templates Bla-KPC and CT_Cds2 did not give a signal above background (no target). The plots show monitoring of substrate cleavage following T-bag incubation reactions containing target, off-target or no target;

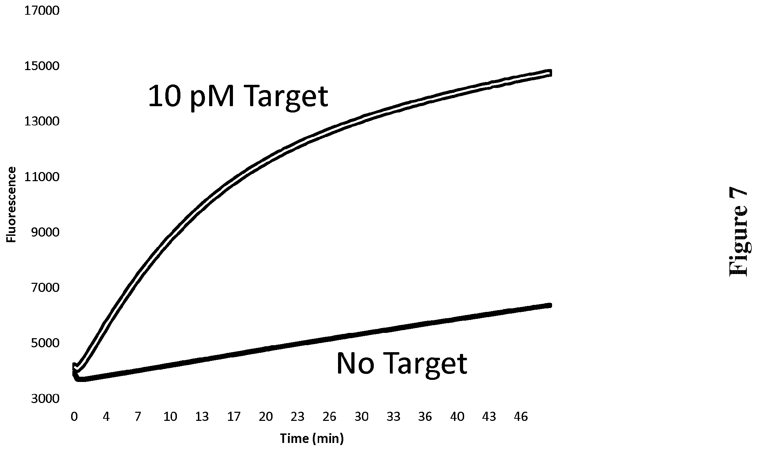

**Figure 7** depicts the results of an exemplary assay according to embodiments of the present invention in which successful detection of 10 pM of synthetic oligo template for the OXA gene which confers resistance to Beta-lactamase is demonstrated. The plots show monitoring of substrate cleavage following T-bag incubation reactions either containing or lacking target;

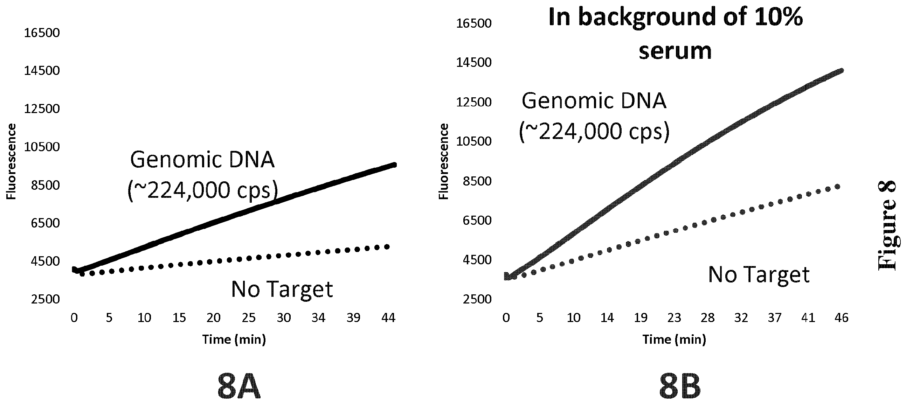

**Figure 8** depicts the results of an exemplary assay according to embodiments of the present invention in which successful detection of the human TFRC gene in human genomic DNA was demonstrated. The plots show monitoring of substrate cleavage following T-bag incubation reactions containing or lacking target. The experiment was performed in the presence (8B) or absence (8A) of 10% human serum; and

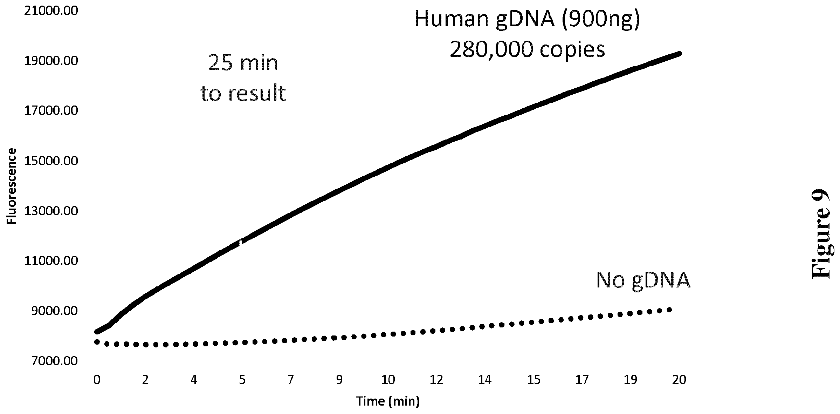

**Figure 9** depicts the results of an exemplary assay according to embodiments of the present invention in which successful detection of the human TFRC gene in human genomic DNA was demonstrated. In this experiment three MNAzymes, targeting the human TFRC gene, were used to initiate the reaction. The plots show monitoring of substrate cleavage following 20 minute T-bag incubation reactions which either contain or lack gDNA target.

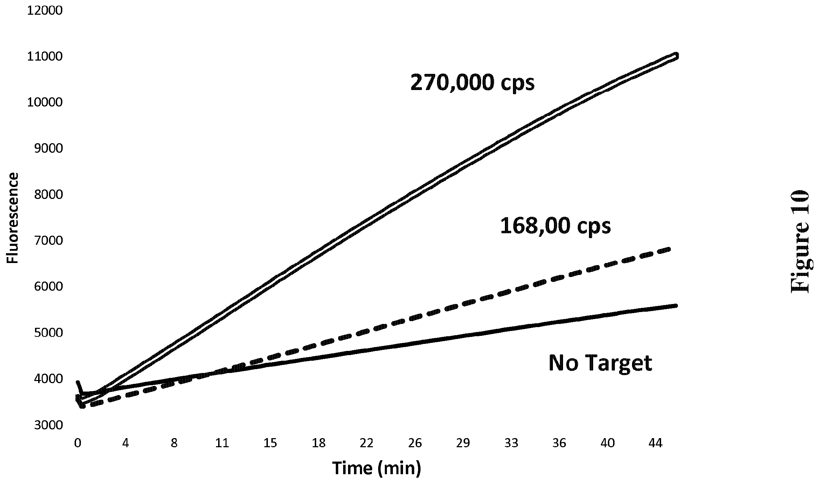

**Figure 10** shows the results of an exemplary assay according to embodiments of the present invention in which successful detection of the human TFRC gene in human genomic DNA was demonstrated. In this experiment MNAzymes targeting the human TFRC gene were used to initiate the reaction. Plots show monitoring of substrate cleavage following 20 minute T-bag incubation reactions which either contained gDNA with an estimated 168,000 or 270,000 copies of the TFRC gene or lacked gDNA target;

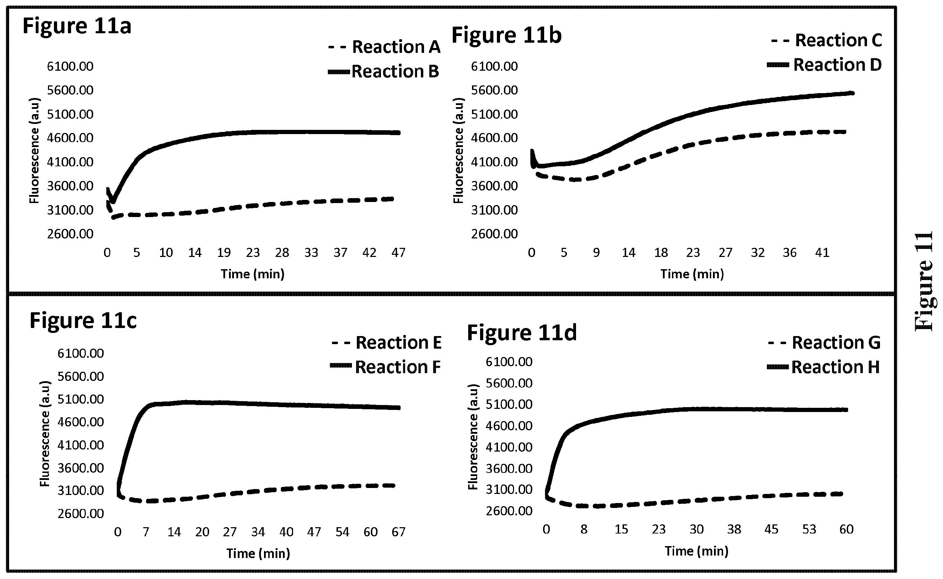

**Figure 11** depicts the results of an exemplary assay involving two SubZymes. Solid lines are reactions containing target, while dotted lines lack target. SubZyme A (11c and 11d) comprises an 8-17 catalytic nucleic acid component and a 10-23 catalytic nucleic acid substrate component. SubZyme C (11a and 11b) comprises a 10:23 catalytic nucleic acid component and a non-complementary 10-23 catalytic nucleic acid substrate component. The results show that a SubZyme comprised of a mixture of 8-17 and 10-23 catalytic nucleic acid components has greater stability long term than a SubZyme comprised of only 10-23 catalytic nucleic acid components;

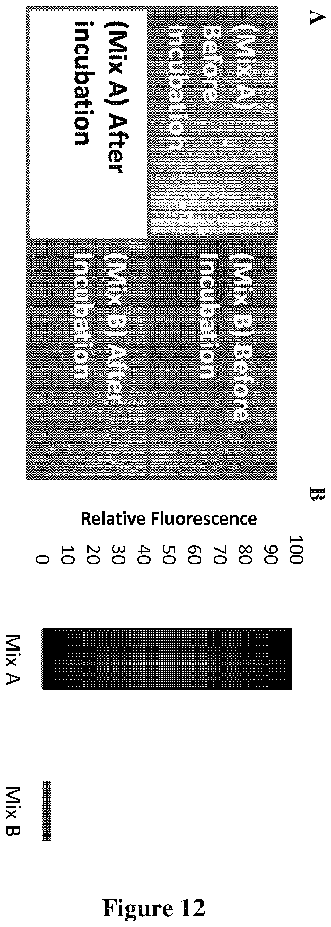

**Figure 12** depicts results of an exemplary SubZyme according to embodiments of the present invention which has been tethered onto a planar glass slide. The results show that non-tethered SubZymes (Mix A) were able to hydrolyse the nucleic acid substrate and generate flurescence, whereas detection with the tethered SubZymes (Mix B) was not apparent; and

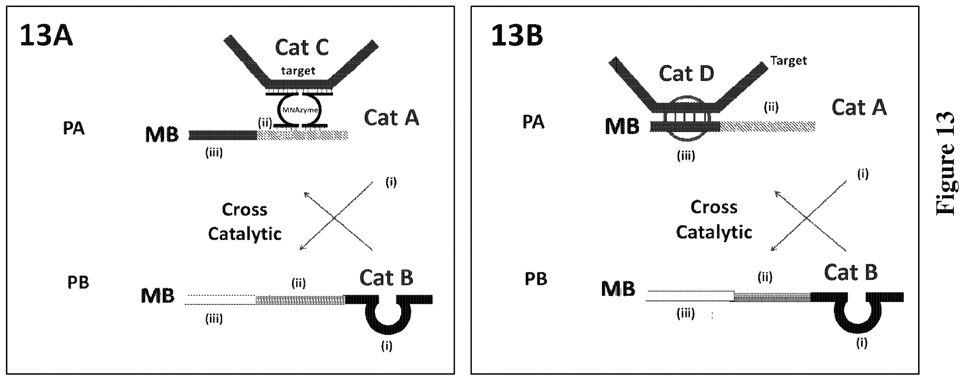

**Figure 13** depicts two exemplary pairs of SubZymes according to embodiments of the present invention, both of which are capable of cross-catalysis. Both SubZyme pairs denoted as polynucleotide A (PA) & polynucleotide B (PB), are depicted as being attached to optional magnetic beads (MB) which can be used to manipulate the SubZymes. PA and PB may be optionally attached to magnetic beads (MB) by either the 5' or the 3' end. SubZyme A-MB (PA) comprises functional domains namely; (i) Cat A (for example an 8-17 DNAzyme), (ii) Sub A (for example a substrate A which is cleavable by a 10:23 nucleic acid enzyme), and optionally (iii) either linker sequence (**Fig 13A**) or sequence which is complementary to a target nucleic acid (**Fig 13B**) and which additionally comprises one strand of an recognition site for an endonuclease or exonuclease, for example a restriction enzyme (RE)/Nicking enzyme (NE). SubZyme B-MB (PB) comprises functional domains namely; (i) a Cat B (for example a 10:23 DNAzyme B), (ii) Sub B (for example a substrate B which, is cleavable by 8:17 DNAzyme/Cat A) and optionally (iii) linker sequence (**Fig 13A and 13B**)**.** In the scheme in **Fig 13A****,** the presence of a target nucleic acid results in the formation of, for example, a 10:23 MNAzyme C (Cat C) capable of cleaving SubA. Cleavage of SubA within PA results in release of the Cat A which can subsequently move through a selectively permeable barrier and cleave Sub B within PB to release Cat B. Cat B can in turn move through a selectively permeable barrier and cleave a new Sub A within another PA and release more Cat A, thus initiating a cross catalytic signal amplification cascade. In the scheme in **Fig 13B****,** the presence of a target nucleic acid hybridizes to target specific sequence (iii) in PA thus creating double stranded duplex region which forms a recognition site for a Catalytic Enzyme D/Cat D (e.g. an RE/NE). Cleavage of PA by the Cat D can release Cat A, which can subsequently move through a selectively permeable barrier, and cleave Sub B within PB. Cleavage of PB releases Cat B which can in turn move through a selectively permeable barrier and cleave Sub A within another PA to release more Cat A, thus initiating a cross catalytic signal amplification cascade; and

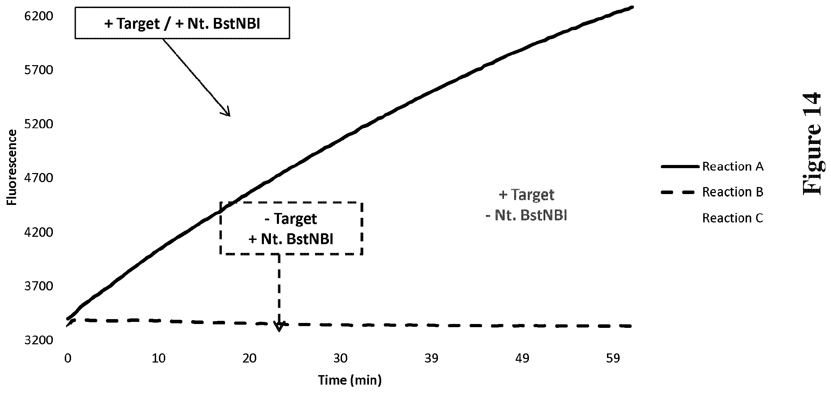

**Figure 14** depicts the results of an exemplary assay according to embodiments of the present invention in which a restriction endonuclease was used to demonstrate a first step of assay initiation as an alternative to an MNAzyme. This strategy is illustrated in **Figure 13B****.** The Plot shows monitoring of substrate cleavage following T-bag incubation reactions containing or lacking the nicking endonuclease Nt. BstNBI (+/- 4 units) and ompA target (+/-2 nM). In this experiment, an increase in signal was only observed in the presence of both the nicking endonuclease and the ompA target, indicating that both are required for the target induced release of surface-bound DNAzymes (Cat A); and

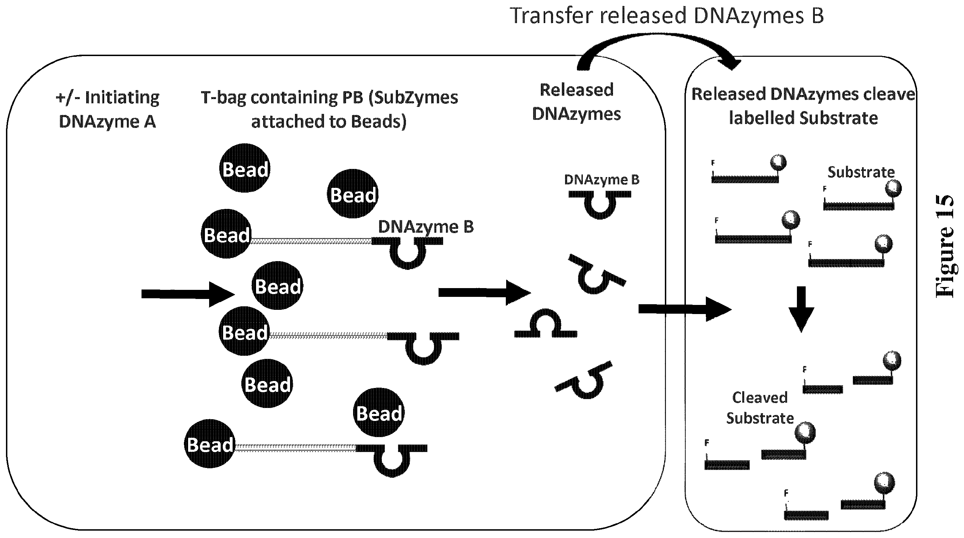

**Figure 15** depicts an exemplary scheme according to embodiments of the present invention in which permeable barriers (referred to as 'T-bags') are used as a mechanism to encapsulate SubZymes tethered to Beads and spatially confine them so they are unable to interact with reaction components restricted to the outside of the T-bag permeable barriers. The tube contains PB (SubZyme/Bead) inside a T-bag. In the absence of an initiating DNAzyme (DNAzyme A), the SubZyme-Beads remain encapsulated within the T-Bag via the semi-permeable barrier. However, when an initiating DNAzyme (DNAzyme A) is added to the tube, it can migrate through the walls of the T-Bag where it can cleave the Substrate B within SubZyme-Bead thus separating DNAzyme B from the Bead. DNAzyme B is now free to migrate from the T-bag into solution. If magnetic beads are used, a magnet can optionally be used to retain uncleaved SubZyme-Bead and cleaved Bead fragments after a period of incubation. The supernatant containing the free DNAzymes (DNAzyme B) to be transferred to another tube containing labelled substrate.. The fluorescence which is generated following separation of the fluorophore and the quencher by DNAzyme B cleavage can be monitored in real time, and;

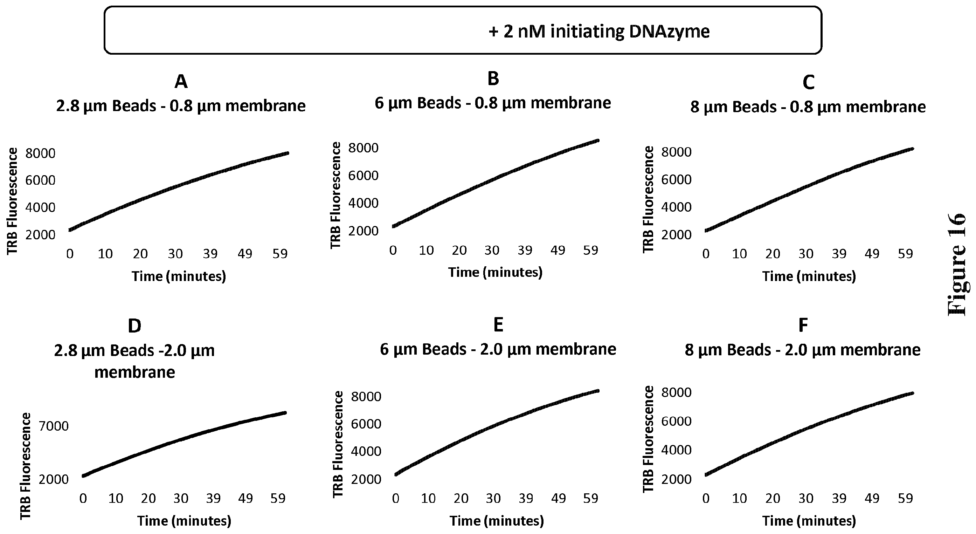

**Figure 16** (parts **A-F**) depicts the results of an exemplary assay according to embodiments of the present invention in which SubZymes, tethered to magnetic beads of various sizes, are spatially confined within the boundaries of a T-bag made with membrane composed of different pore sizes. The plots show results of monitoring of substrate cleavage following T-bag incubation reactions containing, or lacking, initiating DNAzyme. This strategy is illustrated in **Figure 15****.** The results show negligible signal in the absence of initiating DNAzyme, indicating that SubZymes are unable to penetrate the T-bag membrane whilst tethered to magnetic beads that are of a larger diameter than the pore size of the membrane. There is a strong signal in the presence of initiating DNAzyme A, indicating that the initiating DNAzyme can diffuse into T-bags, cleave SubZyme-MB and release surface-bound DNAzymes. It also indicates that the released DNAzymes are able to migrate outside of the T-Bag and cleave fluorescent labelled substrate molecules. Similar results are shown for various magnetic bead sizes (2.8 µm, 6 µm and 8 µm) and various pore sizes (0.8 µm and 2.0 µm) - see parts **A-F;** and

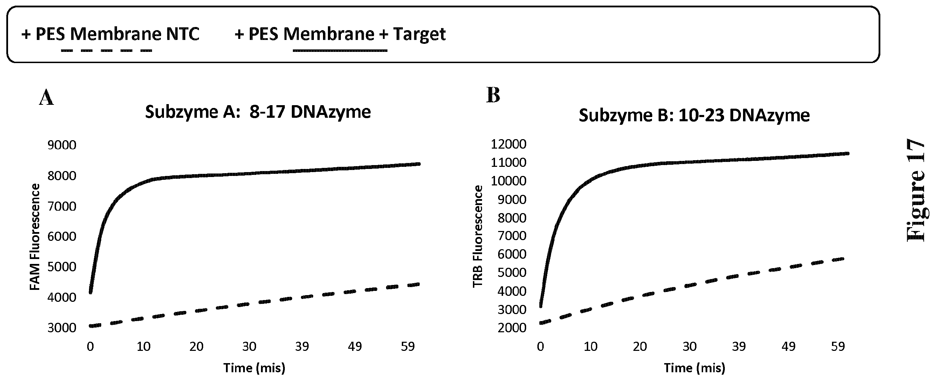

**Figure 17** depicts the results of an exemplary assay according to embodiments of the present invention in which the addition of PES membrane *per sec* to SubZyme-MB reactions was tested to determine its effect on the catalytic activity of DNAzymes. The Plot shows monitoring of substrate cleavage after SubZyme-MB incubation reactions containing or lacking the PES membrane (+/- membrane) and/or initiating DNAzyme (+/- 2 nM Dz). In this experiment the addition of PES membrane to SubZyme-MB reactions appears to enhance DNAzyme activity. This was observed as a faster increase in fluorescence over time for reactions containing PES membrane compared with reactions lacking membrane; and

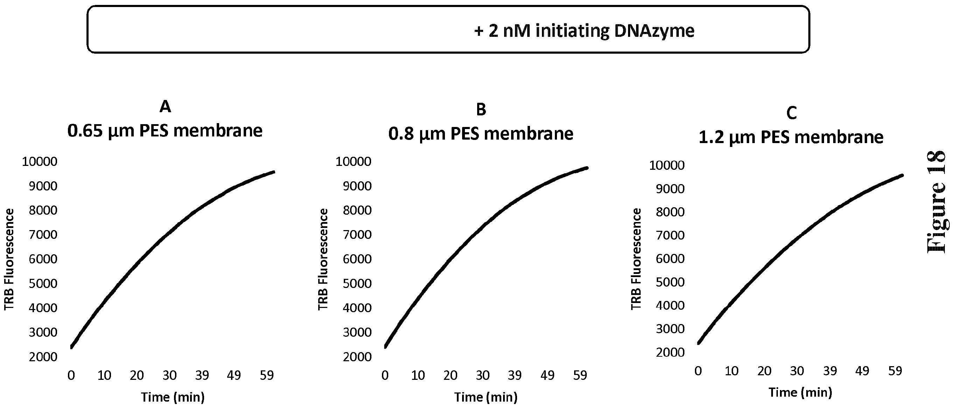

**Figure 18** (parts **A-C**) depicts the results of an exemplary assay according to embodiments of the present invention in which SubZyme-MBs are spatially confined within the boundaries of T-bags made with polyethersulfone (PES). Further, PES membranes of three different pore sizes were tested. This strategy is illustrated in **Figure 15****.** The plots show monitoring of substrate cleavage following T-bag incubation reactions containing, or lacking, initiating DNAzyme (2 nM). The results show negligible signal in the absence of initiating DNAzyme, indicating that SubZyme-MBs are unable to migrate out of the PES membrane which have 0.65 µm (part **A**), 0.8 µm (part **B**) and 1.2 µm (part **C**) pore sizes. There is a strong signal in the presence of initiating DNAzyme A, indicating that alternative membranes, such as PES, can be employed for the encapsulation and physical separation of SubZyme-MBs from other reaction components;

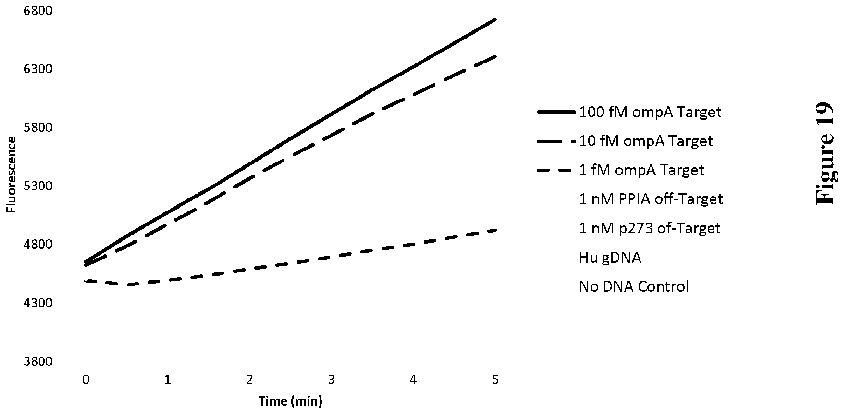

**Figure 19** depicts the results of an exemplary assay according to embodiments of the present invention in which a titration of oligonucleotide target homologous to the *Chlamydia trachomatis* ompA gene was undertaken. Plots show monitoring of substrate cleavage following T-bag incubation reactions containing ompA targets (100 fM, 10 fM and 1 fM), off-targets (1 nM), human genomic DNA (~2 x 10⁵ gene copies) or no DNA. In this experiment, concentrations of 100 fM, 10 fM and 1 fM were easily detected above background signal generated in the absence of target oligonucleotides. In contrast Human genomic DNA and 1 nM of off-target oligo templates PPIA and p273 did not give a signal above background (no DNA control);

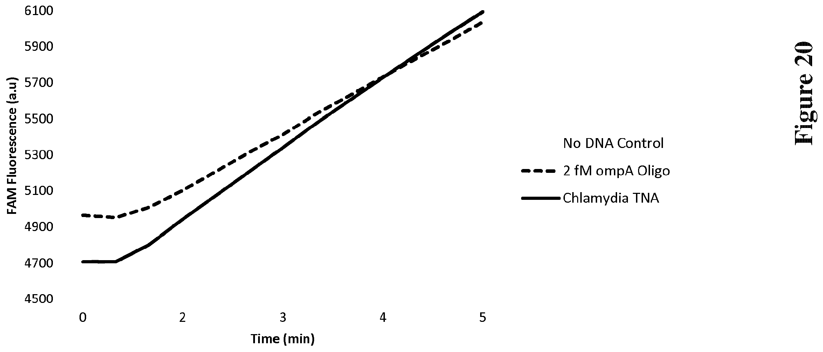

**Figure 20** depicts the results of an exemplary assay according to embodiments of the present invention in which successful detection of the *Chlamydia trachomatis* ompA gene in *Chlamydia trachomatis* total nucleic acid (TNA) samples was demonstrated. The plots show monitoring of substrate cleavage following T-bag incubation reactions containing, or lacking, target;

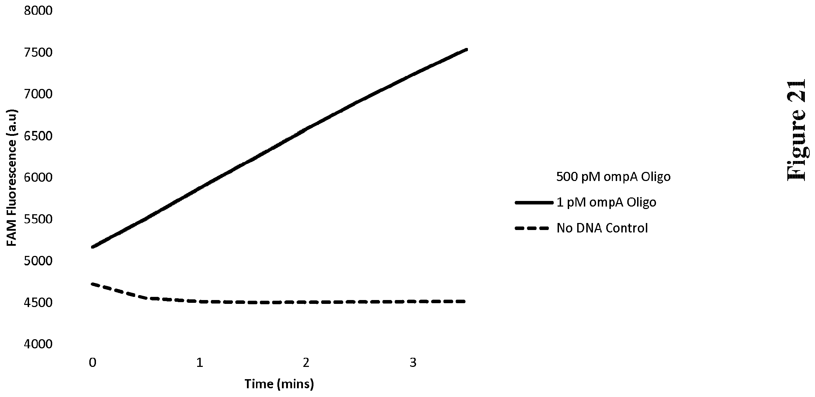

**Figure 21** depicts the results of an exemplary assay according to embodiments of the present invention in which rapid detection of oligonucleotide target homologous to the *Chlamydia trachomatis* ompA gene in less than 15 minutes was demonstrated. The plots show monitoring of substrate cleavage (3 minutes) following 12 minute T-bag incubation reactions containing, or lacking, target oligonucleotides (0 pM, 1 pM or 500 pM);

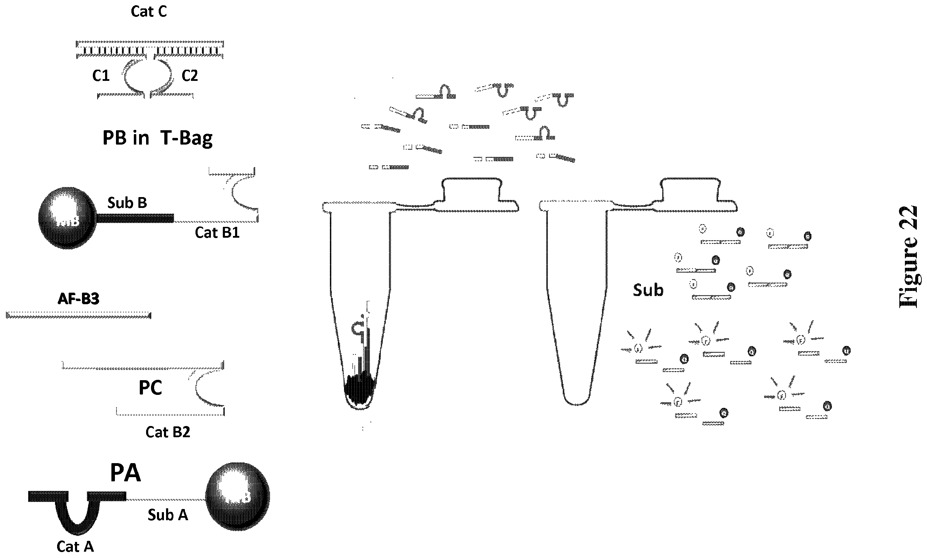

**Figure 22** depicts an exemplary scheme according to embodiments of the present invention in which permeable barriers (referred to as 'T-bags') and two types of SubZymes facilitate signal amplification following target detection. The catalytic component within PA (SubZyme A) is a DNAzyme (Cat A); within PB (SubZyme B1) is a Partzyme (Cat B1); and within PC (Subzyme B2) is a Partzyme (Cat B2). In the presence of target, an initiating MNAzyme (Cat C) forms and cleaves Sub A within PA releasing Cat A from the MB and allowing it to migrate through the T bag wall where it can cleave Sub B within PB. This releases Cat B1 from the MB allowing it to migrate out of the T bag where it can hybridize with Cat B2 (PC) and Assembly Facilitator (AF-B3) to form active Feedback MNAzyme (Cat B). The Cat B can also cleave Sub A in PA and continue the cascade. After incubation, a magnet can be used to retain SubZymes and fragments still attached to MBs. The supernatant, containing free Cat A, Cat B and Cat C, can be transferred to another tube containing labelled Sub A and/or Sub B. The fluorescence generated following substrate cleavage by DNAzymes and/or MNAzymes can be monitored;

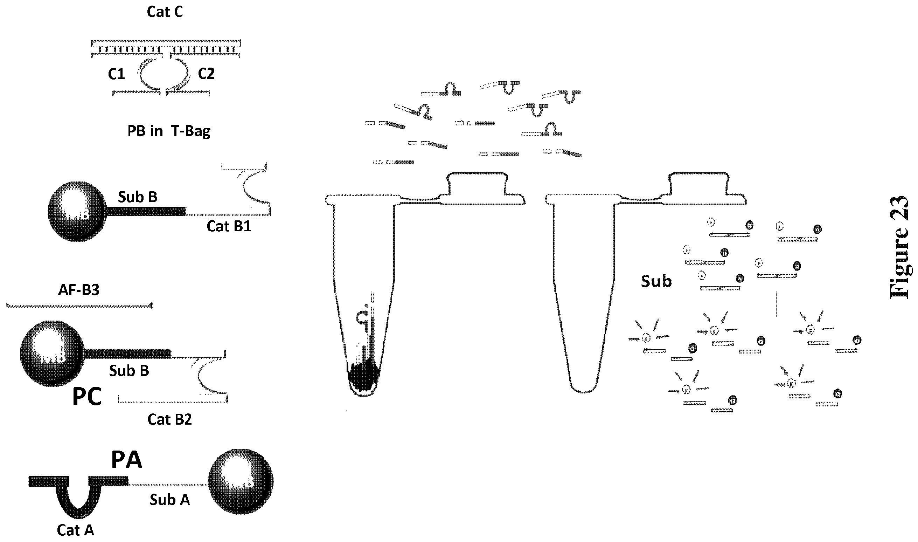

**Figure 23** depicts an exemplary scheme according to embodiments of the present invention in which permeable barriers (referred to as 'T-bags') and three SubZymes are used to amplify a signal following target detection. The catalytic component within PA (SubZyme A) is a DNAzyme (Cat A); within PB (SubZyme B1) is a Partzyme (Cat B1); and within PC (SubZyme B2) is a Partzyme (Cat B2) and PA, PB and PC are conjugated to beads. The reaction proceeds as described in **Figure 22****;** however, here, an extra level of separation can be realized so that PA and PC are separated from PB;

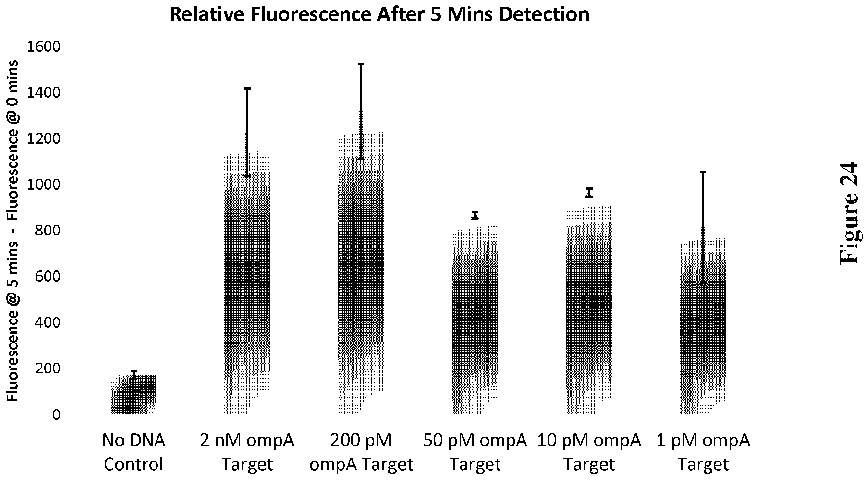

**Figure 24** depicts the results of an exemplary assay according to embodiments of the present invention in which an exemplary pair of SubZymes are capable of cross-catalysis. In this experiment two different types of SubZymes are demonstrated to be capable of cross-catalysis, PA comprises a DNAzyme linked to a substrate and PB comprises a Partzyme linked to a bead by a substrate. This strategy is illustrated in **Figure 22****.** The plots show monitoring of substrate cleavage following T-bag incubation reactions containing ompA targets (2 nM, 200 pM, 50 pM, 10 pM and 1 pM) or no target. In this experiment, all concentrations of target were easily detected above background signal generated in the absence of no target oligonucleotides;

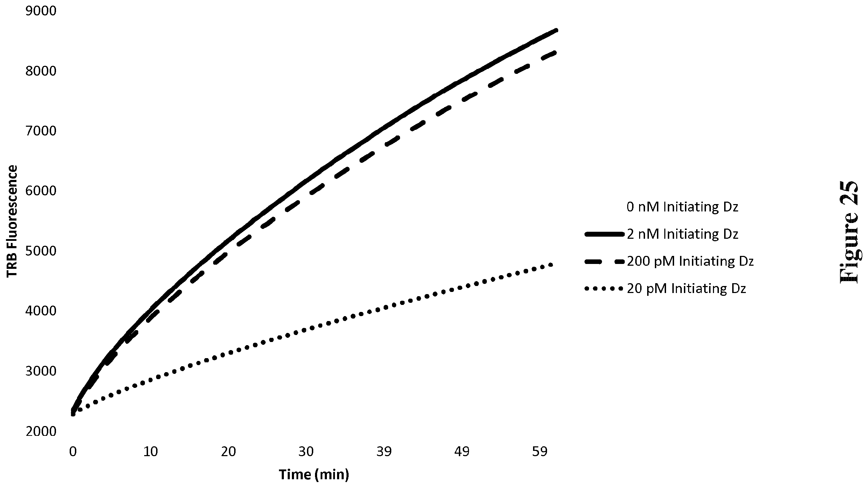

**Figure 25** depicts the results of an exemplary assay according to embodiments of the present invention in which a cross-catalytic feedback cascade using SubZymes comprised entirely of 8-17 components is demonstrated. The results demonstrate that the method is capable of detecting 2 nM, 200 pM and 20 pM of initiating DNAzyme. The signal is well differentiated from the no DNAzyme control;

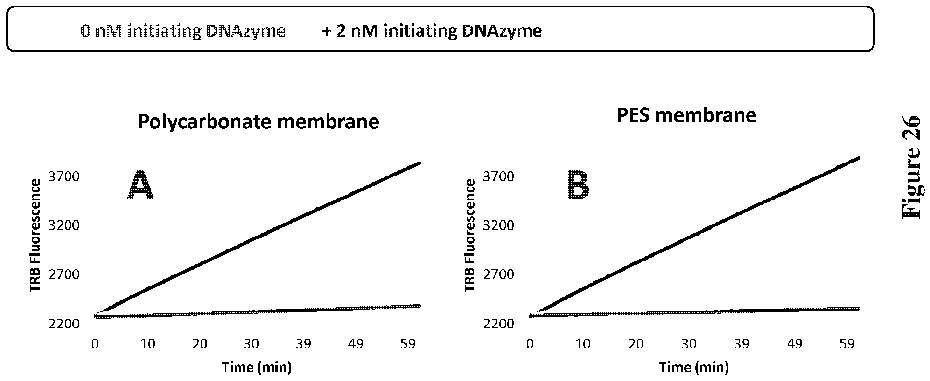

**Figure 26** (parts **A** and **B**) depicts the results of an exemplary assay according to embodiments of the present invention in which silica beads are employed as an alternative bulky surface for use in T-bag assays. This strategy is illustrated in **Figure 15****.** The results show negligible signal in the absence of initiating DNAzyme A, indicating that SubZymes are unable to diffuse through the PES membranes (26A) and PES membranes (26B) whilst tethered to silica beads (1 µm diameter). There is a strong signal in the presence of initiating DNAzyme A, indicating that the initiating DNAzyme can diffuse into T-bags, cleave SubZymes-Beads and release surface-bound DNAzymes. The results therefore demonstrate that the bead and the membrane can be composed of various materials as long as the bead is larger than the diameter of the membrane pores;

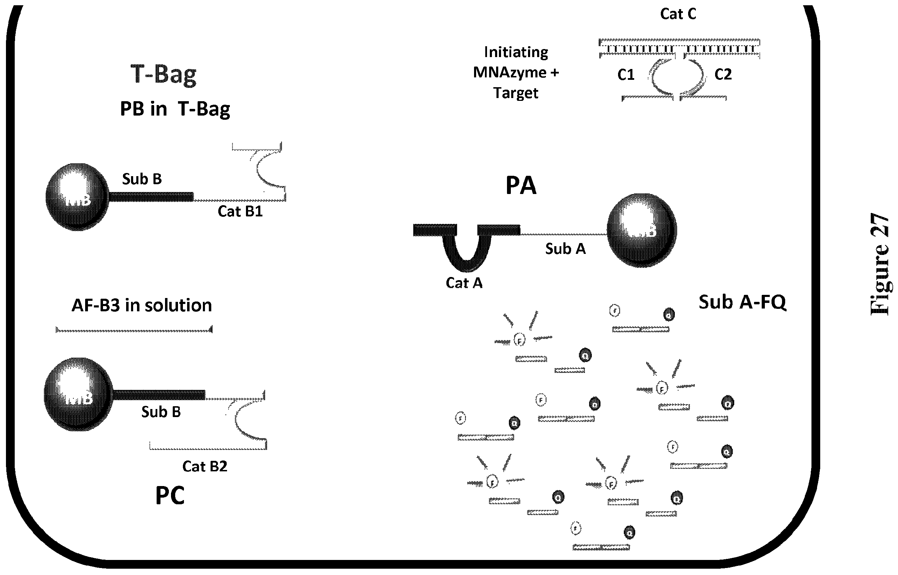

**Figure 27** depicts an exemplary scheme according to embodiments of the present invention in which permeable barriers (referred to as 'T bags') and SubZymes are used to amplify a signal and detect a signal in a single tube following target detection. The components within PA (SubZyme A) are DNAzyme (Cat A) and Sub A; within PB (SubZyme B1) are a Partzyme (Cat B1) and Sub B; and within PC (SubZyme B2) are a Partzyme (Cat B2) and optionally Sub B. In the presence of target an initiating MNAzyme (Cat C), formed from partzymes C1 and C2, cleaves Sub A within PA releasing Cat A from the MB and allowing it to migrate through the T bag wall where it can cleave Sub B within PB. This releases Cat B1 from the MB allowing it to migrate out of the T bag where it can hybridize with Cat B2 (PC) and Assembly Facilitator (AF-B3) to form active Feedback MNAzyme (Cat B). The Cat B can cleave Sub A in PA and continue the cascade. Cat B can also cleave labelled Sub A-FQ and generated fluorescence

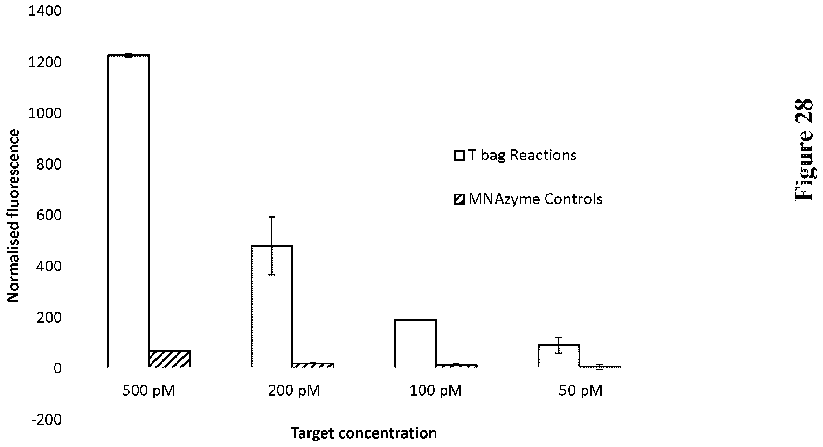

Figure 28 depicts the results of an exemplary assay according to embodiments of the present invention in which the successful detection of 500 pM, 200 pM, 100 pM and 50 pM of synthetic oligonucleotides target is demonstrated using the T bag method illustrated in Figure 27. In contrast the MNAzyme control reactions did not give a signal above background (no target).

### Definitions

Certain terms and phrases are used herein which shall have the meanings set forth as follows.

As used in this application, the singular form "a", "an" and "the" include plural references unless the context clearly dictates otherwise. For example, the term "an MNAzyme" also includes a plurality of MNAzymes. Unless the context requires otherwise or specifically stated to the contrary, integers, steps, or elements of the invention recited herein as singular integers, steps or elements clearly encompass both singular and plural forms of the recited integers, steps or elements.

Unless indicated differently, the terms "comprising" and "having" mean "including principally, but not necessarily solely". Furthermore, variations of the word "comprising", such as "comprise" and "comprises", have correspondingly varied meanings. Thus, for example, a sample "comprising" target molecule A may consist exclusively of target molecule A or may include one or more different type/s of target molecules (e.g. target molecule B and/or target molecule C).

The terms "target" and "target molecule" as used herein refer to any molecule capable of detection by the molecular complexes described herein including, but not limited to, nucleic acids, proteins, glycoproteins, lipids, lipoproteins, viruses, bacteria, archaea, fungi, antibodies, metabolites, pathogens, toxins, contaminants, poisons , small molecules, polymers, metal ions, metal salts, small organic compounds, whole cells and entire organisms. For example, a target may be a nucleic acid which serves as an assembly facilitator to direct the assembly of an MNAzyme, or any molecule capable of binding to an aptamer causing activation of a catalytic nucleic acid comprising the aptamer (e.g. apta-MNAzyme or other aptazyme). Target nucleic acids include, but are not limited to, DNApolynucelotides and RNA polynucleotides.

The terms "catalytic nucleic acid", "catalytic nucleic acid molecule", "catalytic nucleic acid enzyme" and "nucleic acid enzyme" are used interchangeably herein and have the same meaning. These terms encompass any nucleic acid, or portion thereof, capable of the specific recognition and catalytic modification of one or more substrates (e.g. nucleic acid substrates). For example, the substrate or substrates may be nucleic acids, and the catalytic modification may be ligation or cleavage. Catalytic nucleic acid enzymes as used herein include DNA molecules or DNA-containing molecules, RNA or RNA-containing molecules, and DNA-RNA or DNA-RNA-containing molecules. Non-limiting examples of catalytic nucleic acid enzymes include DNAzymes (also known as DNA enzymes and deoxyribozymes), ribozymes (also known as RNA enzymes and RNAzymes) and multi-component nucleic acid enzymes (MNAzymes) also known in the art in the art as PlexZymes.

As used herein, the term "subZyme oligonucleotide" or "subZyme" refers to an oligonucleotide comprising a component that is a catalytic nucleic acid or a portion thereof (e.g. a partzyme oligonucleotide), and a component that is a catalytic nucleic acid substrate. The catalytic nucleic acid of a subZyme oligonucleotide is unable to catalytically modify the catalytic nucleic acid substrate component of the same subZyme oligonucleotide. The catalytic nucleic acid or portion thereof, and catalytic nucleic acid substrate may be in an uninterrupted sequence along the subZyme oligonucleotide, or may be separated, for example, by intervening nucleotides and/or by any suitable linker available in the art. A subZyme oligonucleotide may comprise deoxyribonucleotide and/or ribonucleotide bases, and/or analogues, derivatives, variants, fragments or combinations thereof.

The subZyme oligonucleotide may optionally comprise additional molecule/s which may, by way of non limiting example, aid in its separation or purification (e.g. from a reaction mix) including, but not limited to, beads, microparticles and enzymes. The SubZyme oligonucleotide may comprise an additional functional group enabling attachment of the oligonucleotide to surfaces. In some embodiments of the invention and by way of non-limiting example, a subZyme oligonucleotide may comprise a 10-23 DNAzyme and a substrate for an 8-17 DNAzyme, or an 8-17 DNAzyme and a substrate for a 10-23 DNAzyme. By way of non-limiting example, a SubZyme may comprise catalytic nucleic acids selected from the group consiting of an 8-17 DNAzyme, a 10-23 DNAzyme, a 9-86 DNAzyme, a 12-91 DNAzyme, a GR-5 DNAzyme, a 17E DNAzyme, an RFD-EC1 DNAzyme, an F-8 DNAzyme, a 39-E DNAzyme, an E2 DNAzyme, an Mg5 DNAzyme, an A43 DNAzyme, a DAB22 DNAzyme, a PS2.M DNAzyme, a hammerhead ribozyme, an L-histidine-dependent DNAzyme, and an HRP DNAzyme. SubZyme oligonucleotides/subZymes according to the present invention may comprise a portion of a catalytic nucleic acid, such as for example a partzyme oligonucleotide. The portion of the catalytic catalytic nucleic acid may be capable of partial or complete enzymatic activity as compared to the complete nucleic acid from which it is derived. Alternatively, the portion of the catalytic nucleic acid may not be capable of catalytic activity until combined with other portion/s of the parent catalytic nucleic acid from which it is derived (e.g. a partzyme oligonucleotide).

The subZyme oligonucleotide may optionally comprise additional molecule/s which may, by way of non limiting example, aid in detection. Examples include, but are not limited to, fluorophore and quencher moietys for fluorescence detection; gold or silver particles for SPR and/or colorimetric detection; enzymes for electrochemical and/or pH detection and enzymes and functional groups for luminescence detection.

As used herein, the terms "polynucleotide" and "nucleic acid" are used interchangeably and have the same meaning, referring to a single-stranded or double-stranded polymer of deoxyribonucleotide and/or ribonucleotide bases, or analogues, derivatives, variants, fragments or combinations thereof including, but not limited to, DNA, methylated DNA, alkylated DNA, RNA, methylated RNA, microRNA, siRNA, shRNA, mRNA, tRNA, snoRNA, stRNA, smRNA, pre- and pri-microRNA, other non-coding RNAs, ribosomal RNA, locked nucleic acids, bridged nucleic acids, peptide nucleic acids, derivatives thereof, amplicons thereof or any combination thereof. By way of non-limiting example, the source of a given nucleic acid as described herein may be any one or more of a synthetic, mammalian, human, animal, plant, fungal, bacterial, viral, or archael source.

As used herein, the terms "oligonucleotide" and "oligo" are used interchangeably and have the same meaning, referring to, a single-stranded polymer of deoxyribonucleotide and/or ribonucleotide bases, or analogues, derivatives, variants, fragments or combinations thereof. Non-limiting examples of oligonucleotides according to the present invention include nucleic acid targets for detection by the methods and compositions described herein; catalytic nucleic acid enzymes (e.g. DNAzymes, ribozymes, MNAzymes); MNAzyme components such as partzyme oligonucleotides; aptamers; SubZyme oligonucleotides; and nucleic acid enzyme substrates (for example, those which can be modified by an MNAzyme, DNAzyme and/or ribozyme). Oligonucleotides may comprise at least one addition or substitution, including but not limited to any one or more of those set out in **Table 1** below. An oligonucleotide as referred to herein may be synthesised by any method including, for example, by chemical synthesis (e.g. from component nucleotides, or the addition of nucleotide(s) to a pre-existing fragment of the oligonucleotide). An oligonucleotide can also be constructed by ligating or otherwise joining multiple fragments of the oligonucleotide. A "ligation product" as referred to herein is a nucleic acid comprising an oligonucleotide composed of two or more oligonucleotides that have been joined (ligated) together, for example, by a ligase enzyme.

The terms "nucleotide" and "nucleotide residue" and "base" as used herein have the same meaning and encompass nucleotides comprising the bases A, C, G, T, or U, as well as derivatives or analogues thereof (non-limiting examples of which are listed in **Table 1**).

The term "derivative" as used herein in relation to a nucleic acid or nucleotide includes any functionally equivalent nucleic acid or nucleotide, including any fusion molecule produced integrally (e.g. by recombinant means) or added post-synthesis (e.g. by chemical means). Such fusions may comprise oligonucleotides of the invention with RNA or DNA added thereto or conjugated to a polypeptide (e.g. puromycin or other polypeptide), a small molecule (e.g., psoralen), or an antibody.

The term "analogue" as used herein in relation to a nucleic acid or nucleotide includes a compound having a physical structure that is related to a DNA or RNA molecule or residue, and may be capable of forming a hydrogen bond with a DNA or RNA residue or an analogue thereof (i.e. it is able to anneal with a DNA or RNA residue or an analogue thereof to form a base-pair), but such bonding is not so required for said compound to be encompassed within the term "analogue". Such analogues may possess different chemical and biological properties to the ribonucleotide or deoxyribonucleotide residue to which they are structurally related. Methylated, iodinated, brominated or biotinylated residues are examples of analogues. Active DNAzymes have been described which contain nucleotide analogues, including deoxyinosine, C-5-immidazole deoxyuridine, 3-(aminopropynyl)-7-deaza-dATP, 2'-O-methyl RNA, 2'O-methyl cap. Other analogues could also be compatible with catalytic activity of catalytic nucleic acid enzymes such as DNAzymes, ribozymes and MNAzymes. Alteration of a nucleic acid with catalytic activity, for example by substitution of one base for another, by substitution of an analogue for a base, or alteration of the sugar component or phosphodiester backbone, can be straight forward for the skilled artisan. For example, alterations can be made during synthesis or by modification of specific bases after synthesis. Empirical testing of catalytic nucleic acids incorporating alterations such as base changes or base analogues allows for assessment of the impact of the altered sequences, or specific analogues, on catalytic activity. Analogues of the bases A, C, G, T and U are known in the art, and a subset is listed in **Table 1.** Non- limiting examples of analogues which can inhibit nuclease digestion are also well known in the art. Such analogues can be strategically placed within oligonucleotides to prevent cleavage by an exonuclease and/or an endonuclease.

**Table 1: Examples of Nucleotide Analogues**

| **Abbreviation** | **Name** |
|---|---|
| ac4c | 4-acetylcytidine |
| BNA | Bridged nucleic acid |
| chm5u | 5-(carboxyhydroxylmethyl)uridine |
| Cm | 2'-O-methylcytidine |
| Cmnm5s2u | 5-carboxymethylaminomethyl thiouridine |
| D | Dihydrouridine |
| Fm | 2'-O-methylpseudouridine |
| Galq | beta, D-galactosylqueosine |
| Gm | 2'-O-methylguanosine |
| 1 | Inosine |
| i6a | N6-isopentenyladenosine |
| LNA | Locked nucleic acid |
| m1a | 1-methyladenosine |
| m1f | 1-methylpseudouridine |
| m1g | 1-methylguanosine |
| ml1 | 1-methylinosine |
| m22g | 2,2-dimethylguanosine |
| m2a | 2-methyladenosine |
| m2g | 2-methylguanosine |
| m3c | 3-methylcytidine |
| m5c | 5-methylcytidine |
| m6a | N6-methyladenosine |
| m7g | 7-methylguanosine |
| mam5u | 5-methylaminomethyluridine |
| mam5s2u | 5-methoxyaminomethyl-2-thiouridine |
| Manq | beta, D-mannosylmethyluridine |
| mcm5s2u | 5-methoxycarbonylmethyluridine |
| mo5u | 5-methoxyuridine |
| ms2i6a | 2-methylthio-N6-isopentenyladenosine |
| ms2t6a | N-((9-beta-ribofuranosyl-2-methylthiopurine-6-yl)carbamoyl)threonine |
| mt6a | N-((9-beta-ribofuranosylpurine-6-yl)N-methyl-carbamoyl)threonine |
| Mv | Uridine-5-oxyacetic acid methylester |
| o5u | Uridine-5-oxyacetic acid (v) |
| Osvw | Wybutoxosine |
| PNA | Peptide nucleic acid |
| P | Pseudouridine |
| PS | phosphothioate |
| Q | Queosine |
| s2c | 2-thiocytidine |
| s2t | 5-methyl-2-thiouridine |
| s2u | 2-thiouridine |
| s4u | 4-thiouridine |
| T | 5-methyluridine |
| t6a | N-((9-beta-D-ribofuranosylpurine-6-yl)carbamoyl)threoninetm 2'-O-methyl-5-methyluridine |
| Um | 2'-O-methyluridine |
| Yw | Wvbutosine |
| X | 3-(3-amino-3-carboxypropyl)uridine, (acp3)u |
| AraU | beta, D-arabinosyl |
| AraT | beta, D-arabinosyl |

The terms "multi-component nucleic acid enzyme" and "MNAzyme" are used interchangeably herein and will be taken to have the same meaning. They are formed from two or more partzyme oligonucleotides which, only in the presence of an MNAzyme assembly facilitator oligonucleotide or an MNAzyme assembly facilitator polynucleotide (for example, a target nucleic acid), assemble to form a catalytically active nucleic acid enzyme that is capable of catalytically modifying one or more substrates. For example, partzyme oligonucleotides A and B may each bind to a target nucleic acid by complementary base pairing with the nucleic acid target. The MNAzyme only forms when the sensor arms of partzymes A and B hybridise adjacent to each other on the target nucleic acid. The substrate arms of the MNAzyme engage the substrate, the modification of which (e.g. cleavage or ligation) is catalysed by the catalytic core of the MNAzyme, formed by the interaction of the partial catalytic domains on partzyme oligonucleotides A and B. It will be understood that terms "multi-component nucleic acid enzyme" and "MNAzyme" as used herein encompass all known MNAzymes and modified MNAzymes including those disclosed in any one or more of PCT patent publication numbers WO/2007/041774, WO/2008/040095, WO2008/122084, WO2012/065231, WO/2013/033792, WO/2013/123552, and WO2013/188912, related US patent numbers 8394946, 8945836, 9127311, 8962238, and 9506108, and related US patent publication numbers 20140017669, 20160348161, and 20160083785. Non-limiting examples of MNAzymes and modified MNAzymes encompassed include those with cleavage catalytic activity (as exemplified herein), disassembled or partially assembled MNAzymes comprising one or more assembly inhibitors, MNAzymes comprising one or more aptamers ("apta-MNAzymes"), MNAzymes comprising one or more truncated sensor arms and optionally one or more stabilising oligonucleotides, MNAzymes comprising one or more activity inhibitors, multi-component nucleic acid inactive proenzymes (MNAi), and MNAzymes with ligase catalytic activity ("MNAzymes ligases").

The term "aptazyme" as used herein, refers to a catalytic nucleic acid (e.g. a DNAzyme, a ribozyme or an apta-MNAzyme) which has been modified to incorporate an aptamer domain to allosterically regulate its activity such that it is dependent on the presence of a target analyte. Methods for incorporating an aptamer into a catalytic nucleic acid enzyme or catalytic nucleic acid enzyme component, include, but are not limited to, direct conjugation of the aptamer to one or more domains of the catalytic nucleic acid or catalytic nucleic acid component, incorporation of the aptamer into a non-functional region of the catalytic nucleic acid, or conjugation of the aptamer adjacent to a functional region of the catalytic nucleic acid. An aptamer of an aptazyme may be wholly or partially hybridised to an inhibitory molecule to inhibit catalytic activity of the aptazyme in the absence of the analyte. The inhibitory molecule may have reduced binding affinity for the aptamer in comparison to the target molecule.

The terms "assembly facilitator molecule", "assembly facilitator", "MNAzyme assembly facilitator oligonucleotide", "feedback assembly facilitator" and "MNAzyme assembly facilitator" are used interchangeably herein and refer to nucleic acids that can hybridise with a sensor arm of one or more partzyme oligonucleotides, and thereby facilitate the assembly of a catalytically active MNAzyme. Assembly facilitators may facilitate the assembly of MNAzymes which have cleavage, ligase or other enzymatic activities. An assembly facilitator may be a single molecule or comprise multiple separate molecules, which hybridise to a sensor arm of one or more partzymes oligonucleotide/s. The assembly facilitator may be a target to be detected or quantified (e.g. a nucleic acid selected from the group consisting of DNA, methylated DNA, alkylated DNA, RNA, methylated RNA, microRNA, siRNA, shRNA, tRNA, mRNA, snoRNA, stRNA, smRNA, pre- and pri-microRNA, other non-coding RNAs, ribosomal RNA, derivatives thereof, amplicons, or any combination thereof).

As used herein, the terms "partzyme", "partzyme component", and "partzyme oligonucleotide" are used interchangeably and have the same meaning, each referring to DNA-containing and/or RNA-containing oligonucleotide, two or more of which, only in the presence of an MNAzyme assembly facilitator, can self-assemble into an "MNAzyme." A partzyme oligonucleotide, comprises three domains: a "catalytic" domain, which forms part of the catalytic core of the MNAzyme which catalyses the modification of a substrate; a "sensor arm" domain, which associates with and/or binds to an assembly facilitator (e.g. a target nucleic acid); and a "substrate arm" domain, which associates with and/or binds to a substrate.

The terms "substrate", and "substrate molecule", are used interchangeably herein and refer to any molecule capable of recognition and catalytic modification by a catalytic molecule (e.g. a catalytic nucleic acid enzyme or a protein enzyme). A substrate may comprise, for example, a single-stranded or double-stranded nucleic acid capable of specific recognition and catalytic modification by a catalytic nucleic acid enzyme. A substrate that is catalytically modified may be detected by indirect and/or direct means. For example, the catalytic modification of a substrate may be detected indirectly by virtue of one or more subsequent steps in a cascade that rely on the substrate being catalytically modified. Additionally or alternatively, the catalytic modification of a substrate may be detected, for example, by directly detecting one or more modified substrate products and/or any other signal directly generated by modification of the substrate (e.g. a fluorescent signal generated by cleaving the substrate and thereby spatially separating previously paired fluorophore and quencher molecules present on the unmodified substrate). A substrate that can be detected directly upon catalytic modification by a catalytic molecule is also referred to herein as a "reporter substrate", a "reporter probe substrate", a "reporter probe" or a "probe".

As used herein the term "aptamer" encompasses a nucleic acid or peptide sequence that has the ability to recognize and bind to one or more ligands with high affinity and specificity due to their higher level structure (e.g. a 3-D binding domain or pocket). Aptamers can bind various ligands, non-limiting examples of which include nucleic acid, proteins, prions, small organic compounds, or entire organisms. Preferred aptamers herein are short single-strand DNA or RNA oligomers which can be isolated, for example, from complex libraries of synthetic nucleic acid by an iterative process of adsorption, recovery, and reamplification. Aptamers can be generated to recognise almost any target molecule, ranging from small molecules such as amino acids, or antibiotics to proteins, nucleic acid structures or whole cells.

The term "ligand" as used herein refers to any molecule capable of binding to an aptamer with high affinity and specificity, including but not limited to, proteins, prions, polypeptides, peptides or nucleic acids, glycoproteins, lipids, lipoproteins, viruses, bacteria, archaea, fungi, antibodies, metabolites, pathogens, toxins, contaminants, poisons, small molecules, polymers, metal ions, metal salts, small organic compounds, whole cells and entire organisms. A ligand may also be referred to as a "target analyte" or "analyte".

Reference herein to "hybridisation" between two or more nucleic acids, or, to two or more nucleic acids that are "hybridised", will be understood to require complementary base pairing between all or a portion of the nucleic acids.

The term "selectively permeable barrier" refers to a material (e.g. a membrane) that allows the passage of particular substances through it (in one direction or in both directions) while preventing other different substances from doing so. The selectively permeable barrier can thus be designed to maintain physical separation between two non-identical molecules. The selectively permeable barrier may achieve the physical separation based on its properties including, but not limited to, those which facilitate charge-based separation, weight-based separation, size-based separation, shape-based separation, separation based on lipid solubility, and/or separation based on affinity to carrier molecules present in the membrane. By way of non-limiting example the selectively permeable barrier may comprise any one or more of polyethersulfone, polycarbonate, nitrocellulose, regenerated cellulose, cellulose acetate, polyamide, propylene, nylon, aluminium oxide or polytetrafluoroethylene.

### Abbreviations

The following abbreviations are used herein and throughout the specification:
RE: restriction enzyme/endonuclease
*NE:* nicking enzyme/endonuclease
*DSN:* duplex-specific nuclease
*LAMP:* loop-mediated isothermal amplification
RCA: rolling circle amplification
*TMA:* transcript-mediated amplification
3SR: self-sustained sequence replication
*NASBA:* nucleic acid sequence based amplification
*MNAzyme:* multi-component nucleic acid enzyme
*DNAzyme or Dz:* deoxyribonucleic acid enzyme;
*PCR:* polymerase chain reaction;
F: fluorophore dye molecule;
Q: quencher molecule;
*JOE or 6-JOE:* 6-carboxy-4',5'-dichloro-2',7'-dimethoxyfluorescein;
*FAM or 6-FAM:* 6-Carboxyfluorescein.
*TxR:* texas red
*Oligo:* oligonucleotide
*IB:* Iowa Black
*IDT:* Integrated DNA Technologies
*SPR:* Surface Plasmon Resonance
*Sub:* Substrate
Pz: Partzyme
*Cat:* Catalytic Nucleic Acid
*PES:* polyethersulfone
*MB:* Magnetic Bead

### Detailed Description

The following detailed description conveys exemplary embodiments of the present invention in sufficient detail to enable those of ordinary skill in the art to practice the present invention. Features or limitations of the various embodiments described do not necessarily limit other embodiments of the present invention or the present invention as a whole. Hence, the following detailed description does not limit the scope of the present invention, which is defined only by the claims.

As discussed above, numerous limitations are evident in currently available assays for target molecule detection and methods designed to amplify the signals generated by such assays. One or more of these limitations are addressed by the compositions and methods of the present invention.

Compositions and methods are provided for the detection, identification and/or quantification of a target.

### Compositions

Provided herein are compositions for carrying out the methods of the invention. By way of non-limiting example only, the compositions may comprise any one or more of catalytic nucleic acid enzymes (e.g. MNAzymes and/or partzyme oligonucleotide component/s thereof, DNAzymes, and/or ribozymes), subZyme oligonucleotide/s, substrates for catalytic nucleic acid enzymes, and/or oligonucleotides which comprise one strand of a recognition site for a protein enzyme).

Various components of the compositions may be provided in a functionally inactivated form. Additionally or alternatively, various components of the compositions may be provided in a functionally active form.

Non-limiting examples of components suitable for inclusion in the compositions are provided below.

### - Catalytic nucleic acid enzymes

Compositions of the present invention comprise different types of catalytic nucleic acid enzymes and, in some embodiments, one or more components thereof (e.g. one or more partzyme oligonucleotides and/or assembly facilitator oligonucleotides) and/or the complement of catalytic nucleic acid enzymes or components thereof.

The substrates for catalytic nucleic acids may be capable of providing a detectable signal upon catalytic modification. For example, the substrate may comprise one or more detectable labels (e.g. a fluorophore and quencher).

The compositions of the present invention comprise catalytic nucleic acid enzymes that are DNAzymes. In some embodiments, the compositions may further include catalytic nucleic acid enzymes that are MNAzymes, ribozymes and/or aptazymes, and/or components thereof (e.g. partzyme(s) and/or assembly facilitator(s)), and/or those which are a component of larger entities such as subZyme oligonucleotides.

Any suitable DNAzyme in accordance with the claims may be utilised. The DNAzymes may be known/existing DNAzymes or newly generated by *in vitro* selection. The DNAzymes may be capable of cleaving or ligating either RNA or DNA molecules. Divalent metal ions such as, for example, Ba²⁺, Sr²⁺, Mg²⁺, Ca²⁺, Ni²⁺, Co²⁺, Mn²⁺, Zn²⁺, and/or Pb²⁺ may be provided as co-factors for the DNAzymes. The DNAzymes may comprise a catalytic domain (catalytic core) flanked by two non-conserved substrate binding domains ("hybridizing arms"), which are regions of sequence that specifically bind to a substrate. Non-limiting examples of suitable DNAzymes include 10-23 DNAzymes which comprise a catalytic domain of 15 deoxyribonucleotides flanked by two substrate-recognition arms, and 8-17 DNAzymes.

The compositions may comprise ribozymes. Any suitable ribozyme may be utilised. The ribozymes may be natural ribozymes or artificially generated ribozymes. The ribozymes may be capable of cleaving or ligating either RNA or DNA molecules. Divalent metal ions such as, for example, Ba²⁺, Sr²⁺, Mg²⁺, Ca²⁺, Ni²⁺, Co²⁺, Mn²⁺, Zn²⁺, and/or Pb²⁺ and/or monovalent cations may be provided as co-factors for the ribozymes. The ribozymes may comprise a catalytic domain (catalytic core) flanked by two non-conserved substrate binding domains ("hybridizing arms"), which are regions of sequence that specifically bind to a substrate. Alternatively, other ribozyme structures are contemplated wherein the structures may comprise separate target and substrate binding arms and a catalytic core. Non-limiting examples of suitable ribozymes include hammerhead ribozymes, hairpin ribozymes, branching ribozymes, maxizymes, Group I ribozymes, Group II intron ribozymes, HDV ribozyme, RNase P, CPEB3 ribozyme, *glmS* ribozyme, peptidyl transferase 23S rRNA, VS ribozyme, CoTC ribozyme and GIR1 leadzyme.

The compositions of the present invention may comprise any one or more of MNAzymes, partzyme oligonucleotide components capable of forming catalytically active MNAzymes, MNAzyme assembly facilitator oligonucleotides/polynucleotides, and/or MNAzyme substrates. As well known to those in the field, MNAzymes are catalytically active nucleic acid enzymes which self-assemble from two or more partzymes upon hybridisation to an appropriate assembly facilitator (e.g. a target). Each partzyme oligonucleotide component comprises a partial catalytic core, which upon assembly of the MNAzyme combine to form a single catalytic core capable of modifying a substrate.

Non-limiting examples of suitable MNAzymes and methods for their generation are disclosed, for example in any one or more of PCT patent publication numbers WO/2007/041774, WO/2008/040095, WO2008/122084, WO2012/065231, WO/2013/033792, WO/2013/123552, and WO2013/188912, related US patent numbers 8394946, 8945836, 9127311, 8962238, and 9506108, and related US patent publication numbers 20140017669, 20160348161 and 20160083785. Suitable MNAzymes include those with cleavage catalytic activity, those with ligation activity, disassembled or partially assembled MNAzymes comprising one or more assembly inhibitors, MNAzymes comprising one or more aptamers ("apta-MNAzymes"), MNAzymes comprising one or more truncated sensor arms and optionally one or more stabilizing oligonucleotides, MNAzymes comprising one or more activity inhibitors, multi-component nucleic acid inactive proenzymes (MNAi), and MNAzymes with ligase catalytic activity ("MNAzyme ligases"), each of which is described in detail in one or more of WO/2007/041774, WO/2008/040095, WO2008/122084, US 2007-0231810, US 2010-0136536, and/or US 2011-0143338. The partzyme oligonucleotides self-assemble in the presence of an MNAzyme assembly facilitator to form an MNAzyme. In some embodiments, the presence of an MNAzyme can be detected, and is indicative of the presence of a target, because the MNAzyme forms only in the presence of the target, wherein the target comprises the assembly facilitator.

As known to those skilled in the field, MNAzyme structures are based on one or more DNAzymes (e.g. 10-23 and 8-17 DNAzymes) and/or ribozymes. The MNAzymes may comprise ribonucleotide bases and/or deoxyribonucleotide bases and/or analogues thereof. For example, one or more of a sensor arm, a substrate arm, or the catalytic core of the MNAzyme may comprise one or more ribonucleotide bases and/or one or more deoxyribonucleotide bases and/or one or more analogues thereof. In some embodiments the MNAzyme comprises at least one deoxyribonucleotide base, or its analogue, within the catalytic core of the MNAzyme. The deoxyribonucleotide base, or its analogue, may be required for catalytic activity.

MNAzymes of the compositions may contain one or more substitutions such as analogues, derivatives, modified or altered bases, ribonucleotides, alterations of the sugar or phosphate backbone, various deletions, insertions, substitutions, duplications or other modifications, or any combination of these, well known to those skilled in the art. Such modifications, substitutions, deletions, insertions, etc may be made in the sensor and/or substrate arms and/or in the catalytic core portions such that the molecule retains catalytic activity. Substitutions and modifications to arms that bind the substrate or assembly facilitator may be well tolerated and allow tailoring of the molecules to different substrates/assembly facilitators. For example, modification of the sensor arms allows tailoring to different assembly facilitators, while modification of the substrate arms allows tailoring to different substrates.

The compositions of the present invention may comprise one or more components of a catalytic nucleic acid enzyme. For example, the compositions may comprise individual component(s) of an MNAzyme (e.g. one or more partzyme oligonucleotides, and/or one or more assembly facilitator oligonucleotides).

By way of non-limiting example, the compositions may comprise individual partzyme(s) which, upon recognition of a target molecule, are capable of self-assembly to form a catalytically active MNAzyme capable of modifying one or more substrates. The MNAzyme so formed may be designed to assemble only upon hybridisation of partzyme sensor arms to certain assembly facilitators (which may be specific target molecule(s)) and/or to only catalytically modify certain specific substrate(s) capable of hybridisation to substrate arm(s) of the MNAzyme. Accordingly, MNAzymes included in the compositions may be designed to initiate a detection and/or signal amplification cascade according to the present invention by virtue of requiring the presence of a target molecule in order to self-assemble and catalytically modify one or more substrate/s into a product required for the cascade to occur.

For example, by altering only the sensor arms of the partzymes, but by leaving the substrate arms unchanged, a large variety of MNAzymes specific for various targets can be designed all of which may utilize a universal MNAzyme substrate for detection. The skilled artisan will appreciate the advantages that this offers in terms of eliminating the need for customized or unique substrates for each target. Each new target requires only one or more changes in one or more of the sensor arm portions; the substrate arm portion and the catalytic core portion can remain constant. Thus, a single MNAzyme substrate can be used for a single target using an MNAzyme, and multiple targets in a series of assays using altered MNAzymes. A plurality of MNAzyme substrates allows multiplexing to detect multiple targets in a single assay using multiple MNAzymes, one for each target. Such multiplexed methods of using MNAzymes are readily accomplished in solution or with attachment to a support system. It is contemplated herein that multiplexed assays can thus be accomplished in systems involving attaching one or more of the substrate, or the MNAzyme partzymes or assembly facilitator, or additional enzyme activities, to a support as described herein.

Similarly, the MNAzymes may be engineered to specifically hybridise to and catalytically modify certain substrates. For example, by altering only the substrate arms of the partzymes, but by leaving the sensor arms unchanged, a large variety of MNAzymes specific for a given target can be designed which recognise and catalytically modify a series of different MNAzyme substrates. The substrate may be a reporter substrate capable of providing a detectable signal upon catalytic modification by the MNAzyme.

In certain embodiments, MNAzymes of the compositions may be engineered to specifically hybridise to and catalytically modify a universal or generic substrate. Universal MNAzyme substrates may be used to allow rapid assay development by allowing facile design changes to create new MNAzymes which recognize different targets. The substrate arm portion and the catalytic core portion of the partzymes may remain unchanged, with changes only to the sensor arm portion of one or more partzymes required for new targets. Universal substrate sequences are provided and thus the same substrate can be incorporated in assays for many different targets. Further, the same substrate can be incorporated into the methods in various embodiments herein, including assays where the substrate is free in solution or is tethered or attached to a support. A series of universal substrates can be used in a multiplex reaction allowing simultaneous detection of multiple targets. MNAzyme strategies using universal substrates offer a major advantage over detection technologies such as TagMan^{®} or Beacons or Hybridization probes which require the design and use of probes specific for each new target. Since the MNAzyme substrate is universal and useful for any target, cleavage of this universal MNAzyme substrate allows for the generation and amplification of a signal in the presence of any target.

DNAzymes, SubZymes, ribozymes, partzymes, assembly facilitator oligonucleotides / polynucleotides and/or MNAzyme substrates included in compositions of the present invention may comprise an aptamer which is capable of binding to a target analyte. Preferred aptamers may comprise short single-stranded DNA or RNA oligomers or peptides that can be isolated from complex libraries of synthetic nucleic acids or peptides by an iterative process of adsorption, recovery, and re-amplification. Aptamers may therefore be generated against almost any target analyte, ranging from small molecules such as amino acids or antibiotics, to protein and nucleic acid structures. In preferred embodiments, aptamers include, for example, nucleic acid binding molecules which are preferably generated by evolution and selection techniques. The aptamers may comprise DNA molecules, RNA molecules or a combination of both including, but not limited to, the nucleotide analogues as per, for example, **Table** 1 above.

Strategies for combining the use of aptamers with ribozymes or DNAzymes are known in the art. Such molecules are generally chimeric and contain both the aptamer domain and DNAzyme or ribozyme domain, and are activated by the presence of the target ligand. The aptazyme functional activity may be switched on in response to the aptamer domain binding to its analyte. Strategies for generating aptazymes include, but are not limited to, the fusion of the ribozyme or DNAzyme and the aptamer domains together via a communication domain. The communication domain can be evolved via *in vitro* selection methods to improve its ability to allow for ribozyme or DNAzyme activity only in the presence of the target analyte. Another exemplary strategy involves the incorporation of the aptamer into a non-functional stem loop or hairpin that merely plays a structural role in the ribozyme or DNAzyme. Aptamers may also be linked to a DNAzyme or ribozyme and both the aptamer domain and enzyme domain may be partially hybridized to a regulator oligonucleotide, which is used to inhibit the catalytic activity of the enzyme domain in the absence of the analyte. In the presence of the analyte, the aptamer can bind to the analyte, releasing the regulator oligonucleotide from the enzyme domain and restoring its catalytic activity. In this case, the presence of the analyte may remove the regulator oligonucleotide from the DNAzyme or ribozyme and restore its catalytic activity. Aptamers can also be used to bridge two or more components of a DNAzyme or ribozyme together such that the enzyme is then capable of modifying its substrate. A unique class of DNAzymes also exists that contains an aptamer for hemin and in its presence can mimic the activities of peroxidase, catalysing various chemical substrates to generate fluorescent, chemiluminescent, and colorimetric signals.

Strategies for combining the use of aptamers with MNAzymes are also known in the art. Aptazymes which contain MNAzyme components linked to aptamer may also be referred to as Apta-MNAzymes. For example at least one partzyme of a MNAzyme may incorporate an aptamer (an apta-partzyme) as well as a complementary sequence capable of forming a hairpin and therefore inhibiting MNAzyme assembly. An analyte or target to be detected may bind to the apta-partzyme, thus enabling assembly of an active MNAzyme. In the absence of a target analyte the apta-partzyme adopts a hairpin structure which inhibits assembly of an active MNAzyme. In the presence of target analyte, the target analyte binds to the aptamer domain of the apta-partzyme, thus disrupting the hairpin structure and allowing the apta-partzyme to participate in assembly of an active MNAzyme.

In other embodiments the aptamer may be present as part of an assembly facilitator oligonucleotide that incorporates an aptamer as well as complementary inhibitor sequence capable of forming a hairpin structure. In the absence of a target analyte, the assembly facilitator oligonucleotide adopts a hairpin structure which inhibits the ability of this component to direct the assembly of active MNAzymes. In the presence of target analyte, the target analyte binds to the aptamer domain of the assembly facilitator, thus disrupting the hairpin structure and allowing the component to direct the assembly of a catalytically active MNAzyme. One skilled in the art will appreciate that the aptamer may be incorporated into either end of the assembly facilitator molecule or molecules. Further it will be appreciated that multiple aptamers could be incorporated into one or more of the partzyme oligonucleotide components.

In further embodiments an aptamer sequence may be incorporated at the end of a partzyme (forming an 'apta-partzyme') in a configuration whereby an active initiating Apta-MNAzyme is only formed in the presence of the target analyte. In this case the partzymes required for the detection strategy include; a standard partzyme; an apta-partzyme which is a partzyme with an aptamer incorporated into one of its ends; an assembly facilitator which binds to both the apta-partzyme and the partzyme enabling assembly of an active initiating Apta-MNAzyme (in the presence of target analyte); a substrate; and an assembly inhibitor which hybridises to the apta-partzyme in a region which spans at least part of the aptamer sequence and part of the substrate binding arm of the partzyme sequence. In the absence of a target the assembly inhibitor oligonucleotide binds to the apta-partzyme preventing cleavage of the reporter probe substrate. In the presence of a target, the target binds to the aptamer sequence of the apta-partzyme, preventing the binding of the assembly inhibitor and allowing the binding and cleavage of the MNAzyme substrate by the initiating Apta-MNAzyme. As such, an active initiating Apta-MNAzyme can only form and modify a MNAzyme substrate in the presence of target analyte.

It will also be appreciated by persons skilled in the art that one or more aptamers may be incorporated into any of the oligonucleotide components, including the partzyme oligonucleotide/s, the assembly facilitator oligonucleotide or the MNAzyme substrate.

Catalytic nucleic acid enzymes and/or components thereof (e.g. DNAzymes, ribozymes, MNAzymes, partzyme oligonucleotides, assembly facilitator oligonucleotides, substrates, and/or aptazymes) in compositions of the present invention may be provided as a component of a molecular complex hybridised with other molecule(s) by complementary base pairing.

In some embodiments, the compositions may comprise all of the components necessary to activate a molecular switch as described herein, with the exception of a co-factor necessary for the catalytic function of an initiator catalytic nucleic acid enzyme and/or a catalytic nucleic acid enzyme of the switch (e.g. divalent metal ions such as, for example, Ba²⁺, Sr²⁺, Mg²⁺, Ca²⁺, Ni²⁺, Co²⁺, Mn²⁺, Zn²⁺, and/or Pb²⁺, and/or monovalent cations).

### - SubZyme Oligonucleotides

Compositions of the present invention may comprise one or more SubZyme oligonucleotides. In general, a subZyme oligonucleotide according to the present invention comprises at least one catalytic nucleic acid or a portion thereof (e.g. a partzyme oligonucleotide), and at least one catalytic nucleic acid substrate. In a number of embodiments, the catalytic nucleic acidor portion thereof in a subZyme oligonucleotide is unable to catalytically modify the catalytic nucleic acid substrate component of the same SubZyme oligonucleotide.

The catalytic nucleic acid or portion thereof, and the catalytic nucleic acid substrate may be arranged consecutively on the subZyme oligonucleotide without intervening sequence or linker/s. Accordingly, they may constitute an uninterrupted sequence along the subZyme oligonucleotide.

Alternatively, the catalytic nucleic acid or portion thereof, and catalytic nucleic acid substrate of the subZyme oligonucleotide may be separated, for example, by intervening nucleotides and/or by any suitable linker or spacer molecule available in the art.

No particular limitation exists in relation to the number of intervening nucleotides, which may be more than one nucleotide, more than two nucleotides, more than three nucleotides, more than four nucleotides, more than five nucleotides, more than ten nucleotides, more than fifteen nucleotides, more than twenty nucleotides, less than two nucleotides, less than three nucleotides, less than four nucleotides, less than five nucleotides, less than ten nucleotides, less than fifteen nucleotides, less than twenty nucleotides, one nucleotide, two nucleotides, three nucleotides, four nucleotides, five nucleotides, ten nucleotides, fifteen nucleotides, or twenty nucleotides.

No particular limitation exists in relation to the particular form or characteristics of a linker or spacer that may be present in a SubZyme oligonucleotide separating a catalytic nucleic acid or portion thereof, and a catalytic nucleic acid substrate of the SubZyme oligonucleotide. Spacers and linkers can be used to provide varying amounts of separation between the nucleic acid substrate and catalytic nucleic acid components. Examples include, but are not limited to, carbon chains, polyethylene glycols (PEG), poly-A, poly-T, abasic furans, abasic oligonucleotides or photocleavable PC modifications.

Linkers and spacers can also be incorporated between an oligonucleotide and a surface to which it is attached. No particular limitation exists in relation to the particular form or characteristics of a linker or spacer separating a given SubZyme oligonucleotide from a given surface. By creating greater distance, the linkers and spacers can mitigate steric or charge affects. Examples of linkers and spacers that can be used between SubZyme oligonucleotides and solid surfaces include, but are not limited to, carbon, dextran, TEG, PEG, poly-A tail, poly-T tail, PNA, DNA or LNA.

A SubZyme oligonucleotide may comprise deoxyribonucleotide and/or ribonucleotide bases, and/or analogues, derivatives, variants, fragments, linkers, spacers or combinations thereof.

SubZymes can be attached to surfaces using a variety of different attachment chemistries. There are a range of oligonucleotide modifications that enable immobilization of SubZymes onto surfaces including, but not limited to, the following oligonucleotide functional groups amine, carboxyl, disulfide, hydrazide, thiol, acrydite, biotin, NHS ester (azide), cholesterol TEG, digoxigenin, aminooxy, adenylation, desthiobiotin, hexynyl, maleimide, alkyne, dithiol, octadiynyl, aldehyde and epoxy. Alternatively SubZymes may be ligated to, or hybridized with, other oligonucleotides already immobilized on a surface.

The SubZyme oligonucleotides may optionally comprise or be attached to additional molecule/s which may, by way of non limiting example, aid in its separation or purification (e.g. from a reaction mix). These may include by way of non-limiting example magnetic, latex, glass, agarose or silica beads. In some embodiments of the invention and by way of non-limiting example, a SubZyme oligonucleotide may comprise a 10-23 DNAzyme and a substrate for an 8-17 DNAzyme, or an 8-17 DNAzyme and a substrate for a 10-23 DNAzyme. In other embodiments of the invention a SubZyme oligonucleotide may comprise a partzyme oligonucleotide and a substrate for an 8-17 DNAzyme, or a partzyme oligonucleotide and a substrate for a 10-23 DNAzyme.

The subZyme oligonucleotide may optionally comprise or be attached to additional molecule/s which may, by way of non limiting example, aid in the detection using detection systems including, but not limited to, electrochemical, fluorescent, colorimetric, pH, SPR, magnetic resonance and luminescence. Non-limiting examples of additional molecules include magnetic beads, gold particles, silver particles, enzymes, fluorophores, chemical groups, quenchers and quantum dots.

The subZyme oligonucleotide may optionally comprise additional sequence which is complementary to the target to be detected. In preferred embodiments the additional target-specific sequence may include nucleotides required to form a recognition site for a protein endonuclease or exonuclease. By way of non-limiting example, the additional target specific sequence may include one strand of a double stranded recognition site for a restriction enzyme.

SubZyme oligonucleotides/subZymes according to the present invention may comprise a portion of a catalytic nucleic acid (e.g. a partzyme oligonucleotide). The portion of the catalytic nucleic acid may be capable of partial or complete enzymatic activity as compared to the complete nucleic acid from which it is derived. Alternatively, the portion of the catalytic nucleic acid may not be capable of catalytic activity until combined with other portion/s of the parent catalytic nucleic acid from which it is derived (e.g. another partzyme oligonucleotide and/or assembly facilitator). In some embodiments of the invention and by way of non-limiting example, a SubZyme oligonucleotide may comprise a 10-23 partzyme oligonucleotide and a substrate for another different 10-23 DNAzyme,a 10-23 Partzyme and a substrate for an 8-17 DNAzyme, an 8-17 Partzyme and a substrate for a 10-23 DNAzyme, or an 8-17 Partzyme and a substrate for another different 8-17 DNAzyme.

### - Exoncleases and endonucleases

Compositions of the present invention may comprise one or more exonucleases and/or endonucleases. Non-limiting examples of suitable endonucleases include restriction endonucleases, Mung Bean nuclease, Endonuclease IV *(E. coli),* RNase A, RNase I *(E. coli),* RNase III *(E. coli)* or RNase H *(E. coli).* Non-limiting examples of suitable exonucleases include Exonuclease I *(E. coli),* Exonuclease III *(E. coli),* Exonuclease VII and T7 Exonuclease. The endonucleases may be restriction enzymes that recognize and cleave both strands of a double stranded nucleic acid duplex, or may be Nicking enzymes that recognize a double stranded nucleic acid duplex but only cleaves only strand.

### - Oligonucleotides

Oligonucleotides included in compositions of the present invention, such as DNAzymes, SubZyme oligonucleotides, ribozymes, aptazymes, MNAzymes, their respective substrates, and MNAzyme components (e.g. partzyme oligonucleotides, assembly facilitator oligonucleotides) may contain one or more substitutions such as analogues (e.g. those listed in **Table** 1), derivatives, modified or altered bases, ribonucleotides, alterations of the sugar or phosphate backbone, various deletions, insertions, substitutions, duplications or other modifications, or any combination of these, well known to those skilled in the art. Such modifications, substitutions, deletions, insertions, etc may be made at any position provided the oligonucleotide retains its function. Substitutions and modifications to the oligonucleotides may be well tolerated and allow tailoring of the molecules to function under certain conditions or for improvement of the efficiency of reaction. The skilled addressee will appreciate that the DNAzymes, ribozymes, SubZyme oligonucleotides and their substrates, MNAzymes and their substrates, and MNAzyme components (e.g. partzymes, assembly facilitator oligonucleotides/polynucleotides) may comprise either deoxyribonucleotides or ribonucleotides, or both. The oligonucleotides may comprise at least one deoxyribonucleotide, and may in some cases consist of deoxyribonucleotides and/or analogues thereof.

### - Catalytic Nucleic Acid Substrates

Compositions of the present invention comprise substrates capable of modification by catalytic nucleic acid enzymes that are DNAzymes. In some embodiments, compositions of the present invention comprise substrates capable of modification by catalytic nucleic acid enzymes such as ribozymes, aptazymes and/or MNAzymes or apta-MNAzymes.

The substrate may be any single- or double-stranded polymer of deoxyribonucleotide or ribonucleotide bases, or analogues, derivatives, variants, fragments or combinations thereof, which is capable of being recognized, acted upon or modified by an enzyme including a catalytic nucleic acid enzyme.

The substrate may be modified by various enzymatic activities including but not limited to cleavage or ligation, wherein modification of the substrate by the enzyme may provide a detectable effect indicative of the catalytic activity of the enzyme.

Substrates for nucleic acid enzymes may also comprise or be attached to non-nucleic acid constituents such as, for example, an amino acid, peptide or protein or any chemical constituent outlined in Table 6.1 of ("New strategies in Chemical synthesis and Catalysis", B. Pignataro in Wiley-VCH, 2012).

The substrate may be a reporter substrate comprising one or more features to facilitate the quantification and/or detection of a modified form of the substrate arising due to the catalytic activity of an enzyme. Reporter substrates can be free in solution or bound (or "tethered"), for example, to a surface, or to another molecule. A reporter substrate can be labelled by any of a large variety of means including, for example, fluorophores (with or without one or more additional components, such as quenchers), radioactive labels, gold and/or silver particles, biotin (e.g. biotinylation) or chemiluminescent labels. These various labels may provide a means of generating a detectable signal upon modification (e.g. cleavage) by a catalytic nucleic acid enzyme.

The substrate may be a universal substrate that is recognized by and acted on catalytically by a plurality of catalytic nucleic acid enzymes. The universal substrate may be tethered to a solid support. Substrates included in compositions of the present invention may be provided as discrete components capable of recognition and modification by a catalytic nucleic acid. Other aspects of the present invention relate to substrates that can be recognized and cleaved by more than one catalytic nucleic acid. By way of example, a single nucleic acid substrate may be cleavable by both a DNAzyme and an MNAzyme provided the substrate binding arms of the DNAzyme, and the substrate binding arms of the partzyme components of the MNAzyme, are both complementary to the same said single nucleic acid substrate. In such cases the specific catalytic core sequence of the DNAzyme, and the catalytic core portions of the partzyme pair of the MNAzyme, may be compatible with cleavage at a "cleavage" site within the substrate.

### - Selectively Permeable Barriers

Compositions of the present invention comprise a selectively permeable barrier for the separation of various components for use in the methods described herein. By virtue of its properties, the selectively permeable barrier will generally allow the passage of certain assay components through it while preventing others from doing so. The selectively permeable barrier may allow two-way passage of components for use in the methods described herein.

By way of non limiting example only, the selectively permeable barrier may serve to separate any one or more of the following assay component/s from one or more other assay component/s: MNAzymes, MNAzyme component/s (e.g. assembly facilitator oligonucleotides, partzyme oligonucleotides), MNAzyme substrates, DNAzymes, DNAzyme substrates, ribozymes, ribozyme substrates, SubZyme oligonucleotides.

In certain embodiments, the selectively permeable barrier may serve to physically separate different types of SubZyme oligonucleotides from one another. In some embodiments the selectively permeable barrier is used to physically separate a first SubZyme oligonucleotide from a non-identical second SubZyme oligonucleotide.

The first SubZyme oligonucleotide may comprise a catalytic nucleic acid enzyme sequence ('cat A') and a component that is a substrate for a catalytic nucleic acid enzyme ('sub A'). The second SubZyme oligonucleotide may comprise a catalytic nucleic acid enzyme sequence ('cat B') and a component that is a substrate for a catalytic nucleic acid enzyme ('sub B'). Cat A may be incapable of catalytically modifying (e.g. cleaving) sub A, and may be capable of catalytically modifying (e.g. cleaving) sub B. Cat B may be incapable of catalytically modifying (e.g. cleaving) sub B, but may be capable of catalytically modifying (e.g. cleaving) sub A.

In other embodiments, a SubZyme oligonucleotide may comprise a portion of a catalytic nucleic acid enzyme (e.g. a partzyme oligonucleotide), and a component that is a substrate for a catalytic nucleic acid enzyme. The portion of the catalytic nucleic acid enzyme may not be capable of hybridising to the substrate of the SubZyme oligonucleotide.

The selectively permeable barrier may achieve the physical separation of assay components based on properties of the barrier including, but not limited to, those which facilitate charge-based separation, size-based separation, shape-based separation, weight-based separation, separation based on lipid solubility, separation based on molecule mobility and/or separation based on affinity to carrier molecules present in the membrane.

In some embodiments, the selectively permeable barrier can achieve physical separation of assay components by virtue of size restriction. For example, the barrier may comprise pores of a certain maximum size that prevents specific assay components from moving through the barrier. For example, an assay component such as a SubZyme oligonucleotide may comprise a physical feature (e.g. a secondary structure arising from a region of self-complementarity) and/or may be attached to an entity (e.g. a bead or microparticle) that is too large to move through the pores of the barrier.

In other embodiments, the selectively permeable barrier can achieve physical separation of assay components by virtue of charge characteristics. For example, the barrier may comprise pores with a certain electrical charge (e.g. ion channels) that prevents specific assay components from moving through the barrier. For example, an assay component such as a SubZyme oligonucleotide may comprise a certain charge and/or may be attached to an entity (e.g. a bead) with a certain charge that prevents it moving through pores of the barrier with the same or similar charge.

In still other embodiments, the selectively permeable barrier can achieve physical separation of assay components by virtue of lipid solubility characteristics. For example, the barrier may comprise lipids that prevent certain assay components from moving through the barrier if they are lipophobic. For example, an assay component such as a SubZyme oligonucleotide may comprise lipophobic elements and/or may be attached to an entity (e.g. a bead) with lipophobic characteristics that prevents it moving through the lipophilic barrier.

In still other embodiments, the selectively permeable barrier can achieve physical separation of assay components by virtue of shape restriction. For example, the barrier may comprise pores of a certain shape that prevents specific assay components from moving through the barrier. For example, an assay component such as a SubZyme oligonucleotide may comprise a physical feature (e.g. a secondary structure arising from a region of self-complementarity) and/or may be attached to an entity (e.g. a bead or microparticle) of a shape that is incompatible with movement through the pores of the barrier.

In further embodiments, the selectively permeable barrier can achieve physical separation of assay components by virtue of affinity to carrier molecules present within the barrier. For example, the barrier may comprise carrier molecules that facilitate the transport of specific assay components through the barrier, but not others. For example, an assay component such as a SubZyme oligonucleotide may comprise a physical feature and/or may be attached to an entity (e.g. a bead) having characteristics that facilitate attachment to a carrier molecule present in the barrier, and thereby facilitate transport of the subZyme oligonucleotide through the barrier.

The skilled person will recognise that the physical separation of assay components by the selective permeable barrier can be achieved by any standard means known in the art with no particular limitation to those specifically described herein.

The selectively permeable barrier may be made of any suitable material/s known in the art. In general, the material/s will be compatible with the working components of the assay. Non-limiting examples of suitable materials include polyethersulfone, polycarbonate, nitrocellulose, regenerated cellulose, cellulose acetate, polyamide, propylene, nylon, aluminium oxide or polytetrafluoroethylene.

### - Detectable Labels

Components within the compositions of the present invention may comprise detectable labels capable of generating output signal determined by fluorescence spectroscopy, surface plasmon resonance, mass spectroscopy, electrochemistry, NMR, electron spin resonance, polarization fluorescence spectroscopy, circular dichroism, immunoassay, chromatography, radiometry, photometry, scintigraphy, electronic methods, UV, visible light or infra red spectroscopy, enzymatic methods or any combination thereof.

Nanoscopic and microscopic gold particles attached to oligonucleotides can be used to generate colourimetric signals whereby gold particles in close proximetery appear blue and when separated appear red. For, example separation may be achieved by cleavage of a substrate or SubZyme by an MNAzyme or DNAzyme. Oligonucleotides can be labelled with fluorophore and quencher dyes which when separated can result in an increase in florescence. For, example separation may be achieved by cleavage of a substrate or SubZyme by an MNAzyme or DNAzyme.

Oligonucletides may be labelled with gold or silver particles at one end and attached to a sensor surface at the other end to facilitate measurement by surface plasmon resonance. For example, an attached particle may be released from the surface of the sensor by cleavage of a substrate or SubZyme by an MNAzyme or DNAzyme resulting in a detectable shift in surface plasmon resonance. Oligonucleotides can be attached to a surface at one end and attached to enzymes involved in electrochemical reactions at the other end. Release of the enzyme from the surface so that it can partake in an electrochemical reaction may be achieved by cleavage of a substrate or SubZyme by an MNAzyme or DNAzyme.

### Detection and Signal Amplification Methods

The present invention provides various methods for the detection, identification, and/or quantification of at least one target. Further, the present invention provides various cascades for the amplification of signals generated by these methods.

The methods utilise compositions of the present invention and components thereof. In many embodiments the methods include signal amplification cascades.

### - Exemplary Assay According to the Present Invention

An exemplary scheme according to embodiments of the present invention is illustrated in **Figure 4****.** In this scheme the selectively permeable barrier is referred to as a 'T-bag' which encloses and physically separates certain assay components from others. SubZymes are used to amplify a signal following detection of the target. Exemplary Subzyme pairs are illustrated in **Figures 1** and **2** and an exemplary T-bag manufacture process is illustrated in **Figure 3****.** Both SubZyme species, PA (SubZyme A-MB) and PB (SubZyme B-MB), are attached to a Magnetic Bead (MB) which is too large to pass though the selectively permable barrier/T-bag. Though the SubZymes are attached to MBs they are free in solution and mobile within separate compartments of the assay defined by the T-bag. The reaction comprises SubZyme A-MB which is on one side of the permaeable barrier outside the T-bag and SubZyme B - MB on the other side of the permaeable barrier within the T-bag. In addition, partzyme oligonucleotides which are complementary to the target to be detected and to the substrate sequence within SubZyme A-MB are present outside the T-bag. In the presence of the target, the partzyme oligonucleotides align and form a Cat C (e.g. an MNAzyme) which cleaves the Sub A present within SubZyme A-MB resulting in release of the Cat A from the MB which allows Cat A to migrate through and into the T-bag. Within the T-bag released Cat A can cleave the Sub B within SubZyme B-MB. As a result the Cat B is separated from the MB. The Cat B is free to migrate from inside the T-bag into solution outside of the T-bag where Cat B can cleave Sub A in SubZyme A-MB and initiate a cascade reaction. In this scenario one target molecule can initiate a reaction by hybrizing with partzyme oligonucleotides to form one MNAzyme which can cleave a first SubZyme, capable of cleaving a second SubZyme, which in turn is capable of cleaving more of the first SubZyme and initiating a cross catalytic cascade reaction wherein many of the first and second subZymes are cleaved to release many DNAzymes following the single initiating event.

After a period of incubation uncleaved SubZyme-MB and cleaved MB fragments can be separated from released DNAzymes (Cat A and Cat B). As illustrated in **Figure 4****,** this can be achieved, for example, by using a magnet to separate uncleaved SubZyme-MB and cleaved MB fragments from DNAzymes (Cat A and Cat B) present in the supernatant. These DNAzymes may be transferred to another tube or compartment containing labelled Substrate A and/or B which the DNAzymes can cleave. The fluorescence which is generated following separation of the fluorophore and the quencher by DNAzyme cleavage can be monitored in real time.

As depicated in **Figure 2****,** preferred embodiments may comprise SubZyme pairs incorporating different types of DNAzymes and different types of substrate sequences. For example, a SubZyme pair useful in the following invention may comprise a SubZyme A (PA) encompassing an 8-17 DNAzyme and a 10-23 substrate for use with a SubZyme B (PB) encompassing a 10-23 DNAzyme and a 8-17 substrate; or *vice versa.* Since the 10:23 and 8:17 DNAzymes have different requirements for specific sequence at the cleavage sites SubZymes with one type of DNAzyme and another type of substrate have no potential for self-cleavage even when stored or incubated under low stringency conditions. The Subzyme pair can however cleave each other in a cross catalytic manner as depicted in **Figure 2****.** The SubZymes may be either unlabelled, or labelled which allows signal generation upon SubZyme cleavage which can be monitored in real time or at the end of the reaction. The use of labelled substrates within SubZymes abrogates the need for transfer of released DNAzymes to a second tube or compartment containing DNAzyme substrates with a sole function of generating a signal indicative of the presence of the target to be detected. Substrate sequence(s) present within SubZymes may be suitable for cleavage by a DNAzyme and/or an MNAzyme. By way of non-limiting example, a SubZyme may be cleavable by a DNAzyme and an MNAzyme which was originally derived by splitting a DNAzyme.

### - Cascade inititation

The skilled person will recognize that the signal amplification cascades of the present invention may be used to detect nucleic acid targets. The mechanism of initiation of the cascade in the presence of target can also vary. By way of non-limiting example and with reference to Figure 13 two exemplary pairs of SubZymes capable of cross-catalysis are illustrated together with alternative modes of reaction initiation. Both SubZyme pairs, denoted as polynucleotide A (PA) & polynucleotide B (PB), are depicted as being attached to optional magnetic beads (MB) which can be used to manipulate the SubZymes. SubZyme A-MB (PA) comprises functional domains namely; (i) Catalytic nucleic acid A (e.g.8-17 DNAzyme A), (ii) substrate A which is cleavable by a Catalytic nucleic acid B (e.g.10-23 DNAzyme B), and optionally (iii) either linker sequence (**Figure 13A**) or sequence which is complementary to a target nucleic acid (**Figure 13B**) and which additionally comprises one strand of a endonuclease recognition site for aretriction enzyme or Nicking enzyme. SubZyme B-MB (PB) comprises functional domains namely; (i) Catalytic nucleic acid B (e.g. 10:23 DNAzyme B), (ii) substrate B which is cleavable by a Catalytic nucleic acid A (e.g. 8-17 DNAzyme A), and optionally (iii) linker sequence (**Figures 13A** and **13B**).

In the scheme shown in **Figure 13A****,** the presence of a target nucleic acid results in the formation of Catalytic nucleic acid C (MNAzyme C) capable of cleaving Sub A A. Cleavage of Sub A within PA results in release of the Cat A which can subsequently move through a selectively permeable barrier and cleave Sub B within PB to release Cat B. The skilled person will recognise that while the cleavage of PA by the MNAzyme (Cat C) is shown in **Figure 13A** to release Cat A from a mobile support, cleavage of PA by the MNAzyme may alter other characteristic(s) of PA including, but not limited to charge, size, shape, weight, lipid solubility, and so on, any of which may confer a capacity to permeate the selectively-permeable barrier. Cat B can in turn move through a selectively permeable barrier and cleave a new Sub A within another PA and release more Cat A, thus initiating a cross catalytic a signal amplification cascade.

In the exemplary scheme in **Figure 13B****,** the target nucleic acid hybridizes to specific sequence (iii) (refer to uppermost diagram) in PA thus creating double stranded duplex region which forms a recognition and cleavage site for a Catalytic Enzyme D (e.g. a nuclease such as a restriction enzyme (RE) or a nicking enzyme (NE)). An RE could be used to cleave both strands of the target-PA duplex whereas an NE could be used to cleave the PA strand of the target-PA duplex. The sequence (iii) in PA to which the target hybridises may be located between the DNAzyme A substrate sequence and the MB (as illustrated) such that cleavage by Catalytic Enzyme D at the cleavage site formed results in release of Cat A. The skilled addressee will recognise that other arrangements could be utilised such as, for example, where the sequence (iii) in PA to which the target hybridises is located between the Sub A sequence (ii) and the Cat A, or alternatively where the sequence (iii) in PA to which the target hybridises is located between the Cat A and the MB such that cleavage by the Catalytic Enzyme D at the cleavage site formed results in release of Cat A. The skilled addressee will recognise that forming the Catalytic Enzyme D cleavage site does not preclude a component of the target nucleic acid from also hybridising to some or all of the Sub A sequence of PA, or some or all of the Cat A sequence of PA. Cleavage of PA by the Catalytic Enzyme D can modify PA (depicted in **Figure 13B** as release of Cat A from a mobile support) in a manner that allows it to permeate through a selectively permeable barrier. The skilled person will recognise that while the cleavage of PA by the Catalytic Enzyme D (here an endonuclease) is shown in **Figure 13B** to release Cat A from a mobile support, the cleavage of PA may alter other characteristic(s) of PA including, but not limited to charge, size, shape, weight, lipid solubility, and so on, any of which may confer a capacity to permeate the selectively-permeable barrier. The released Cat A permeates the membrane and comes into contact with and cleaves Sub B within PB. Cleavage of Sub B within PB releases Cat B which can in turn move through the selectively permeable barrier and cleave a Sub A within another PA to release more Cat A, thus initiating a cross catalytic signal amplification cascade.

The skilled person will recognise that there are many other enzymes in addition to restriction enzymes and their subtype nicking enzymes which could cleave PA following hybridization of a target to an optional target specific region within PA. By way of non-limiting example, Ribonuclease H (RNAse H) could be employed to initiate the cascade if PA contained, for example, four or more ribonucleotides in the target specific region. RNAse H is a non-sequence specific endoclease enzyme that can catalyze the cleavage of RNA in an RNA/DNA duplex. Formation of target SubZyme hybrids could provide the RNA/DNA duplexes that are cleavable by RNAse H enzymes, whilst leaving the other single stranded regions of the SubZyme and the target intact.

As a second non-limiting example, duplex-specific nuclease (DSN) could be employed to initiate the cascade in the presence of RNA or ssDNA targets. DSN is a nuclease enzyme that shows a strong preference for cleaving DNA in DNA-RNA duplexes and double stranded DNA, yet, it is inherently inactive towards single stranded DNA, single stranded RNA and double stranded RNA. Formation of target and SubZyme hybrids could provide the RNA/DNA duplexes that are cleavable by DSN enzymes, whilst leaving the other single stranded regions of the SubZyme and the target intact.

The skilled person will recognise there are many other enzymes in addition to endonucleases such as restriction enzymes and their subtype nicking enzymes which may be used to cleave the PA polynucleotide following hybridization with a target. By way of non-limiting examples exonucleases such as exonuclease III or T7 could be employed to initiate the cascade. Exonuclease III enzyme removes nucleotides from 3' termini of blunt or recessed termini or DNA duplexes, but is not active on single stranded DNA. T7 Exonuclease removes nucleotides from the 5' end of DNA duplexes or DNA/RNA duplexes. Formation of target subZyme hybrids could provide duplexes that are cleavable by exonuclease III or T7, whilst leaving the other single stranded regions of the subZyme intact.

### - Further exemplary embodiments

In the exemplary scheme depicted in **Figure 22** permeable barriers (referred to as 'T-bags') and SubZymes are used to amplify a signal following detection of the target. This strategy is distinct from the scheme illustrated in **Figure 4** in that two different types of SubZymes are employed. PA (SubZyme A) comprises a Cat A (8-17 DNAzyme A) linked to a substrate (Sub A) which is cleavable by a Cat B and Cat C (a 10-23 MNAzyme). PB (SubZyme B1) comprises a Cat B1 (a 10:23 Partzyme B1) conjugated to a substrate (Sub B) which is cleavable by Cat A (8-17 DNAzyme A). PC comprises a Cat B2 (a 10:23 Partzyme B2). The tube contains PA (SubZyme A-MB) attached to a MB (magnetic bead), PC, Assembly facilitator AF-B3, Partzymes C1 and C2 for the chosen target, and PB attached to an MB (SubZyme B2-MB) inside a T-bag. In the presence of the target, the partzymes C1 and C2 align and form an initiating MNAzyme (Cat C). The MNAzyme cleaves Sub A within PA separating Cat A (8-17 Dz) from the MB which allows it to migrate through the T-bag wall where it can cleave Sub B within PB thus separating the Partzyme Cat B1 from the MB. Cat B1 is now free to migrate from inside the T-bag out into solution where it can hybridize with Cat B2 (PC) and Assembly Facilitator AF-B3 to form an active Feedback MNAzyme (Cat B). The Cat B can cleave Sub A in PA and continue the cascade. After a period of incubation a magnet can be used to retain uncleaved SubZyme A-MB and cleaved MB fragments thus allowing the supernatant containing the free DNAzymes A and Partzymes B1 and B2, PC, AF-B3, Partzymes C1 and C2 and target to be transferred to another tube containing labelled substrate A and/or Sub B. The fluorescence which is generated following separation of the fluorophore and the quencher by DNAzyme and/or MNAzyme cleavage of labelled substrates can be monitored in real time.

In the exemplary scheme depicted in **Figure 23** permeable barriers (referred to as 'T-bags') and SubZymes are used to amplify a signal following detection of the target. This strategy is distinct from the scheme illustrated in **Figure 4** in that two different types of SubZymes are employed. PA (SubZyme A) comprises Cat A (an 8-17 DNAzyme A) linked to a substrate (Sub A) which is cleavable by a Cat B and Cat C (a 10-23 MNAzyme). PB (SubZyme B1 comprises a Cat B1 (a 10:23 Partzyme B1) conjugated to Sub B (an 8-17 substrate) which is cleavable by the Cat A (8-17 DNAzyme A).. PC comprises a Cat B2 (a 10:23 Partzyme B2) conjugated to the same Sub B which is cleavable by Cat A. PA, PB and PC are optionally conjugated to beads eg magnetic beads (MB) as depicted. The tube contains PA (SubZyme A-MB); PC (SubZyme B2-MB), Assembly Facilitor AF-B3, Partzymes C1 and C2 for the chosen target which are free in solution and PB (SubZyme B1-MB) inside a T-bag constructed as outlined in **Figure 3B****.** In the presence of the target, the partzymes C1 and C2 align and form an initiating MNAzyme C (Cat C). The MNAzyme C cleaves Sub A within PA separating the Cat A from the MB which allows it to migrate through the T-bag wall where it can cleave Sub B within PB thus separating the Cat B1 (partzyme B1) from the MB. Cat A can also cleave Sub B of PC to release Cat B2 (partzyme B2) from the MB. Partzyme B1 is now free to migrate from inside the T-bag out into solution where it can hybridize with Partzyme B2 and Assembly Facilitator AF-B3 to form an active Feedback MNAzyme (Cat B). Cat B can cleave Sub A in PA and continue the cascade. After a period of incubation a magnet can be used to retain uncleaved SubZyme -MB and cleaved MB fragments thus allowing the supernatant containing the free DNAzyme A (Cat A), partzymes C1, C2, B1 and B2, AF-B3, and target to be transferred to another tube containing labelled Sub A and/or Sub B. The fluorescence which is generated following separation of the fluorophore and the quencher by DNAzyme and/or MNAzyme cleavage can be monitored in real time.

In the exemplary scheme depicted in **Figure 27****,** permeable barriers (referred to as 'T bags') and SubZymes are used to amplify a signal following detection of the target. This strategy is distinct from the schemes illustrated in Figure 4, Figure 22 and Figure 23 in that the fluorescent labelled substrate (Sub A-FQ) is present within the reaction tube during the initial incubation, thereby simplifying the reaction into a single step and negating the need to transfer released DNAzymes into a second tube. PA (SubZyme A) comprises Cat A linked to a Sub A which is cleavable by a Cat B and Cat C. PB (SubZyme B1) comprises a Cat B1 (Partzyme B1) conjugated to Sub B which is cleavable by the Cat A. PC (SubZyme B2) comprises a Cat B2 (Partzyme B2) conjugated to the same Sub B which is cleavable by Cat A. PA, PB and PC are conjugated to beads e.g. magnetic beads (MB) as depicted. Sub A-FQ comprises Sub A which is 5' labelled with a Fluorophore and 3' labelled with a quencher and is cleavable by Cat B and Cat C. Physical separation of the PB (Cat B1) from the PC (Cat B2), through employment of a permeable barrier (T bag) inhibits the formation of an active MNAzyme B (Cat B) with an assembly facilitator B3 (AF-B3) so that it cannot hydrolyze Sub A-FQ. This permits the presence of Sub A-FQ within the reaction to enable incubation and detection steps to be combined in a single step. Sub A-FQ can only be cleaved by initiating MNAzyme (Cat C) in the presence of target and by MNAzyme B (Cat B) upon cleavage of PB and release of Cat B2 from inside the T bag. The tube contains Sub A-FQ, PA, PC, AF-B3, Partzymes C1 and C2 for the chosen target and PB inside a T bag constructed as outlined in Figure 3B. In the presence of the target, the partzymes C1 and C2 align and form an initiating MNAzyme C (Cat C). The MNAzyme C cleaves Sub A within PA separating the Cat A from the MB which allows it to migrate through the T bag wall where it can cleave Sub B within PB thus separating the Cat B1 from the MB. Cat A can also cleave Sub B of PC to release Cat B2 from the MB. Cat B1 is now free to migrate from inside the T bag out into solution where it can hybridize with Cat B2 and AF-B3 to form an active Cat B. Cat B can cleave Sub A in PA and continue the cascade. During incubation, fluorescence which is generated following separation of the fluorophore and the quencher within Sub A-FQ by Cat B and Cat C cleavage can be measured. In an alternate embodiment the Assembly Facilitator, for example the AF-B3 component of Cat B, may be attached to a bead, for example a MB, as an alternate to attachment of PB or PC to a bead.

### - Methods using Multiple Enzymes to analyze multiple targets

The skilled person will recognize that the methods provided herein may be used to detect a single target per reaction, or to detect multiple targets in a single reaction. When detecting multiple targets, one or more MNAzymes may be used depending on the assay and what is to be detected. For example, a single MNAzyme may suffice when detecting multiple related structures such as, for example, a group of sequences sharing a critical sequence (recognized by the MNAzyme) and varying only, for example, in length, or in sequence outside of the critical sequence. Any sequence with the critical sequence could be detected. Multiple MNAzymes are contemplated to be useful when detecting related sequences differing by as little as a single nucleotide or even where vastly different targets are to be detected, and it is desirable to know the presence or absence of each.

### - Detection of Ionic Compounds

Also described herein are methods for determining the absence of ionic compounds, detecting the presence of ionic compounds, and/or quantifying ionic compounds, in a sample (e.g. an environmental sample or a biological sample). The ionic compounds may be monovalent or divalent ions. The ionic compounds may be metal ion cofactors required for the activity of a catalytic nucleic acid enzyme.

The methods comprise providing a molecular complex as described herein (a molecular switch) and at least one additional component capable of activating the switch to thereby provide a detectable signal. The functional activity of the additional component may be reliant on the presence of the ionic compound in a sample to be tested. For example, the methods may comprise contacting a sample suspected of containing ionic compounds with a molecular switch comprising a first catalytic nucleic acid enzyme hybridised to and functionally inactivated by a blocker oligonucleotide, and an initiator catalytic nucleic acid enzyme (e.g. a DNAzyme, ribozyme, assembled MNAzyme, or components capable of assembly into an MNAzyme).

The ionic compound may additionally be a co-factor required for catalytic activity of the first catalytic nucleic acid enzyme of the complex.

Consequently the methods may utilise catalytic nucleic acids (e.g. DNAzymes, ribozymes, aptazymes and/or MNAzymes) which require a specific metal ion cofactor for catalytic activity, to detect the presence or determine the absence of the metal ion in a sample (e.g. environmental or biological samples). For example, the methods may facilitate DNAzyme-mediated detection of Pb²⁺ in an environmental sample such as water.

### - Aptamers

Also described herein are methods that may be performed with aptamers, wherein said aptamers may facilitate the detection, identification and/or quantification of targets including targets other than nucleic acids.

Methods of using MNAzymes to detect targets, including non-nucleic acid entities are described herein. Such methods may use aptamers which may comprise a nucleic acid or protein, polypeptide, or peptide or combination thereof that has the ability to recognize one or more ligands. Aptamers may bind target ligands, for example, proteins, polypeptides, peptides or nucleic acids, glycoproteins, lipids, lipoproteins, cells, viruses, bacteria, archaea, fungi, antibodies, metabolites, pathogens, toxins, contaminants, poisons, entire organisms, small molecules, polymers, metal ions, metal salts, prions or any derivatives, portions or combinations thereof, or any other entity.

Preferred aptamers herein may comprise short single-stranded DNA or RNA oligomers or peptides that can be isolated from complex libraries of synthetic nucleic acids or peptides by an iterative process of adsorption, recovery, and reamplification. Aptamers may therefore be generated against almost any target/ligand, ranging from small molecules such as amino acids or antibiotics, to protein and nucleic acid structures. In some examples, aptamers include, for example, nucleic acid binding molecules which are preferably generated by evolution and selection techniques. Aptamers may comprise DNA or RNA molecules, or a combination of both, including but not limited to the nucleotide analogues as per, for example, **Table** 1 above.

Persons skilled in the art will appreciate that the aptamer may be incorporated into a DNAzyme, ribozyme or any of the MNAzyme components. DNAzymes and ribozymes which are coupled to aptamers are known in the art as aptazymes. Such aptazymes may have their catalytic activity switched on or off by the presence of ligands with affinity to their aptamer components. Further it will be appreciated that multiple aptamers can be incorporated into one or more of the partzyme oligonucleotide components.

In further examples, an aptamer sequence may be incorporated at the end of a partzyme (apta-partzyme) in a configuration whereby an active MNAzyme is only formed in the presence of the target analyte. In this case the partzymes for the MNAzyme detection strategy include; a standard partzyme; an apta-partzyme which is a partzyme with an aptamer incorporated into one of its ends; an assembly facilitator which binds to both the apta-partzyme and the partzyme enabling assembly of an active MNAzyme (in the presence of target); a substrate; and an assembly inhibitor which hybridises to the apta-partzyme in a region which spans at least part of the aptamer sequence and part of the partzyme sequence. In the absence of a target the assembly inhibitor binds to the apta-partzyme thus blocking binding (and cleavage) of the reporter probe substrate. In the presence of a target, the target binds to the aptamer sequence of the apta-partzyme, preventing the binding of the assembly inhibitor and allowing the binding and cleavage of the MNAzyme substrate. As such, an active MNAzyme can only form and modify an MNAzyme substrate in the presence of target.

### - Methods using Insoluble Supports

It is also to be understood that generally the methods of the present invention may utilise insoluble supports on which one or more reaction component/s such as subZymes, DNAzymes, MNAzyme components (substrate, partzyme or assembly facilitator/target) are attached. For example, methods for detecting targets using an MNAzyme, whereby a reaction component (e.g. a SubZyme, DNAzyme, MNAzyme, or component/s thereof) may be anchored to a support, are contemplated herein. The support may be an insoluble material or a matrix which retains the reaction component and excludes it from freely moving in the bulk of the reaction mixture. Alternatively, the support may be an insoluble material or a matrix that retains the reaction component but otherwise allows mobility of the reaction component in the reaction mixture as it is not fixed to another surface. There are advantages in using signal amplification approaches where catalytic nucleic acid enzymes, substrates, and other components are immobilised onto mobile entities, for example beads, rather than fixed supports/surfaces. For example, beads have an increased surface area to volume ratio which permitts improved surface density of tethered components. Other advantages include miniaturisation which can facilitate (i) enhanced reaction rates by faster diffusion, and (ii) reduced reaction volumes which ultimately lowers reagent costs. Further, beads exhibit a high degree of practicality with respect to their production and incorporation into devices. A skilled person will appreciate that the support can be selected from a wide variety of matrices, polymers, and the like, in a variety of forms including beads convenient for use in microarrays, as well as other materials compatible with the reaction conditions. In certain embodiments, the support may be a plastic material, such as plastic beads or wafers, metallic material, magnetised material, or that of the well or tube in which a particular assay is conducted. In certain embodiments the support may be microcarriers or nanocarriers. In certain embodiments the support may be encoded.

### - Optimisation of Methods

The skilled person will readily understand that the methods described herein may be optimized using a variety of experimental parameters in order to enhance the detection, identification and/or quantification of a target. The particular experimental parameters that are optimized, and the level of such optimization, will depend upon the particular method being employed and the particular target being sought to be detected, identified and/or quantified. Such parameters include, but are not limited to time, temperature, pH, concentration and identity of salts and buffers, concentrations of oligonucleotides, co-factors, detergents, cations and other reagents including, but not limited to, dimethylsulfoxide (DMSO), EDTA, ATP, glycerol, length of complementarity, GC content and melting point (Tm) of nucleic acids components of MNAzymes, SubZymes, DNAzymes, substrates.

In some embodiments, for example, those methods involving detection of specific nucleic acid sequences, experimental parameters including the temperature at which the method is performed, may be optimized so as to discriminate between binding of an MNAzyme component to a target nucleic acid that does or does not comprise a sequence variation. The temperature at which such methods may be performed may be in the range of about 20°C to about 96°C, about 20°C to about 75°C, 20°C to about 60°C or about 20 to about 55°C.

In certain embodiments, optimized reactions for practicing the methods described herein are provided. In such optimized reactions, the signal detected is increased by up to 10%, 20%, or 30% above un-optimized reactions. More preferred reaction conditions may improve signal detected by at least 35%, or 40%, and preferably up to 50% or more. In other embodiments, optimized reactions may provide an increase of catalytic activity of more than 50%, and up to 66%, 75% or even 100%. In still other embodiments, a fully optimized reaction method may offer 100%, 200% or even 300% or more increase in signal detection. Other preferred reaction conditions can improve the catalytic activity by up to 1000% or more over methods practiced with unoptimized reaction conditions. A highly preferred reaction condition for optimizing the methods provided herein is the inclusion of certain divalent cations. The catalytic activity of most nucleic acid enzymes and protein nucleic acid-modifying enzymes may be influenced in a concentration-dependent fashion by the concentration of divalent cations. Preferred optimized reactions are optimized for one or more of Ba²⁺, Sr²⁺, Mg²⁺, Ca²⁺, Ni²⁺, Co²⁺, Mn²⁺, Zn²⁺, and Pb²⁺.

### Kits

Also described herein are kits for practising the methods disclosed herein. Typically, kits for carrying out the methods of the present invention contain all the necessary reagents to carry out the method.

The kits may comprise any composition according to the present invention or component(s) thereof. By way of non-limiting example only, the kits may comprise catalytic nucleic acid enzymes (e.g. MNAzymes and/or partzyme components thereof, DNAzymes, SubZymes, aptazymes and/or ribozymes). The kits may optionally contain endonucleases or exonucleases.

The kits may be fragmented kits or combined kits as defined herein. Fragmented kits comprise reagents housed in separate containers, and may include small glass containers, plastic containers or strips of plastic or paper. Such containers may allow the efficient transfer of reagents from one compartment to another compartment whilst avoiding cross-contamination of the samples and reagents, and the addition of agents or solutions of each container from one compartment to another in a quantitative fashion. Such kits may also include a container which will accept the test sample, a container which contains the reagents used in the assay, containers which contain wash reagents, and containers which contain a detection reagent. Typically, a kit will also include instructions for using the kit components to conduct the appropriate methods. Kits described herein and methods of the invention may be used in conjunction with automated analysis equipment and systems, for example, including but not limited to, real time PCR machines, spectrophotometer, electrochemical platforms or Surface Plasmon Resonance platforms.

For example, the kit may comprise a first container and second container. The first container may comprise catalytic nucleic acids and/or portions thereof (e.g. MNAzymes, DNAzymes, partzyme oligonucleotides) and/or a SubZymes). The second container may comprise catalytic nucleic acid substrates, and/or different type/s of catalytic nucleic acids and/or portions thereof, and/or different types of SubZymes, as compared to the contents of the first container). The kits may be fragmented kits or combined kits as defined herein.

The kits may also comprise one or more containers, containing for example, wash reagents, and/or other reagents as required in the performance of the methods of the invention.

For application of detection, identification or quantitation of different targets, a single kit may be applicable, or alternatively different kits, for example containing reagents specific for each target, may be required. Methods of the invention and kits described herein find application in any circumstance in which it is desirable to detect, identify or quantitate any entity.

### Examples

The present invention will now be further described in greater detail by reference to the following specific examples, which should not be construed as in any way limiting the scope of the invention.

### Example One: Materials and Methods

### - Sequences and Annotations

The Examples below refer to various nucleotide sequences, in a number of cases by sequence identification number (SEQ ID NO). The specific seqeunces utilised in the following Examples are set out in **Table 2** below.

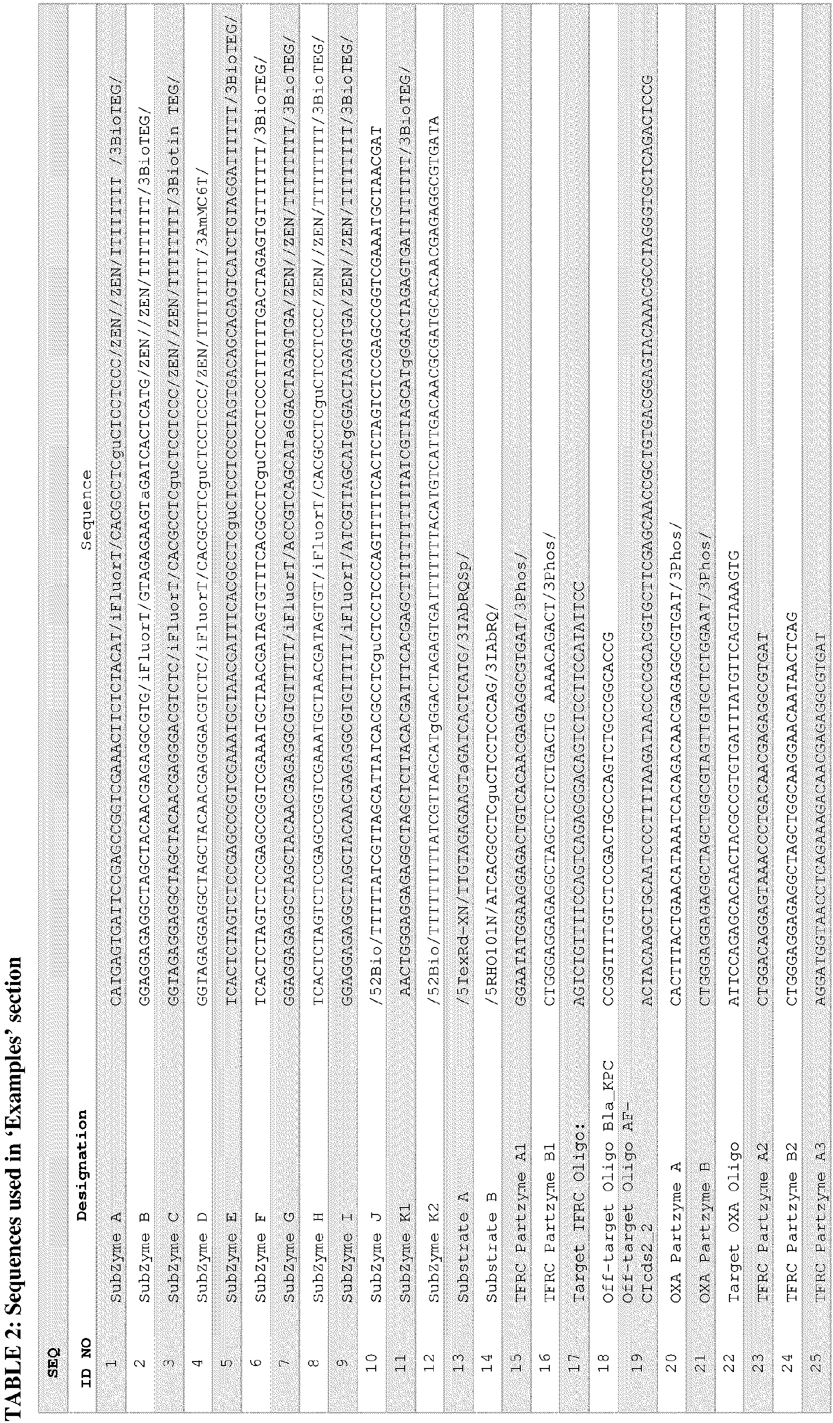

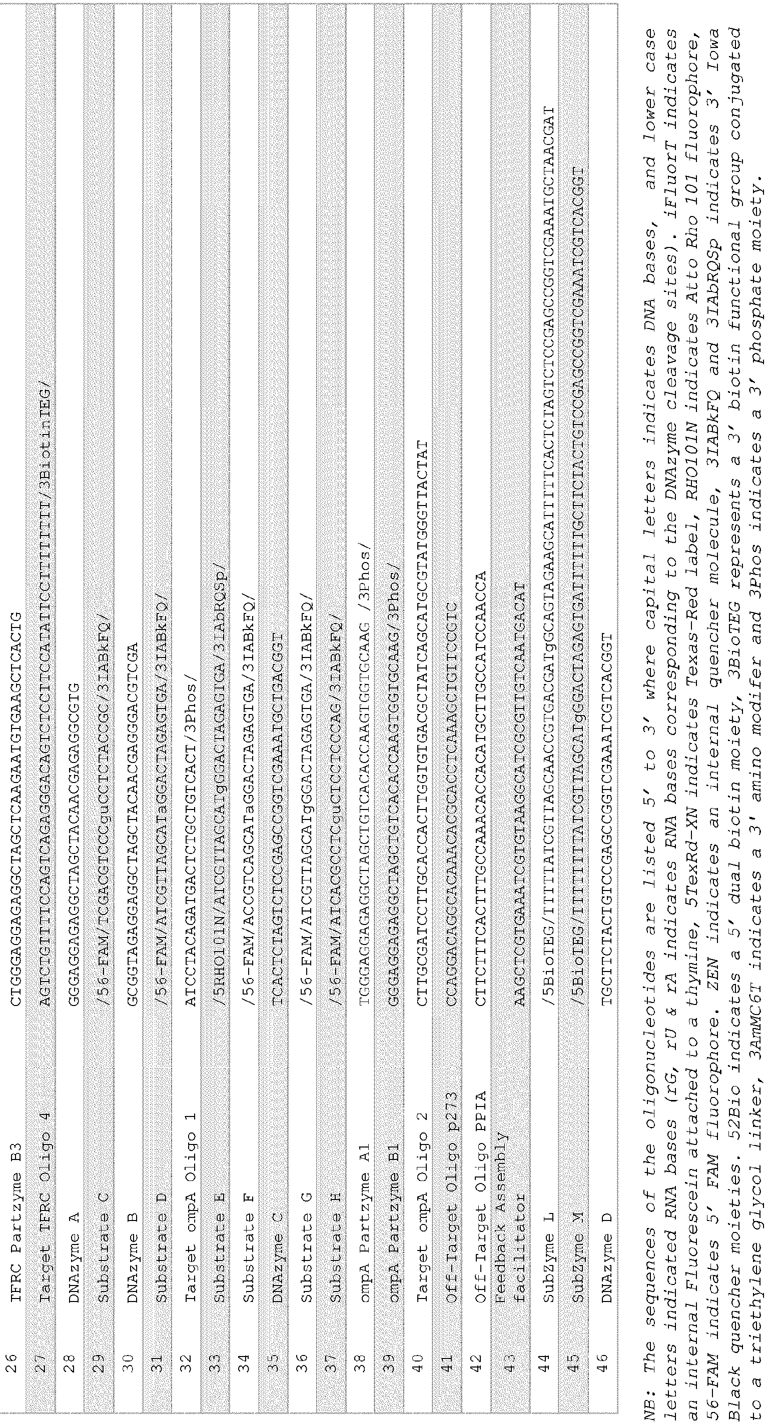

### - Reagents

Streptavidin-functionalised Dynabeads (M-270) were purchased from Invitrogen. The characteristics and properties of streptavidin-functionalised Dynabeads (M-270) provided by the manufacturer are as following: superparamagnetic, hydrophilic bead surface, 2.8 µm in diameter, size distribution (CV<3%), no blocking proteins used, isoelectric point of pH 4.5, highly charged (-50 mV at pH7), iron content of 14%, low aggregation of beads in high salt solutions.

Streptavidin functionalized BioMAG Plus beads (1.5 µm), ProMag beads (1µm), ProMag HP beads (3µm) and COMPEL Magnetic beads (6 µm and 8 µm) were purchased from Bangs Laboratories, Inc. The characteristics and properties of these beads provided by the manufacturer are as following: spherical shape, iron oxide crystals dispersed in a polymer matrix, respective magnetite contents of 26.5 wt%, 16 wt%, >90 wt%, 5.7 wt% and wt% and respective densities of 1.8 g/cm³, 1.4 g/cm³, 2.5 g/cm³, 1.1 g/cm³ and 1.1 g/cm³. All beads are supplied at 1% solids (w/v) except for the BioMAG Plus beads which are supplied at 5.0 mg/mL solids.

NaOH (5 M) was purchased from Australian Chemical Reagents (ACR). Tris buffer (pH 8.0), NaCl (5M), MgCl₂ (1M) and nuclease free water were purchased from Ambion. 10X PCR Buffer II was purchased from Applied Biosystems. Nitrocellulose membrane was purchased from Bio-Rad, polyethersulfone membrane and polycarbonate membrane were purchased from Sterlitech and double sided sticky tape was purchased from Sellotape.

Human genomic DNA was purchased from Promega (193 ng/µL). All synthetic oligonucleotides were purchased from Integrated DNA Technologies (IDT) and prepared as 20 µM stock solutions using nuclease free water. *Chlamydia trachomatis* (CT) (serovar D) TNA samples were obtained in vitro, using standard tissue culture techniques as described below.

The human epithelial cell line (HEp-2) (ATCC^{®} CCL-23^{™}) were grown in Dulbecco's Modified Eagle medium (DMEM) (Sigma Aldrich) supplemented with 10% heat-inactivated Fetal Bovine Serum (FBS) (Sigma Aldrich), 100 mg/mL Streptomycin (Gibco^{®}, Invitrogen Corporation), 50 mg/mL Gentamicin (Gibco^{®} by Life Technologies) and 20mM Glutamine (Sigma Aldrich), incubated at 37°C with 5% CO₂.

*Chlamydia trachomatis* was inoculated on a HEp-2 monolayer, present on a T75 flask (Nunc^{™}, Thermo Fisher) with a Multiplicity of Infection (MOI) of 1. The infection was completed by centrifugation-assisted inoculation at 500 g for 30 minutes at a temperature of 28°C and subsequently incubated. At 4 hours post-infection (PI), DMEM was replaced with addition of 1mg/mL of Cychlohexamide (Sigma Aldrich) and again incubated at 37°C with 5% CO₂ until harvested. At an exponential growth phase, 24 hours PI, cells were manually harvested using a cell scraper and sucrose-phosphate-glutamate (SPG) buffer (250mM sucrose, 10nM sodium phosphate and 5mM L-glutamate). Harvested material was stored for further processing at -80°C. Total nucleic acid samples (TNA) were extracted using the QIAamp MinElute Virus Spin Kit (QIAgen) following the standard protocol.

### - Preparation of SubZymeBead Complexes (SubZyme_Bead)

Assays described in the Examples below employed SubZymes A to K **(SEQ ID No: 1 - 12**) which were attached to Beads using one of the following two protocols. Examples 2 - 7 used Attachment Method One (3A), and Examples 10 - 11 and 13 - 20 used Attachment Method Two (3B). When a SubZyme sequence is attached to a Bead its name includes '-Bead'. When a SubZyme sequence is attached to a magnetic bead its name includes '-MB' (e.g. SubZyme A attached to a magnetic bead is named "SubZyme A-MB").

Attachment Method 1: The M270 Dynabead storage vial was vortexed and thoroughly mixed to ensure that the magnetic beads (MBs) were homogenously dispersed. Dynabeads were washed three times to remove the preservative and to denature any ribonucleases by washing two times with 100 µL of 'Wash Buffer A' (100 mM NaOH, 50 mM NaCl) for three mins each and one time with 100 µL of 'Wash Buffer B' (100 mM NaOH) for three mins. SubZyme A and SubZyme B were immobilised onto the MBs using the highly specific biotin-streptavidin interaction. The washed MBs were incubated in a 50 µL final volume containing 'Incubation Buffer' (2 M NaCl, 10 mM Tris-HCl pH 7.5) and 5 µM of either SubZyme A or SubZyme B for one hour at room temperature. The incubation mixtures were measured using a spectrophotometer (Trinean Xpose) at A₂₆₀ before and after incubation to determine the immobilisation efficiencies. The incubation solutions were removed and discarded and the SubZyme-MB complexes were thoroughly washed to remove excess SubZyme molecules. A series of three 5 minute washes were performed in 200 µL of 'Incubation Buffer' at 55°C, followed by two 5 minute washes in 200 µL of 'Reaction Buffer' (15 mM MgCl₂, 1 X PCR Buffer II) at 55°C.

The final wash solutions were collected and incubated with 200 nM of the complementary substrate to ensure that the unbound SubZyme strands were thoroughly removed. Quality control tests were performed using the functionalised beads to estimate the concentration of attached SubZyme-MB complexes and to ensure that the DNAzyme component remained catalytically active. Finally, the SubZyme-MBs were stored dry at 5°C in the fridge.

Attachment Method 2: The Bead storage vials were vortexed and thoroughly mixed to ensure that the Beads were homogenously dispersed. Beads were washed three times to remove the preservative using 150 µL of Buffer C (1.5 M NaCl, 10 mM Tris-HClpH 7.5). SubZymes were immobilised onto the Beads using the highly specific biotin-streptavidin interaction. The washed Beads were incubated in a 50 µL final volume containing 'Buffer C' (1.5 M NaCl, 10 mM Tris-HCl pH 7.5) and 150 nM of SubZyme for five minutes at room temperature. The incubation solutions were removed and discarded and the SubZyme-Bead complexes were thoroughly washed to remove excess SubZyme molecules. SubZyme-Beads were washed once with 400 µL of Buffer C (1.5 M NaCl, 10 mM Tris-HCl pH 7.5) followed by five washes with 400 µL of nuclease free water and two washes performed in 150 µL of 'Reaction Buffer' (45 mM MgCl₂, 1 X PCR Buffer II) at 55°C. Finally, the SubZyme-Beads were re-suspended in 20 µL of nuclease free water and stored at 5°C in the fridge. Magnetic beads were washed using a magnetic rack to retain magnetic beads and discarding the supernatant. Non-magnetic beads were washed by centrifuging the silica bead solution for 3 minutes at 10,000 rpm to pellet the beads followed by removing and discarding the supernatant.

### - Preparation of Permeable barriers ("T-Bags")

Some assays described in the Examples below employed permeable barriers (T bags,) that were prepared using one of the following two protocols. Examples 2 - 7 used T bag Method One (3A) and Examples 10 - 20 used T bag Method Two (3B).

T bag Method 1: Some of the permeable barriers (T bags) were manually prepared according to the diagram depicted in **Figure 3A** using the following materials: nitrocellulose membrane (0.45 µm), double sided sticky tape and SubZyme -MB. Four strips of sticky tape were arranged on a sheet of nitrocellulose as outlined in **Figure 3A** to create a small well. The SubZyme-MB complexes were reconstituted in 10 µL of 'Immobilisation Buffer' and gently flicked to redisperse. A 1 µL aliquot of the SubZyme-MB complex solution was added inside the well and left to dry at room temperature for 2 mins. The well was covered with a small circular nitrocellulose disc (~7 mm diameter) and the T bag was trimmed with scissors. Finally, the disc-shaped T bag was placed inside a 2 mL Eppendorf tube for storage.

T bag Method 2: Some of the permeable barriers (T bags) were manually prepared according to the diagram depicted in **Figure 3B** using the following materials: membrane made of polycarbonate or polyethersulfone material (PES), double sided sticky tape and SubZyme-Beads. Double sided sticky tape was first folded in half so that the sticky sides are folded back on itself and the non-adhesive liner is exposed on the outer surfaces. Using a hole-punch, small wells of 6 mm diameter were punched into the folded sticky tape. After removing the liner from one side, the sticky tape wells were placed onto strips of membrane and pressure was applied before removing the the protective liner from the other side of the sticky tape. An aliquot (0.5 µL) of SubZyme-Bead complex was added inside the well and left to dry at room temperature for approximately 30 seconds. The well was covered with another strip of membrane and the T bag was trimmed into a circular disc with scissors. Finally, the disc-shaped T bag was placed inside a 2 mL Eppendorf tube for storage.

### Example Two: Detection of Synthetic Human TFRC Gene Homologues

In this experiment, the strategy outlined in **Figure 4** was used to detect target and amplify signal in the presence of target oligonucleotides homologues to the human TFRC gene. SubZyme-MBs were prepared using Attachment Method One (Example One) and T bags were prepared using T bag Method One (Example One; **Figure 3A**). The results obtained are shown in Figure **5A and 5B****.**

The oligonucleotides specific to this experiment include; PA (SubZyme A) (**SEQ ID NO: 1**), PB (SubZyme B) (**SEQ ID NO: 2**), Substrate A (**SEQ ID NO: 13**), Substrate B (**SEQ ID NO: 14**), TFRC Partzyme A1 (**SEQ ID NO: 15**), TFRC Partzyme B1 (**SEQ ID NO: 16**) and Target TFRC Oligo (**SEQ ID NO: 17**). The sequences are listed in the Sequence Listing. With reference to **Figure 4****,** SubZyme A is PA, SubZyme B is PB and Partzymes A1 and B1 form the initiating MNAzyme (Cat C) when assembled on TFRC target.

Each reaction contains 0.2 µM TFRC Partzyme A1, 0.2 µM TFRC Partzyme B1, 1X PCR Buffer II, 5.6 µL of SubZyme A-MB, 1 T-Bag containing SubZyme B-MB and 15 mM MgCl₂ in a 125 µL final volume. Reactions either contained target TFRC oligo at final concentrations of 1 pM, 100 fM, 1 fM and 500 aM; or lacked target (no DNA control). Reactions were incubated at 50°C on a heat block in 2 mL reaction tubes (Eppendorf) for either 15 minutes (**Figure 5A**) or 20 minutes (**Figure 5B**) with intermittent mixing via manual inversion.

After the initial incubation, samples were transferred from the heat-block and the T bags were removed and discarded. Samples were briefly centrifuged and the tubes were placed on a magnetic rack to separate un-cleaved SubZyme-MBs and cleaved partial substrate-MB fragments from cleaved "free" DNAzymes 1 and 2 present in the supernatant. Two aliquots of 48 µL of the supernatant were transferred to separate wells in a 96 well plate (Bio-Rad). To each of these wells, 2 µL of substrate mix containing equimolar concentrations of dual labelled Substrate A and Substrate B were added (0.15 µM and 0.2 µM final concentrations for **Figures 5A** and **5B** respectively). The plate was sealed using strip caps (Bio-Rad) and briefly centrifuged prior to monitoring change in florescence in real time for 45 mins.

The results shown in **Figure 5A** illustrates the signal obtained from 1 pM of Oligo AF-TFRC (double thin line), 100 fM of AF-TFRC (broken line) and no target (thick black line). The vertical line at 10 minutes of real time monitoring is equivalent to 25 minutes total reaction time (i.e. 15 mins T-Bag incubation plus ten minutes real time monitoring) and is indicative of a time required to detect 1 pM & 100 fM of target. The reactions shown in **Figure 5B** were incubated for 20 minutes and contain either no target, (thick black line), 100 fM of AF-TFRC (thin double line), 1 fM of AF-TFRC (dotted line) or 0.5 fM of AF-TFRC (dashed line). In this experiment the limit of detection achieved is 100fM and lower target concentrations (1fM and 500 aM) did not produce a signal over background (no-target reaction).

These results are consistent with the following scenario. In the presence of target oligonucleotide the partzymes align and form a MNAzyme. The MNAzyme cleaves SubZyme A-MB in solution separating the 8-17 DNAzyme from the surface of the MB. The "free" 8-17 DNAzyme can subsequently migrate through the T bag selectively permeable membrane where it can cleave SubZyme B-MB causing separation of the 10-23 DNAzyme from the surface of the MB. The 10-23 DNAzyme is then free to migrate out of the T bag permeable membrane into solution where it can cleave SubZyme A-MB and continue the cascade. After a period of incubation a magnet is used to separate the un-cleaved SubZyme-MBs and cleaved partial substrate-MB fragments from the cleaved 'free' DNAzymes.

Fluorescence from the Texas Red channel was measured using a CFX96 real time PCR detection system (Bio-Rad) with acquisition taking place every 5 seconds for a total of 201 or 150 cycles (**Figures 5A** and **5B** respectively) at a constant temperature of 50°C. The results in **Figures 5A** and **5B** are the averages from duplicates that were plotted using Microsoft Excel (Version 14). **Figure 5** depicts an exemplary titration of oligonucleotide target. In this experiment concentrations of 1pM and 100 fM were easily detected, however, concentrations of 1 fM & 500 aM did not give a signal above background (no target).

### Example Three: Detection of Synthetic Human TFRC Gene Homologues and Off-Target Sequence Analysis

In this experiment the strategy outlined in **Figure 4** was used to firstly detect target and amplify signal in the presence of target oligonucleotides homologous to the human TFRC gene, and secondly, to analyse off-target sequences (Bla_KPC and CTcds2_2 genes that are homologous to carbapenamse resistant bacteria and *Chlamydia trachomatis* respectively) to examine the specificity of the reaction. SubZyme-MBs were prepared using Attachment Method One (Example One) and T bags were prepared using T bag Method One (Example One; Figure 3A). The results of this experiment are shown in **Figure 6****.**

The oligonucleotides specific to this experiment include; PA (SubZyme A) (**SEQ ID NO: 1**), PB (SubZyme B) (**SEQ ID NO: 2**), Substrate A (**SEQ ID NO: 13**), Substrate B (**SEQ ID NO: 14**), TFRC Partzyme A1 (**SEQ ID NO: 15**), TFRC Partzyme B1 (**SEQ ID NO: 16**), Target TFRC Oligo (**SEQ ID NO: 17**), Off-target Oligo Bla_KPC (**SEQ ID NO: 18**) and Off-target Oligo AF-CTcds2_2 (**SEQ ID NO: 19**). The sequences are listed in the Sequence Listing. With reference to **Figure 4****,** SubZyme A is PA, SubZyme B is PB and partzymes A1 and B1 form the initiating MNAzyme (Cat C) when assembled on TFRC target.

Each reaction contained 0.2 µM TFRC Partzyme A1, 0.2 µM TFRC Partzyme B1, 1X PCR Buffer II, 2.5 µL of SubZyme A-MB, 1 x T-Bag containing SubZyme B-MB and 15 mM MgCl₂ in 125 µL final volume. Reactions further contained either 1pM of Target TFRC Oligo or 1nM off target Oligo Bla_KPC or 1 nM of off target Oligo CTcds2_2 or no DNA target. The reactions were incubated at 50°C for 20 minutes in 2 mL reaction tubes on a heat block with intermittent mixing via manual inversion. After the initial incubation, the samples were transferred from the heat-block and the T bags were removed and discarded. Samples were briefly centrifuged and the tubes were placed on a magnetic rack to separate un-cleaved SubZyme-MBs and cleaved partial substrate-MB fragments from cleaved "free" DNAzymes. Two aliquots of 48 µL of the supernatant were transferred to a 96 well plate (Bio-Rad) and 2 µL of substrate mix containing equimolar concentrations of Substrate A and Substrate B (0.15 µM final concentration) were added to each of the wells. The plate was sealed and briefly centrifuged. The plate was placed on a CFX96 real time PCR detection system (Bio-Rad) with acquisition every 5 seconds for 150 cycles at a constant temperature of 50'C. Fluorescence from the Texas Red channel was monitored in real time. The results in **Figure 6** are the averages from duplicates that were plotted using Microsoft Excel (Version 14).

The results in **Figure 6** show four reactions including a no target control (solid black line), a reaction containing 1 pM of the matched TFRC target (thin double line), and two reactions containing 1 nM of off-target Oligo Bla-KPC oligo (dotted line) or 1 nM of off target Oligo CT_CDS (dashed line).

The results presented in **Figure 6** demonstrate that the T bag signal amplification reaction is highly specific and is only triggered in the presence of MNAzymes formed with matching nucleic acid sequence (target assembly facilitator). In the current experiment, high concentrations of synthetic oligonucleotides that are homologous to bacterial genes (Bla-KPC and Ct-Cds2) were added to the T bag assay designed to detect human genomic DNA (TFRC gene). The results demonstrate that the addition of bacterial oligonucleotides did not trigger the cascade and resulted in no signal above background signal (no target control).

### Example Four: Adaptation of Assay to Detect a Different Target Sequence

In this experiment it was demonstrated that the strategy outlined in **Figure 4** can be easily adapted to detect any nucleic acid target. SubZyme-MBs were prepared using Attachment Method One and T vags were prepared using T bag Method One, as described in Example One (3A). With reference to **Figure 4****,** SubZyme A is PA, SubZyme B is PB and partzymes A1 and B1 form the initiating MNAzyme (Cat C) when assembled on TFRC target.

The oligonucleotides specific to this experiment include; PA (SubZyme A) (**SEQ ID NO: 1**), PB (SubZyme B) (**SEQ ID NO: 2**), Substrate A (**SEQ ID NO: 13**), Substrate B (**SEQ ID NO: 14**), OXA Partzyme A (**SEQ ID NO: 20**), OXA Partzyme B (**SEQ ID NO: 21**) and Target OXA Oligo (**SEQ ID NO: 22**). The sequences are listed in the Sequence Listing.

Each reaction contained 0.2 µM OXA Partzyme A, 0.2 µM OXA Partzyme B, 1X PCR Buffer II, 2.45 µL of SubZyme A-MB, 1 x T-Bag containing SubZyme-MB and 15 mM MgCl₂ in 125 µL final volume. The reactions were incubated at 50°C in 2 mL reaction tubes for 20 minutes on a heat block with intermittent mixing via manual inversion. Reactions either contained 10 pM of the Target OXA Oligo or no DNA.

After the initial incubation, the samples were transferred from the heat-block and the T bags were removed from the tubes and discarded. Samples were briefly centrifuged and the tubes were placed on a magnetic rack to separate un-cleaved SubZyme A-MBs and cleaved partial substrate-MB fragments from cleaved DNAzymes freed into solution. Two aliquots of 48 µL of the supernatant were transferred to separate wells in a 96 well plate and 2 µL of substrate mix containing equimolar concentrations of Substrate A and Substrate B (0.2 µM final concentration) were added to each of the wells. Fluorescence from the Texas Red channel was measured in a CFX96 real time PCR detection system (Bio-Rad) with acquisition taking place every 5 seconds for 150 cycles at a constant temperature of 50°C. The results shown in **Figure 7** are the averages from duplicates that were plotted using Microsoft Excel (Version 14) and show a strong signal in the presence of OXA target. Since the only alteration in this experiment to the protocol which detected TFRC in example 3 is the use of MNAzyme with target binding arms specific to OXA, as opposed to TFRC, this demonstrates that the amplification cascade using the SubZymes can be universal for the detection of any target nucleic acid.

### Example Five: Detection of Human TFRC Gene in Human Genomic DNA in the Presence of Human Serum

In this experiment the strategy outlined in **Figure 4** was used to detect target and amplify signal in the presence the human TFRC gene in human genomic DNA. Further, it aimed to determine whether or not the presence of 10 % human serum would affect the reaction. SubZyme-MBs were prepared using Attachment Method One (Example One) and T Bags were prepared using T Bag Method One (Example One). With reference to **Figure 4****,** SubZyme A is PA, SubZyme B is PB and Partzymes A1 and B1 form the initiating MNAzyme (Cat C) when assembled on TFRC target.

The oligonucleotides specific to this experiment include; PA (SubZyme A) (**SEQ ID NOo: 1**), PB (SubZyme B) (**SEQ ID NO: 2**), Substrate A (**SEQ ID NO: 13**), Substrate B (**SEQ ID NO: 14**), TFRC Partzyme A1 (**SEQ ID NO: 15**) and TFRC Partzyme B1 (**SEQ ID NO: 16**). The sequences are listed in the Sequence Listing.

Each of the reactions contained 0.2 µM TFRC Partzyme A1, 0.2 µM TFRC Partzyme B1, 1X PCR Buffer II, 1 µL of SubZyme A-MB, 1 x T-Bag containing SubZyme B-MB, and 15 mM MgCl₂ in 70 µL final volume. Reactions also contained 772 ng of genomic DNA which had been heat denatured at 95°C for 2 minutes. The reactions were performed in the absence (Reaction in **Figure 8A**) or presence (Reaction in **Figure 8B**) of 7 µL of human serum that had been pre-heated at 70°C for 5 min to denature any ribonucleases.

After the initial incubation, the samples were transferred from the heat-block and the T bags were removed from the tubes and discarded. Samples were briefly centrifuged and the tubes were placed on a magnetic rack to separate the un-cleaved SubZyme A-MBs and partial cleaved substrate A-MB fragments from cleaved DNAzymes freed into solution. One 48 µL aliquot of supernatant from each reaction was transferred to a 96 well plate (Bio-Rad) and then mixed with 2 µL of substrate mix containing equimolar concentrations of Substrate A and Substrate B (0.2 µM final concentration) and briefly centrifuged. Fluorescence from the Texas Red channel was measured in a CFX96 real time PCR detection system (Bio-Rad) with acquisition taking place every 10 seconds for 111 cycles at a constant temperature of 50°C.

The results presented in **Figure 8A** demonstrate that the method is capable of detecting the human TFRC gene (solid line) in 772 ng of human genomic DNA which corresponds to around 223,500 copies of the gene (2 x 10⁵ gene copies). The signal is well differentiated from the no gDNA control (dashed line). The reactions which contained 10% serum (**Figure 8B**) were similar to that performed in the absence of serum suggesting the method is tolerant to contaminating serum.

### Example Six: Detection of Human TFRC Gene in Human Genomic DNA with Three Partzymes

In this experiment the strategy outlined in **Figure 4** was used to detect target and amplify signal from the human TFRC gene present in human genomic DNA. In this example the reaction was triggered using three MNAzymes (six partzymes) which assemble on different regions of the TFRC gene. SubZyme-MBs were prepared using Attachment Method One and T Bags were prepared using T Bag Method One, as described in Example One. With reference to **Figure 4****,** SubZyme A is PA, SubZyme B is PB and partzymes A1 and B1 form the initiating MNAzyme (Cat C) when assembled on TFRC target. Partzymes A2 and B2 form a second initiating MNAzyme (Cat E) and Partzymes A3 and B3 form a third initiating MNAzyme (Cat F) when assembled on TFRC target.

The oligonucleotides specific to this experiment include; PA (SubZyme A) (**SEQ ID NO: 1**), PB (SubZyme B) (**SEQ ID NO: 2**), Substrate A (**SEQ ID NO: 13**), Substrate B (**SEQ ID NO: 14**), TFRC Partzyme A1 (**SEQ ID NO: 15**), TFRC Partzyme B1 (**SEQ ID NO: 16**), TFRC Partzyme A2 (**SEQ ID NO: 23**), TFRC Partzyme B2 (**SEQ ID NO: 24**), TFRC Partzyme A3 (**SEQ ID NO: 25**) and TFRC Partzyme B3 (**SEQ ID NO: 26**). The sequences are listed in the Sequence Listing.

The reactions contained 0.2 µM TFRC Partzyme A1, 0.2 µM TFRC Partzyme B1, 0.2 µM TFRC Partzyme A2, 0.2 µM TFRC Partzyme B2, 0.2 µM TFRC Partzyme A3, 0.2 µM TFRC Partzyme B3, 1X PCR Buffer II, 1 µL of SubZyme A-MB, 1 x T bag containing SubZyme B-MB, and 15 mM MgCl₂ in 70 µL final volume. Reactions also contained, or lacked, human genomic DNA which had been subjected to heat denaturation at 95°C for 2 minutes before its addition to the reaction. Reactions were incubated at 50°C for 20 minutes in 2 mL tubes on a heat block with intermittent mixing via manual inversion.

After the initial incubation, the samples were transferred from the heat-block and the T bags were removed from the tubes and discarded. Samples were briefly centrifuged and the tubes were placed on a magnetic rack to separate un-cleaved SubZyme A-MBs and cleaved partial substrate A-MB fragments from DNAzymes freed into solution. A 48 µL aliquot of the supernatant was transferred to a 96 well plate (Bio-Rad) and mixed with 2 µL of substrate mix containing equimolar concentrations of Substrate A and Substrate B (0.2 µM final concentration). The plate was sealed and briefly centrifuged. Fluorescence from the Texas Red channel was measured in a CFX96 real time PCR detection system (Bio-Rad) with acquisition taking place every 10 seconds for 120 cycles at a constant temperature of 50°C.

The results presented in **Figure 9** depict detection of the human TFRC gene in human genomic DNA using signal amplification in a T bag format. The experiment was performed in the presence (solid line) or absence (dotted line) of human genomic DNA (280,000 or 2.8 x 10⁵ gene copies).

### Example Seven: Detection of Human TFRC Gene in Human Genomic DNA

In this experiment the strategy outlined in **Figure 4** was used to firstly detect target and amplify signal in the presence of the human TFRC gene. SubZyme-MBs were prepared using Attachment Method One and T Bags were prepared using T Bag Method One, as described in Example One. With reference to **Figure 4****,** SubZyme A is PA, SubZyme B is PB and Partzymes A1 and B1 form the initiating MNAzyme (Cat C) when assembled on TFRC target.

The oligonucleotides specific to this experiment include; PA (SubZyme A) (**SEQ ID NO: 1**), PB (SubZyme B) (**SEQ ID NO: 2**), Substrate A (**SEQ ID NO: 13**), Substrate B (**SEQ ID NO: 14**), TFRC Partzyme A1 (**SEQ ID NO: 15**) and TFRC Partzyme B1 (**SEQ ID NO: 16**). The sequences are listed in the Sequence Listing.

Each of the reactions contain 0.2 µM TFRC Partzyme A1, 0.2 µM TFRC Partzyme B1, 1X PCR Buffer II, 2.5 µL of SubZyme A-MB, 1 x T-Bag comprising SubZyme B-MB, and 15 mM MgCl₂ in 125 µL final volume. Reactions ether lacked genomic DNA or contained 965 ng or 579 ng of human genomic DNA which had been heat denatured at 95°C for 2 minutes. The reactions were incubated at 50°C for 20 minutes in 2 mL reaction tubes on a heat block with intermittent mixing via manual inversion. The experiment was performed in the presence of 965 ng (solid line), 579 ng (dashed line) or absence (dotted line) of human genomic DNA.

After the initial incubation, the samples were transferred from the heat-block and the T bag was removed from the tube and discarded. Samples were briefly centrifuged and the tubes were placed on a magnetic rack to separate the un-cleaved SubZyme A-MBs and cleaved partial substrate A-MB fragments from DNAzymes freed into solution. Two 48 µL aliquots of the supernatant were transferred to separate wells in a 96 well plate (Bio-Rad) and mixed with 2 µL of substrate containing equimolar concentrations of Substrate A and Substrate B (0.2 µM final concentration). The plate was sealed and briefly centrifuged. Fluorescence from the Texas Red channel was measured in a CFX96 real time PCR detection system (Bio-Rad) with acquisition taking place every 5 seconds for 150 cycles at a constant temperature of 50°C. The results are the averages from duplicates that were plotted using Microsoft Excel (Version 14). **Figure 10** depicts this exemplary titration of human genomic DNA using signal amplification in a T-Bag format. In this experiment concentrations of 270,000 and 168,000 copies of TFRC gene were detected above background signal (no target).

### Example Eight: Improved stability by Combining 8-17 and 10-23 Catalytic Nucleic Acids

In this experiment, the long-term stability of two exemplary SubZymes were compared. SubZyme A is comprised of an 8-17 catalytic nucleic acid component and a 10-23 catalytic nucleic acid substrate component. SubZyme C is comprised of a 10-23 catalytic nucleic acid component and a 10-23 catalytic nucleic acid substrate component. The results obtained are shown in **Figure 11****.**

The oligonucleotides specific to this experiment include; PA (SubZyme A) (**SEQ ID NOo: 1**), PA (SubZyme C) (**SEQ ID NO: 3**), TFRC Partzyme A1 (**SEQ ID NO: 15**), TFRC Partzyme B1 (**SEQ ID NO: 16**), Target TFRC Oligo (**SEQ ID NO: 17**), Target TFRC Oligo 4 (**SEQ ID NO: 27**) and DNAzyme A (**SEQ ID NO: 28**). The sequences are listed in the Sequence Listing.

SubZyme C is comprised of a 10-23 catalytic nucleic acid component and a 10-23 catalytic nucleic acid substrate component, the substrate component contains an internal fluorescein and two internal ZEN Quencher moieties. Reactions A, B, C and D contain 0.2 µM TFRC Partzyme A1, 0.2 µM TFRC Partzyme B1, 0.2 µM SubZyme C, 1X PCR Buffer II, and 25 mM MgCl₂ in a 20 µL final reaction volume. All reactions were performed in duplicate at 52°C in a Bio-Rad^{®} CFX96 thermocycler and fluorescence was measured in FAM channel with acquisition taking place every 5 seconds (scan mode: SYBR/FAM Only) for a total of 201 cycles for reactions A and B and 200 cycles for reactions C and D. Reactions A and C do not contain Target TFRC Oligo. Reaction B contains 1581 pg (5 nM) of Target TFRC Oligo 4 and Reaction D contains 512 pg (2 nM) of Target TFRC Oligo.

SubZyme A is comprised of an 8-17 catalytic nucleic acid component and a 10-23 catalytic nucleic acid substrate component, the substrate component contains an internal fluorescein and two internal ZEN Quencher moieties. Reactions E, F, G and H contain 0.2 µM SubZyme A, 1X PCR Buffer II, and 25 mM MgCl₂ in a 20 µL final reaction volume. All reactions were performed in duplicate at 50°C in a Bio-Rad^{®} CFX96 thermocycler and fluorescence signal was measured in FAM channel with acquisition taking place every 5 seconds (scan mode: All channels) for a total of 201 cycles. Reactions E and G do not contain any initiating DNAzyme and Reactions F and H each contain 503 pg (2.5 nM) of initiating DNAzyme.

The results from reactions A and B are shown in **Figure 11A** and illustrate the target dependent cleavage of the substrate component of SubZyme C by a 10-23 MNAzyme with incubation performed immediately after SubZyme C oligonucleotide had been reconstituted. The cleavage event in Reaction B results in the physical separation of the internal fluorophore and quencher and shows an increase in fluorescence over time. There was no cleavage event in Reaction A which results in minimal increase in fluorscence over time. The results shown in **Figure 11B** illustrate the target dependent cleavage of the substrate component of SubZyme C after it had been stored at -20°C for 43 days. Reactions C and D show an increase in FAM signal over time indicative of SubZyme C self cleavage and/or degradation during storage. The results shown in **Figure 11** are the averages from duplicates that were plotted using Microsoft Excel (Version 14).

The results from reactions E and F are shown in **Figure 11C** and illustrate cleavage of the substrate component of SubZyme A by a 10-23 DNAzyme with incubation performed immediately after SubZyme A oligonucleotide had been reconstituted. The cleavage event in Reaction F results in the physical separation of the internal fluorophore and quencher and shows an increase in FAM signal over time. There was no cleavage event in Reaction E and the results show a minimal increase in FAM signal over time. The results shown in **Figure 11D** illustrate cleavage of the substrate component of SubZyme A by a DNAzyme after SubZyme A had been stored at -20°C for greater than 5 months. Reactions G and H show comparable signal to reactions E and F respectively. This demonstrates that SubZyme A had not degraded or self-cleaved during storage. The improved stability is attributed to the design containing 8-17 and 10-23 catalytic nucleic acid components. The results shown in **Figure 11** are the averages from duplicates that were plotted using Microsoft Excel (Version 14).

### Example Nine: SubZyme Immobilization Onto Planar Microarray Slides

The following example highlights some of the issues encountered when SubZymes are tethered onto planar (immobile) surfaces but were not evident when SubZymes were tethered onto mobile three-dimensional surfaces including, but not limited to, magnetic beads. In this experiment, SubZyme activity is monitored after immobilization onto glass slides. The activity of the immobilized SubZyme (Reaction B) is compared with an untethered (free) DNAzyme (Reaction A) and the results obtained are shown in **Figure 12****.**

The oligonucleotides specific to this experiment include; SubZyme D (**SEQ ID NO: 4**), Substrate C (**SEQ ID NO: 29**) and DNAzyme B (**SEQ ID NO: 30**). The sequences are listed in the Sequence Listing.

The control reaction (Reaction A) was performed using a blank aldehyde slide that had been washed according to the washing procedure described in Stralis-Pavese et al (2011), Nat Protoc. 2011;6(5):609-24. The test reaction (Reaction B) was performed on a VSS-25 Vantage Silyated Aldehyde slide that had been functionalised with SubZyme D, also using methods described in Stralis-Pavese et al (2011) Nature Protocols, 6(5), 609-624. Each reaction contains 0.4 µM Substrate C, 0.02% Tween-20, 25 mM MgCl₂ and 1X PCR Buffer II in a 25 µL final volume. Reaction A contained 50 nM of DNAzyme D which is homologous to the catalytic nucleic acid component of SubZyme D. All reactions occurred within Hybriwell adhesive chambers (Grace Bio-Labs, 611204) placed on top of the slide. The slides were incubated on top of a heat-block set at 54°C. Fluorescence was measured before and after 30 minutes incubation using a Leica fluorescent microscope with the following settings; GFP2 emission, PMT Gain set at 3.5 and an exposure time of 59.7 sec. The images were processed using Image J analysis software where the 8 bit green channels were isolated and the RGB values were measured. The images are shown in **Figure 12A** and **Figure 12B****.** The relative RGB values are plotted in **Figure 12C** where the 'before incubation' RGB values are subtracted from the 'after incubation' RGB values.

The results shown in **Figure 12A** illustrate that minimal fluorescence was observed before the control DNAzyme and substrate were incubated at 54°C and that a strong fluorescence was generated after 30 minutes incubation. The results shown in **Figure 12B** illustrate that there was no notable increase in fluorescence observed for the slide containing immobilised SubZyme D after 30 minutes incubation. Immobilised SubZyme may be unable to hydrolyse the substrate for several reasons including that not enough SubZyme was tethered to the planar surface and/or that the SubZyme is no longer catalytically active after immobilisation onto planar, glass surfaces.

### Example Ten: Demonstration of a First Step of Initiation Using a Nicking Endonuclease

In this experiment the strategy outlined in **Figure 13B** was used to detect target and amplify signal in the presence of a synthetic oligonucleotide target that is homologous to the *Chlamydia trachomatis* ompA gene. In this example the reaction was triggered using the nicking endonuclease Nt. BstNBI as an alternative biochemical trigger to MNAzymes. SubZymes were designed to include a target specific sequence that is complementary to the target of interest and also contains a nicking site for the nicking endonuclease.

The oligonucleotides specific to this experiment include; PA (SubZyme E) (**SEQ ID NO: 5**), Substrate D (**SEQ ID NO: 31**) and Target ompA Oligo 1 (**SEQ ID NO: 32**). The sequences are listed in the Sequence List. SubZyme E is attached to magnetic beads (SubZyme E-MB) as described in Example One - Attachment Method 2.

All reactions contained 1X NEB Buffer 3.1, 5 mM MgCl₂ and 0.5 µL of SubZyme E-MBs in 55 µL final volume. Reactions A contained 4 units Nt.BstNBI and 2 nM Target ompA oligo 1. Reactions B contained 2 nM Target ompA oligo 1 and lacked Nt. BstNBI. Reactions C contained 4 units of Nt. BstNBI and lacked Target ompA oligo 1. Reactions were incubated at 55°C for 20 minutes in 2 mL tubes on a heat block. After the initial incubation, the samples were transferred from the heat-block and the samples were briefly centrifuged. A 48 µL aliquot of the reaction solution was transferred to a 96 well plate (Bio-Rad) and mixed with 2 µL of substrate mix containing 0.2 µM (final concentration) of Substrate D. The plate was sealed and briefly centrifuged. Fluorescence from the FAM channel was measured in a CFX96 real time PCR detection system (Bio-Rad) with acquisition taking place every 10 seconds for 150 cycles at a constant temperature of 50°C.

The results shown in **Figure 14** are the averages from duplicates that were plotted using Microsoft Excel (Version 14). A strong signal in FAM channel was evident in the presence of ompA Target and Nt. BstNBI. No signal is detected in the absence of Nt. BstNBI and/or ompA Target. This indicates that double stranded restriction enzyme recognition sites for Nt. BstNB1 were successfully created upon binding of the target with the SubZyme, allowing Nt. BstNBI to catalyze cleavage of the SubZyme strand thus releasing active 8-17 DNAzymes from the beads. There was no signal generated in the absence of ompA Target or Nt. BstNBI, confirming that both are required for the target induced release of DNAzymes. As the target strand is not cleaved by the nicking endonuclease, it remains available to be recycled and hybridize to other SubZymes, resulting in further DNAzyme release. This may provide a mechanism for enhancing signal generation and increasing the sensitivity of detection of target. The results demonstrate that the amplification cascade involving the release of surface-bound DNAzymes using the Subzymes can be initiated using a range of different biochemical triggers including MNAzymes and restriction enzymes such as nicking endonucleases.

### Example Eleven: Spatial Confinement and Diffusion of DNAzymes - SubZymes, MBs and Polycarbonate Membrane

In this experiment the strategy outlined in **Figure 15** was used to demonstrate the relationship between the capacity for SubZyme-Beads to diffuse through, or be retained by, semi-permeable membranes with varying pore sizes. Secondly, the strategy was used to demonstrate the capacity of free DNAzymes to diffuse through membrane into the T bags and then cleave the substrate regions within SubZymes to release surface-bound DNAzymes. Further, it also aimed to determine whether or not the liberated DNAzymes could diffuse out of the T bag and cleave fluorescent substrate to generate signal. PB (SubZyme F) is attached to magnetic beads (SubZyme F-MB) as described in Example One - Attachment Method 2.The permeable barriers used to construct the T bags were manually prepared using polycarbonate membrane according to the diagram depicted in **Figure 3B****.** Reactions were performed using MBs with various bead diameters (2.8 µm, 6 µm and 8 µm) and two different membrane pore sizes (0.8 µm and 2.0 µm).

The oligonucleotides specific to this experiment include; SubZyme F (**SEQ ID NO: 6**), Substrate E (**SEQ ID NO: 33**) and initiating DNAzyme A (**SEQ ID NO: 28**). The sequences are listed in the Sequence List.

The reactions contained 1X PCR Buffer II, 45 mM MgCl₂ in a 60 µL final volume. Reactions contained 1 x T-Bag made of polycarbonate material (either 0.8 µm or 2 µm) containing SubZyme F-MBs. Reactions contained, or lacked, 2 nM of DNAzyme A. Reactions were incubated at 50°C for 20 minutes in 2 mL tubes on a heat block. After the initial incubation, the samples were transferred from the heat-block and the samples were briefly centrifuged. A 48 µL aliquot of the reaction solution was transferred to a 96 well plate (Bio-Rad) and mixed with 2 µL of Substrate E (0.2 µM final concentration). The plate was sealed and briefly centrifuged. Fluorescence from the Texas Red channel was measured in a CFX96 real time PCR detection system (Bio-Rad) with acquisition taking place every 10 seconds for 150 cycles at a constant temperature of 50°C.

The results shown in **Figure 16A-F** are the averages from duplicates that were plotted using Microsoft Excel (Version 14) and show negligible signal in the absence of initiating DNAzyme. This indicates that SubZymes are unable to diffuse through the T-bag membrane whilst tethered to magnetic beads when the diameter of the magnetic beads is larger than the pore size of the membrane. Since a strong signal was observed in the presence of initiating DNAzyme A, this indicates that the initiating DNAzyme can diffuse into the T-bags, cleave the Subzyme-MB, and release surface-bound DNAzymes B. It further indicates that the released DNAzymes B are able to diffuse out of the T-Bag, and when transferred together with supernatant to a new tube, the released DNAzymes B can cleave fluorescent labelled substrate molecules E. Similar results are shown for various magnetic bead sizes (2.8 µm, 6 µm and 8 µm) and various pore sizes (0.8 µm and 2.0 µm).

### Example Twelve: PES Membrane Can Enhance SubZyme and DNAzyme Activity

The following example highlights the importance of empirically testing the use of different membrane materials for the construction of T bags to determine if the material itself can impact upon the catalytic activity of the reaction constituents. Further, this example indicates that the presence of some membrane materials such as polyethersulfone (PES) may enhance the catalytic activity of DNAzymes.

The oligonucleotides specific to this experiment include; PA (SubZyme G) (**SEQ ID NO: 7**), Substrate F (**SEQ ID NO: 34**) and DNAzyme C (**SEQ ID NO: 35**). The sequences are listed in the Sequence Listing. In this example, PA (SubZyme G) is attached to magnetic beads (SubZyme G-MB) as described in Example One - Attachment Method 2.

The reactions contained 1X PCR Buffer II, 1 µL SubZyme G-MBs, 45 mM MgCl₂ in a 60 µL final volume and either lacked or contained 2 nM of DNAzyme C. Reactions either contained or lacked 2x 3 mm discs of PES membrane and were incubated at 50°C for 20 minutes in 2 mL tubes on a heat block. After the initial incubation, the samples were transferred from the heat-block and the samples were briefly centrifuged. Using a magnetic rack to retain the magnetic beads (uncleaved SubZyme G-MBs and MB-partial substrate portion of cleaved SubZyme G-MBs), a 45 µL aliquot of the reaction solution was transferred to a 96 well plate (Bio-Rad) and mixed with 5 µL of Substrate F (0.2 µM final concentration). The PES membrane was discarded. The plate was sealed and briefly centrifuged. Fluorescence from Texas Red channel was measured in a CFX96 real time PCR detection system (Bio-Rad) with acquisition taking place every 10 seconds for 150 cycles at a constant temperature of 50°C. All reactions were performed in duplicate and the results are presented in **Figure 17****.**

In this experiment the addition of PES membrane to reactions of DNAzyme cleavage of SubZyme-MB appears to enhance DNAzyme activity. This was observed as a faster increase in fluorescence over time for reactions containing PES membrane compared with reactions lacking membrane. This indicates faster cleavage of fluorescent substrates by DNAzymes following their release from the surface of magnetic beads.

### Example Thirteen: Demonstration of an Alternative T-Bag Membrane Material

In this experiment the strategy outlined in **Figure 15** was used to determine the generality of the T bag concept using polyethersulfone (PES) as an alternative semi-permeable membrane material for the construction of T bags. Further, the aim was to determine whether or not SubZyme-MB containment, DNAzyme diffusion and the release of surface-bound DNAzymes are possible using PES membrane. PB (SubZyme F) is attached to magnetic beads (SubZyme F-MB) as described in Example One - Attachment Method 2. The T bags were manually prepared using PES membrane according to the diagram depicted in **Figure 3B** and reactions were performed using various membrane pore sizes (0.65 µm, 0.8 µm and 1.2 µm).

The oligonucleotides specific to this experiment include; PB (SubZyme F) (**SEQ ID NO: 6**), Substrate E (**SEQ ID NO: 33**) and DNAzyme A (**SEQ ID NO: 28**). The sequences are listed in the Sequence Listing.

The reactions contained 1X PCR Buffer II, 45 mM MgCl₂ in a 60 µL final volume. Reactions contained 1 x T bag made of PES material (either 0.65 µm, 0.8 µm or 1.2 µm) containing 0.6 µL of SubZyme F-MBs. Reactions contained, or lacked, 2 nM of DNAzyme A. Reactions were incubated at 50°C for 20 minutes in 2 mL tubes on a heat block. After the initial incubation, the samples were transferred from the heat-block and the samples were briefly centrifuged. A 48 µL aliquot of the reaction solution was transferred to a 96 well plate (Bio-Rad) and mixed with 2 µL of Substrate E (0.2 µM final concentration). The plate was sealed and briefly centrifuged. Fluorescence from the Texas Red channel was measured in a CFX96 real time PCR detection system (Bio-Rad) with acquisition taking place every 10 seconds for 150 cycles at a constant temperature of 50°C.

The results shown in **Figure 18A-C** are the averages from duplicates that were plotted using Microsoft Excel (Version 14) and show negligible signal in the absence of initiating DNAzyme A. This indicates that SubZymes are unable to diffuse through the PES membrane whilst tethered to magnetic beads (Dynabeads M270 2.8 µm). There is a strong signal in the presence of initiating DNAzyme A, indicating that the initiating DNAzyme can diffuse into T-bags made of PES membrane and then cleave SubZymes and release surface-bound DNAzymes. It also indicates that the released DNAzymes are able to diffuse out of the PES T bag and cleave fluorescent labelled substrate molecules. Similar results are observed for membranes with different pore sizes (0.65 µm, 0.8 µm and 1.2 µm) indicating that the pore size is not critical as long as it is smaller than the diameter of the SubZyme-MBs. The results shown in **Figure 18** illustrate that alternative membranes, such as PES, can be employed for the encapsulation and physical separation of SubZyme-MBs from other reaction components.

### Example Fourteen: Detection of Chlamydia Trachomatis ompA Gene Homologues and Off-Target Sequence Analysis

In this experiment the strategy outlined in **Figure 4** was used to detect target and amplify signal in the presence of a synthetic oligonucleotide target that is homologous to the *Chlamydia trachomatis* ompA gene, demonstrating that the strategy can be easily adapted to detect any nucleic acid target. A second aim is to analyse off-target sequences (PPIA and p273 genes that are homologous to human genes) to examine the specificity of the reaction. PA (SubZyme H) and and PB (SubZyme I) were attached to magnetic beads (SubZyme H-MB and SubZyme I-MB) as described in Example One - Attachment Method Two. The T bags were manually prepared using polycarbonate membrane (0.8 µm) according to the diagram depicted in **Figure 3B****.** The results obtained are shown in **Figure 19****.** With reference to **Figure 4****,** SubZyme H is PA, SubZyme I is PB and ompA partzymes A1 and B1 form the initiating MNAzyme (Cat C) when assembled on ompA target.

The oligonucleotides specific to this experiment include; PA (SubZyme H) (**SEQ ID NO: 8**), PB (SubZyme I) (**SEQ ID NO: 9**), Substrate G (**SEQ ID NO: 36**), Substrate H (**SEQ ID NO: 37**), ompA Partzyme A1 (**SEQ ID NO: 38**), ompA Partzyme B1 (**SEQ ID NO: 39**), Target ompA Oligo 2 (**SEQ ID NO: 40**), Off-Target Oligo p273 (**SEQ ID NO: 41**) and Off-Target Oligo PPIA (**SEQ ID NO: 42**). The sequences are listed in the Sequence Listing.

The reactions contained 5 nM ompA Partzyme A1, 5 nM ompA Partzyme B1, 1X PCR Buffer II, 0.5 µL of SubZyme H-MB, 1 x T bag containing 0.5 µL of SubZyme I-MB, and 45 mM MgCl₂ in 70 µL final volume. T bags were made of polycarbonate membrane of 0.8 µm pore size. Reactions either contained target ompA oligo 2 at final concentrations of 100 fM, 10 fM and 1 fM, or 1nM off target Oligo p273, or 1 nM of off target Oligo PPIA, or lacked target (no DNA control). Reactions were incubated at 50°C for 20 minutes in 2 mL tubes on a heat block. After the initial incubation, the samples were transferred from the heat-block and the T bags were removed from the tubes and discarded. Samples were briefly centrifuged and the tubes were placed on a magnetic rack to separate un-cleaved SubZyme H-MBs and cleaved partial substrate H-MB fragments from DNAzymes freed into solution. A 48 µL aliquot of the supernatant was transferred to a 96 well plate (Bio-Rad) and mixed with 2 µL of substrate mix containing equimolar concentrations of Substrate G and Substrate H (0.2 µM final concentration). The plate was sealed and briefly centrifuged. Fluorescence from the FAM channel was measured in a CFX96 real time PCR detection system (Bio-Rad) with acquisition taking place every 10 seconds for 150 cycles at a constant temperature of 50°C.

The results shown in **Figure 19** are the averages from duplicates that were plotted using Microsoft Excel (Version 14) and depict an exemplary titration of oligonucleotide target. The results in **Figure 19** illustrates the signal obtained from reactions containing 100 fM of Target ompA Oligo 2 (black line), 10 fM of Target ompA Oligo 2 (broken black line), 1 fM of Target ompA Oligo 2 (small black dashed line), 1 nM PPIA off-Target (grey dashed line), 1 nM p273 off-Target (grey dotted line), ~200,000 copies of human genomic DNA (grey solid line) and no DNA (grey double line). The five minutes of real time monitoring illustrated in **Figure 19** is equivalent to 25 minutes total reaction time (i.e. 20 mins T-Bag incubation plus 5 minutes real time monitoring of fluorescent substrate cleavage) and is indicative of a time required to detect 100 fM, 10 fM and 1 fM of ompA target. In this experiment the protocol was able to detect the lowest concentration tested, namely 1 fM of ompA target. The total concentration of ompA in the sample corresponds to approximately 4,200 copies of the gene (4.2 x 10³ gene copies). Furthermore, the addition of high concentrations of off-target controls (PPIA, p273 and Hu gDNA) did not produce signal above that of the no target controls (No DNA), demonstrating that the T-Bag signal amplification reaction is highly specific and is only triggered in the presence of MNAzymes formed with matching target nucleic acid sequence (ompA Oligo 2 in this example).

These results are consistent with the following scenario. In the presence of target oligonucleotide the Partzymes align and form an active initiating MNAzyme. The MNAzyme cleaves SubZyme H-MB in solution separating the 8-17 DNAzyme from the surface of the MB. The "free" 8-17 DNAzyme can subsequently migrate through the T bag selectively permeable membrane where it can then cleave SubZyme I-MB causing separation of the 10-23 DNAzyme from the surface of the MB. The 10-23 DNAzyme is then free to migrate out of the T bag permeable membrane into solution where it can cleave SubZyme H-MB and continue the cascade. After a period of incubation a magnet can separate the un-cleaved SubZyme-MBs and cleaved partial substrate-MB fragments from the cleaved 'free' DNAzymes. The free DNAzymes (10:23 and 8:17) can be transferred into a second reaction chamber containing fluorescent substrates, within, the free DNAzymes can cleave substrates and generate fluorescence signal.

### Example Fifteen: Detection of Chlamydia Trachomatis ompA Gene in Chlamydia Trachomatis Total Nucleic Acid (TNA) Samples

In this experiment the strategy outlined in **Figure 4** was used to detect target and amplify signal from the *Chlamydia trachomatis* ompA gene present in *Chlamydia* total nucleic acid samples (TNA), obtained using the culture and extraction methods described in Example One. SubZyme H and SubZyme I were attached to magnetic beads (SubZyme H-MB and SubZyme I-MB) as described in Example One - Attachment Method Two. The T bags were manually prepared using polycarbonate membrane (0.8 µm) according to the diagram depicted in **Figure 3B****.** The results obtained are shown in **Figure 20**.With reference to **Figure 4****,** SubZyme H is PA, SubZyme I is PB and ompA partzymes A1 and B1 form the initiating MNAzyme Cat C when assembled on ompA target.

The oligonucleotides specific to this experiment include; PA (SubZyme H) (**SEQ ID NO: 8**), PB (SubZyme I) (**SEQ ID NO: 9**), Substrate G (**SEQ ID NO: 36**), Substrate H (**SEQ ID NO: 37**), ompA Partzyme A1 (**SEQ ID NO: 38**), ompA Partzyme B1 (**SEQ ID NO: 39**), andTarget ompA Oligo 2 (**SEQ ID NO: 40**). The sequences are listed in the Sequence Listing.

The reactions contained 5 nM ompA Partzyme A1, 5 nM ompA Partzyme B1, 1X PCR Buffer II, 0.5 µL of SubZyme H-MB, 1 x T bag containing 0.5 µL of SubZyme I-MB, and 25 mM MgCl₂ in 70 µL final volume. T bags were made of polycarbonate membrane of 0.8 µm pore size. Reactions either lacked DNA (No DNA control), or contained 2 fM of target ompA oligo, or contained *Chlamydia trachomatis* TNA which was the TNA had been subjected to heat denaturation at 95°C for 2 minutes before its addition to the reaction. Reactions were incubated at 50°C for 20 minutes in 2 mL tubes on a heat block.

After the initial incubation, the samples were transferred from the heat-block and the T bags were removed from the tubes and discarded. Samples were briefly centrifuged and the tubes were placed on a magnetic rack to separate un-cleaved SubZyme H-MBs and cleaved partial substrate H-MB fragments from DNAzymes freed into solution. A 48 µL aliquot of the supernatant was transferred to a 96 well plate (Bio-Rad) and mixed with 2 µL of substrate mix containing equimolar concentrations of Substrate G and Substrate H (0.2 µM final concentration). The plate was sealed and briefly centrifuged. Fluorescence from the FAM channel was measured in a CFX96 real time PCR detection system (Bio-Rad) with acquisition taking place every 10 seconds for 150 cycles at a constant temperature of 50°C.

The results presented in **Figure 20** are the averages from duplicates that were plotted using Microsoft Excel (Version 14) and demonstrate that the method is capable of detecting the *Chlamydia trachomatis* ompA gene (solid black line) from *Chlamydia trachomatis* total nucleic acid (TNA) samples. The total concentration of ompA in the sample corresponds to approximately 3,500,000 copies of the gene (3.5 x 10⁶ gene copies). The signal is well differentiated from the no DNA control (grey solid line) and results in a signal which is similar to reactions containing 2 fM of synthetic target ompA oligonucleotide (black broken line).

### Example Sixteen: Rapid Detection of ompA Gene Homologues in 15 Minutes

In this experiment the strategy outlined in **Figure 4** was used to detect target and amplify signal in the presence of a synthetic oligonucleotide target that is homologous to the *Chlamydia trachomatis* ompA gene. Here, samples were incubated with the T bag for only 12 minutes, demonstrating the rapid detection of nucleic acid targets. PA (SubZyme H) and PB (SubZyme I) were attached to magnetic beads (SubZyme H-MB and SubZyme I-MB) as described in Example One - Attachment Method Two. The T bags were manually prepared using polycarbonate membrane (0.8 µm) according to the diagram depicted in **Figure 3B****.** The results obtained are shown in **Figure 21**.With reference to **Figure 4****,** SubZyme H is PA, SubZyme I is PB and ompA partzymes A1 and B1 form the initiating MNAzyme (Cat C) when assembled on ompA target

The oligonucleotides specific to this experiment include; PA (SubZyme H) (**SEQ ID NO: 8**), (PB) SubZyme I (**SEQ ID NO: 9**), Substrate H (**SEQ ID NO: 37**), ompA Partzyme A1 (**SEQ ID NO: 38**), ompA Partzyme B1 (**SEQ ID NO: 39**), andTarget ompA Oligo 2 (**SEQ ID NO: 40**). The sequences are listed in the Sequence Listing.

The reactions contained 5 nM ompA Partzyme A1, 5 nM ompA Partzyme B1, 1X PCR Buffer II, 0.5 µL of SubZyme H-MB, 1 x T bag containing 0.5 µL of SubZyme I-MB, and 25 mM MgCl₂ in 60 µL final volume. T bags were made of polycarbonate membrane of 0.8 µm pore size. Reactions either lacked DNA (No DNA control), or contained 500 pM or 2 pM of target ompA oligo. Reactions were incubated at 50°C for 12 minutes in 2 mL tubes on a heat block.

After the initial incubation, the samples were transferred from the heat-block and the T bags were removed from the tubes and discarded. Samples were briefly centrifuged and the tubes were placed on a magnetic rack to separate un-cleaved SubZyme H-MBs and cleaved partial substrate H-MB fragments from DNAzymes freed into solution. A 46 µL aliquot of the supernatant was transferred to a 96 well plate (Bio-Rad) and mixed with 4 µL of substrate Substrate H (0.2 µM final concentration). The plate was sealed and briefly centrifuged. Fluorescence from the FAM channel was measured in a CFX96 real time PCR detection system (Bio-Rad) with acquisition taking place every 10 seconds for 150 cycles at a constant temperature of 50°C.

The results presented in **Figure 21** are the averages from duplicates that were plotted using Microsoft Excel (Version 14) and demonstrate that the method is capable of detecting 500 pM (grey line) and 1 pM (black line) of synthetic target ompA oligonucleotide in less than 15 minutes total reaction time. The three minutes of real time monitoring illustrated in **Figure 21** is the final stage of a reaction that has 15 minutes total time (i.e. 12 mins T bag incubation plus three minutes real time monitoring) and is indicative of a time required to detect 500 pM and 1 pM of synthetic target ompA oligonucleotide. The signal is well differentiated from the no DNA control (black broken line).

### Example Seventeen: Cross Catalytic Cascade using SubZymes incorporating DNAzymes and PartZymes

In this experiment the strategy outlined in **Figure 22** was used to detect target and amplify signal in the presence of a synthetic oligonucleotide target that is homologous to the *Chlamydia trachomatis* ompA gene. In this experiment SubZyme J (PA) and SubZyme K1 (PB) were attached to magnetic beads (SubZyme J-MB and SubZyme K1-MB) as described in Example One - Attachment Method Two. The T bags were manually prepared using polycarbonate membrane (0.8 µm) according to the diagram depicted in **Figure 3B****.** The initiating MNAzyme C (Cat C) comprised ompA Partzymes A1 and B1 (equivalent to C1 and C2 in **Figure 22**) and target ompA. The results obtained are shown in **Figure 24****.**

The oligonucleotides specific to this experiment include; PA (SubZyme J) (**SEQ ID NO: 10**), PB (SubZyme K1) (**SEQ ID NO: 11**), PC (SubZyme K2) (**SEQ ID NO: 12**), Substrate E (**SEQ ID NO: 33**), ompA Partzyme A1 (**SEQ ID NO: 38**), ompA Partzyme B1 (**SEQ ID NO: 39**), Target ompA Oligo 2 (**SEQ ID NO: 40**) and AF-B3 (Feedback Assembly Facilitator) (**SEQ ID NO: 43**). The sequences are listed in the Sequence Listing.

The reactions contained 50 nM ompA Partzyme A1, 50 nM ompA Partzyme B1, 1X PCR Buffer II, 45 mM MgCl₂, 2 nM SubZyme K2, 2 nM Feedback Assembly Facilitator, 0.6 µL of PA (SubZyme J-MB), 1 x T bag containing 0.6 µL of PB (SubZyme K1-MB) in 60 µL final volume. Reactions either lacked DNA (No DNA control), or contained 2 nM, 200 pM, 50 pM, 10 pM or 1 pM of target ompA oligo. Reactions were incubated at 50°C for 20 minutes in 2 mL tubes on a heat block.

After the initial incubation, the samples were transferred from the heat-block and the T bags were removed from the tubes and discarded. Samples were briefly centrifuged and the tubes were placed on a magnetic rack to separate un-cleaved SubZyme J-MBs, cleaved partial substrate J-MB fragments and un-cleaved SubZyme K1-MBs from DNAzymes and Partzymes freed into solution. A 48 µL aliquot of the supernatant was transferred to a 96 well plate (Bio-Rad) and mixed with 2 µL of substrate Substrate E (0.2 µM final concentration). The plate was sealed and briefly centrifuged. Fluorescence from the Texas red channel was measured in a CFX96 real time PCR detection system (Bio-Rad) with acquisition taking place every 10 seconds for 150 cycles at a constant temperature of 50°C.

The results presented in **Figure 24** are the averages from duplicates that were plotted by subtracting the fluorescence at 0 mins from the fluorescence at 5 mins using Microsoft Excel (Version 14). The results demonstrate that the method is capable of detecting 2 nM, 200 pM, 50 pM, 10 pM, and 1 pM of synthetic target ompA oligonucleotide. The signal is well differentiated from the no DNA control. These results are consistent with the following scenario. In the presence of target oligonucleotide the Partzymes align and form an active initiating MNAzyme. The MNAzyme cleaves SubZyme J-MB in solution separating the 8-17 DNAzyme from the surface of the MB. The "free" 8-17 DNAzyme can subsequently migrate through the T bag selectively permeable membrane where it can cleave Sub B within PB (SubZyme K1-MB) causing separation of Partzyme K1 (Cat B1) from the surface of the MB. The Cat B1 is then free to migrate out of the T bag permeable membrane into solution where it can hybridise with free Partzyme K1 (Cat B2) and Feedback Assembly Facilitator (AF-B3) to form an active Feedback MNAzyme (Cat B) which can cleave Sub A within PA (SubZyme J-MB), thereby continuing a feedback cascade. After a period of incubation a magnet is used to separate the un-cleaved SubZyme-MBs and cleaved partial substrate-MB fragments from the cleaved 'free' DNAzymes and Partzymes. The free DNAzymes (Cat A) and Partzymes (Cat B) are transferred into a second reaction chamber containing fluorescent substrates, wherein, the free DNAzymes can cleave to generate fluorescence signal.

### Example Eighteen: Cross Catalytic Cascade using SubZymes comprised entirely of 8-17 components

This experiment demonstrates that SubZymes composed entirely of 8-17 catalytic nucleic acid components can be used to execute a cross-catalytic signal amplification cascade using SubZymes and selectively permeable barriers. The strategy outlined in **Figure 4** was modified by using an initiating Cat C which was an 8-17 DNAzyme D, and where both PA (SubZyme L) and PB (SubZyme M) comprised 8:17 DNAzymes attached by substrate sequences which were cleavable in a cross catalytic manner.

The oligonucleotides specific to this experiment include; PA (SubZyme L) (**SEQ ID NO: 44**), PB (SubZyme M) (**SEQ ID NO: 45**), Substrate E (**SEQ ID NO: 33**) and DNAzyme D (**SEQ ID NoO: 46**). The sequences are listed in the Sequence List. SubZyme L and SubZyme M were attached to magnetic beads (SubZyme L-MB and SubZyme M-MB) as described in Example One - Attachment Method Two. The T bags were manually prepared using polycarbonate membrane (0.8 µm) according to the diagram depicted in **Figure 3B****.** The results obtained are shown in **Figure 25****.**

The reactions contained 1 X PCR Buffer II, 1.5x concentration of SubZyme L-MB, 1 x T bag containing 1.5 x concentration of SubZyme M-MB, and 45 mM MgCl₂ in 60 µL final volume. Reactions either lacked initiating DNA (No DNAzyme control), or contained 2 nM, 200 pM or 20 pM of initiating DNAzyme (DNAzyme D). Reactions were incubated at 48°C for 60 minutes in 2 mL tubes on a heat block.

After the initial incubation, the samples were transferred from the heat-block and the T bags were removed from the tubes and discarded. Samples were briefly centrifuged and the tubes were placed on a magnetic rack to separate un-cleaved SubZyme L-MBs and cleaved partial substrate L-MB fragments from DNAzymes freed into solution. A 48 µL aliquot of the supernatant was transferred to a 96 well plate (Bio-Rad) and mixed with 2 µL of substrate Substrate E (0.2 µM final concentration). The plate was sealed and briefly centrifuged. Fluorescence from the Texas Red channel was measured in a CFX96 real time PCR detection system (Bio-Rad) with acquisition taking place every 10 seconds for 150 cycles at a constant temperature of 48°C.

The results presented in **Figure 25** are the averages from duplicates that were plotted using Microsoft Excel (Version 14) and demonstrate that the method is capable of detecting 2 nM (solid black line), 200 pM (black broken line) and 20 pM (black dotted line) of initiating DNAzyme. The signal is well differentiated from the no DNAzyme control (grey line).

### Example Nineteen: Demonstration of an Alternative Bead Material

In this experiment the strategy outlined in **Figure 15** was used to determine the generality of the T bag concept using silica beads (1 µm pore size) as an alternative bead material for the construction of T bags. Further, the aim was to determine whether or not SubZyme containment, DNAzyme diffusion and the release of surface-bound DNAzymes are possible using silica beads as an alternative to magnetic beads. PB (SubZyme F) is attached to 1 µm silica beads (SubZyme F-Beads) as described in Example One - Attachment Method 2, however, washing procedures were carried out using centrifugation instead of a magnetic rack. Each washing step entailed centrifuging the silica bead solution for 3 minutes at 10,000 rpm to pellet the beads followed by removing and discarding the supernatant.The T bags were manually prepared using either polycarbonate membrane (0.8 µm pore size) or PES membrane (0.8 µm pore size) according to the diagram depicted in **Figure 3B****.**

The oligonucleotides specific to this experiment include; PB (SubZyme F) (**SEQ ID NO: 6**), Substrate E (**SEQ ID NO: 33**) and DNAzyme A (**SEQ ID NO: 28**). The sequences are listed in the Sequence Listing.

The reactions contained 1X PCR Buffer II, 45 mM MgCl₂ in a 60 µL final volume. Reactions contained 1 x T bag made of either polycarbonate material (0.8 µm) or PES material (0.8 µm) containing 0.6 µL of SubZyme F-SBs. Reactions contained, or lacked, 2 nM of DNAzyme A. Reactions were incubated at 50°C for 20 minutes in 2 mL tubes on a heat block. After the initial incubation, the samples were transferred from the heat-block and the samples were briefly centrifuged. A 48 µL aliquot of the reaction solution was transferred to a 96 well plate (Bio-Rad) and mixed with 2 µL of Substrate E (0.2 µM final concentration). The plate was sealed and briefly centrifuged. Fluorescence from the Texas Red channel was measured in a CFX96 real time PCR detection system (Bio-Rad) with acquisition taking place every 10 seconds for 150 cycles at a constant temperature of 50°C.

The results shown in **Figure 26A** and **B** are the averages from duplicates that were plotted using Microsoft Excel (Version 14) and show negligible signal in the absence of initiating DNAzyme A. This indicates that SubZymes are unable to diffuse through the membranes whilst tethered to silica beads (1 µm diameter). There is a strong signal in the presence of initiating DNAzyme A, indicating that the initiating DNAzyme can diffuse into T bags made of either polycarbonate or PES membrane and then cleave SubZymes-SBs and release surface-bound DNAzymes. It also indicates that the released DNAzymes are able to diffuse out of the T bag and cleave fluorescent labelled substrate molecules. Similar results are observed for membranes made of different materials (PES and polycarbonate). The results indicate that the bead material and the membrane material can be composed of various materials as long as the bead is larger than the diameter of the membrane pores. The results shown in **Figure 26** illustrate that alternative bead materials such as silica, can be employed for the encapsulation and physical separation of SubZymes from other reaction components.

### Example Twenty: Cross Catalytic Cascade using SubZymes and Partzymes with End-point Detection

In this experiment the strategy outlined in **Figure 27** was used to detect target and amplify signal in the presence of a synthetic oligonucleotide target that is homologous to the Chlamydia trachomatis ompA gene. In this experiment PA (SubZyme J), PB (SubZyme K1) and PC (SubZyme K2) were attached to magnetic beads (SubZyme J-MB, SubZyme K1-MB and SubZyme K2-MB) as described in Example One - Attachment Method Two. The T bags were manually prepared using polycarbonate membrane (0.8 µm) according to the diagram depicted in Figure 3B. The initiating MNAzyme C (Cat C) comprised ompA Partzymes A1 and B1 (equivalent to C1 and C2 in **Figure 27**) and target ompA. The results obtained are shown in **Figure 28****.**

The oligonucleotides specific to this experiment include; PA (SubZyme J) (**SEQ ID NO: 10**), PB (SubZyme K1) (**SEQ ID NO**: 11), PC (SubZyme K2) (**SEQ ID NO**: 12), Substrate E (**SEQ ID NO: 33**), ompA Partzyme A1 (**SEQ ID NO**: 38), ompA Partzyme B1 (**SEQ ID NO**: 39), Target ompA Oligo 2 (**SEQ ID NO**: 40) and AF-B3 (Feedback Assembly Facilitator) (**SEQ ID NO**: 43). The sequences are listed in the Sequence Listing.

T bag reactions contained 50 nM ompA Partzyme A1, 50 nM ompA Partzyme B1, 1X PCR Buffer II, 45 mM MgCl₂, 2 nM Feedback Assembly Facilitator (AF-B3), 0.6 µL of PA (SubZyme J-MB), 0.6 µL of PC (SubZyme K2-MB) and 1 x T bag containing 0.6 µL of PB (SubZyme K1-MB) in 60 µL final volume. MNAzyme control reactions contained 50 nM ompA Partzyme A1, 50 nM ompA Partzyme B1, 1X PCR Buffer II and 45 mM MgCl₂ in 60 µL final volume. Reactions either lacked DNA (No DNA control), or contained 500 pM, 200 pM, 100 pM or 50 pM of target ompA oligo. Reactions were incubated at 50°C for 20 minutes in 2 mL tubes on a heat block.

After incubation, the samples were removed from the heat-block and were briefly centrifuged. An aliquot (40 µL) of the solution was transferred to a 96 well plate (Bio-Rad), the plate was sealed and briefly centrifuged. Fluorescence from the Texas red channel was measured in a CFX96 real time PCR detection system (Bio-Rad) with acquisition taking place every ten seconds for five cycles at a constant temperature of 50°C.
The results in **Figure 27** are presented as normalized fluorescence values calculated by subtracting the fluorescence from the no target control (NTC) reactions from the fluorescence from each sample reaction. Results are plotted using Microsoft Excel (Version 14) using the averages and standard deviations calculated from duplicate reactions that were each measured five times. The results demonstrate that the T Bag method depicted in **Figure 27** is capable of detecting 500 pM, 200 pM, 100 pM and 50 pM of synthetic target ompA oligonucleotide. The signals are well differentiated from the no target control. The detection of the same target concentrations are not as well distinguished from the no target controls using the MNAzyme control reactions indicating that the strategy depicted in **Figure 27** offers enhanced signal amplification. These results are consistent with the following scenario. In the presence of target oligonucleotide the Partzymes align and form an active initiating MNAzyme (Cat C). Cat C cleaves Sub A within PA, separating the Cat A from the surface of the MB. The Cat A can subsequently migrate through the T bag selectively permeable membrane where it can cleave Sub B within PB causing separation of Cat B1 from the surface of the MB. The Cat B1 is then free to migrate out of the T bag permeable membrane into solution where it can hybridise with Cat B2 and Feedback Assembly Facilitator (AF-B3) to form an active Feedback MNAzyme (Cat B) which can cleave Sub A within PA to continue feedback amplification. The results also indicate that in addition to initiating feedback amplification, Cat B and Cat C can also simultaneously cleave Sub A-FQ to generate fluorescence, thereby indicating the presence of target in real-time.

## Claims

1. A method for determining a presence of a nucleic acid target in a sample, the method comprising:
(a) providing a reaction mix comprising:
a population of polynucleotide A (PA) and a population of polynucleotide B (PB), wherein
each PA polynucleotide comprises a catalytic nucleic acid A and a catalytic nucleic acid substrate A,
each PB polynucleotide comprises a catalytic nucleic acid B and a catalytic nucleic acid substrate B,
the catalytic nucleic acid A is capable of catalytically modifying the catalytic nucleic acid substrate B and is unable to catalytically modify catalytic nucleic acid substrate A, and
the catalytic nucleic acid B is capable of catalytically modifying the catalytic nucleic acid substrate A and is unable to catalytically modify the catalytic nucleic acid substrate B;
a selectively permeable barrier capable of physically separating the PA from the PB, wherein the PA and PB are unable to permeate the selectively permeable barrier, and the PA and/or PB is/are mobile in the reaction mix; and
(i) two oligonucleotides each capable of hybridising specifically to the catalytic nucleic acid substrate A of a PA polynucleotide, at least one being capable of hybridising specifically to the target, to thereby form a catalytic nucleic acid C capable of cleaving the catalytic nucleic acid substrate A of the PA polynucleotide; or
(ii) a catalytic enzyme D capable of cleaving one or more strands of a nucleic acid duplex formed by hybridisation of the target to the PA; and
(b) contacting:
(i) the PA, the PB, and the oligonucleotide(s) with the sample, and using the selectively permeable barrier to separate the PA from the PB, wherein:
if the target is present in the sample, each of the two oligonucleotides hybridises to the catalytic nucleic acid substrate A of a PA polynucleotide and at least one further hybridises to the target to thereby form the catalytic nucleic acid C which cleaves the PA polynucleotide to thereby release the catalytic nucleic acid A of the PA polynucleotide which is consequently capable of movement through the permeable barrier;
wherein the released catalytic nucleic acid A moves through the permeable barrier, hybridises to the catalytic nucleic acid substrate B of a PB polynucleotide, and cleaves the PB polynucleotide to thereby release the catalytic nucleic acid B of the PB polynucleotide which is consequently capable of movement through the permeable barrier;
wherein the released catalytic nucleic acid B moves through the permeable barrier, hybridises to the catalytic nucleic acid substrate A of a second PA polynucleotide, and cleaves the second PA polynucleotide to thereby release the catalytic nucleic acid A of the second PA polynucleotide which is consequently capable of movement through the permeable barrier;
wherein detecting the cleavage of any said PA and/or PB polynucleotide indicates the presence of the target in the sample; and
(A) the catalytic nucleic acid A is a 10-23 DNAzyme and the catalytic nucleic acid B is an 8-17 DNAzyme; and
the catalytic nucleic acid substrate A is an 8-17 DNAzyme substrate and the catalytic nucleic acid substrate B is a 10-23 DNAzyme substrate; or
(B) the catalytic nucleic acid A is an 8-17 DNAzyme and the catalytic nucleic acid B is a 10-23 DNAzyme; and
the catalytic nucleic acid substrate A is a 10-23 DNAzyme substrate and the catalytic nucleic acid substrate B is an 8-17 DNAzyme substrate; or
(ii) the PA, the PB, and the catalytic enzyme D with the sample, and using the selectively permeable barrier to separate the PA from the PB, wherein:
if the target is present in the sample, the target hybridises with a PA polynucleotide to form the nucleic acid duplex which comprises a recognition and cleavage site for the catalytic enzyme D,
the cleavage site for the catalytic enzyme D is not within the catalytic nucleic acid A, and
the catalytic enzyme D binds to and cleaves the nucleic acid duplex at the cleavage site releasing the catalytic nucleic acid A of the PA polynucleotide which is consequently capable of movement through the permeable barrier,
wherein the released catalytic nucleic acid A moves through the permeable barrier, hybridises to the catalytic nucleic acid substrate B of a PB polynucleotide, and cleaves the PB polynucleotide to thereby release the catalytic nucleic acid B of the PB polynucleotide which is consequently capable of movement through the permeable barrier;
wherein the released catalytic nucleic acid B moves through the permeable barrier, hybridises to the catalytic nucleic acid substrate A of a second PA polynucleotide, and cleaves the second PA polynucleotide to thereby release the catalytic nucleic acid A of the second PA polynucleotide which is consequently capable of movement through the permeable barrier;
wherein detecting the cleavage of any said PA and/or PB polynucleotide indicates the presence of the target in the sample; and
(A) the catalytic nucleic acid A is a 10-23 DNAzyme and the catalytic nucleic acid B is an 8-17 DNAzyme; and
the catalytic nucleic acid substrate A is an 8-17 DNAzyme substrate and the catalytic nucleic acid substrate B is a 10-23 DNAzyme substrate; or
(B) the catalytic nucleic acid A is an 8-17 DNAzyme and the catalytic nucleic acid B is a 10-23 DNAzyme; and
the catalytic nucleic acid substrate A is a 10-23 DNAzyme substrate and the catalytic nucleic acid substrate B is an 8-17 DNAzyme substrate.

2. The method according to claim 1, wherein the catalytic nucleic acid C is not capable of cleaving the catalytic nucleic acid substrate B of the PB polynucleotide.

3. The method according to claim 1 or claim 2, wherein any of the PA and/or PB that are mobile in the reaction mix polynucleotides comprises:
an attached component and/or,
a region of self-complementarity providing a secondary structure, and/or
a charged moiety;
that prevents permeation of the polynucleotide across the selectively permeable barrier;
and optionally:
the cleavage of the catalytic nucleic acid substrate A releases the attached component or the region of self-complementarity from the catalytic nucleic acid A of the PA polynucleotide which is consequently capable of movement through the permeable barrier, and/or
the cleavage of the catalytic nucleic acid substrate B releases the attached component or the region of self-complementarity from the catalytic nucleic acid B of the PB polynucleotide which is consequently capable of movement through the permeable barrier.

4. The method according to any one of claims 1 to 3, wherein:
the catalytic nucleic acid C is a multi component nucleic acid enzyme (MNAzyme), and
the two oligonucleotides are two partzyme oligonucleotides capable of self-assembling to form the MNAzyme only in the presence of the target;
and wherein the two partzyme oligonucleotides each hybridise to the target during the self-assembly to form the MNAzyme.

5. The method according to any one of claims 1 to 4, wherein the catalytic enzyme D is an endonuclease or an exonuclease.

6. The method according to any one of claims 1 to 5, wherein the detection comprises
isolating the released catalytic nucleic acid A and/or the released catalytic nucleic acid B from the reaction mix;
applying the isolated catalytic nucleic acid(s) to a second reaction mix comprising catalytic nucleic acid substrate A and/or catalytic nucleic acid substrate B under conditions suitable for cleavage of the catalytic nucleic acid substrates by the catalytic nucleic acids; and
detecting cleavage of the catalytic nucleic acid substrates by the catalytic nucleic acids.

7. A method for determining a presence of a nucleic acid target in a sample, the method comprising:
(a) providing a reaction mix comprising:
(i) a population of polynucleotide A (PA), a population of polynucleotide B (PB) and a population of polynucleotide C (PC), wherein
each PA polynucleotide comprises a catalytic nucleic acid A and a catalytic nucleic acid substrate A,
each PB polynucleotide comprises a partzyme oligonucleotide B1 and a catalytic nucleic acid substrate B,
each PC polynucleotide comprises a partzyme oligonucleotide B2,
wherein:
a substrate arm of the partzyme oligonucleotide B1 is capable of hybridising to the catalytic nucleic acid substrate A,
a substrate arm of the partzyme oligonucleotide B2 is capable of hybridising to the catalytic nucleic acid substrate A,
the catalytic nucleic acid A is capable of catalytically modifying the catalytic nucleic acid substrate B and is unable to catalytically modify the catalytic nucleic acid substrate A;
(ii) an assembly facilitator oligonucleotide capable of hybridising to a sensor arm of partzyme oligonucleotide B1 and to a sensor arm of partzyme oligonucleotide B2;
(iii) a selectively permeable barrier capable of physically separating the PA from the PB, wherein the PA and PB are unable to permeate the selectively permeable barrier, and the PA, PB and PC and are mobile in the reaction mix; and either one of:
(iv) two partzyme oligonucleotides C1 and C2 each capable of hybridising specifically to the catalytic nucleic acid substrate A of a PA polynucleotide and at least one being capable of hybridising specifically to the target, to thereby form a catalytic nucleic acid C capable of cleaving the catalytic nucleic acid substrate A of the PA polynucleotide;
or
(v) a catalytic enzyme D capable of cleaving one or more strands of a nucleic acid duplex formed by hybridisation of the target to the PA; and
wherein the PB and/or PC polynucleotides that are mobile in the reaction mix comprise:
an attached component, optionally an aptamer hybridised to a ligand, a bead, a microparticle, or a nanoparticle and/or,
a region of self-complementarity providing a secondary structure, and/or
a charged moiety;
that prevents permeation of the polynucleotide across the selectively permeable barrier;
and
(b) contacting:
(i) the partzyme oligonucleotides C1 and C2 with the sample, and using the selectively permeable barrier to separate the PB from the PA, wherein:
if the target is present in the sample, the substrate arm of each of the partzyme oligonucleotides C1 and C2 hybridises to the catalytic nucleic acid substrate A of a PA polynucleotide and at least one of partzyme oligonucleotides C1 and C2 hybridises to the target forming the catalytic nucleic acid C which cleaves the PA polynucleotide to thereby release the catalytic nucleic acid A of the PA polynucleotide which is consequently capable of movement through the permeable barrier;
wherein:
the released catalytic nucleic acid A moves through the permeable barrier, hybridises to the catalytic nucleic acid substrate B of a PB polynucleotide, and cleaves the PB polynucleotide to thereby release the partzyme oligonucleotide B1 of the PB polynucleotide which is consequently capable of movement through the permeable barrier;
the released partzyme oligonucleotide B1 moves through the permeable barrier, the sensor arm of each of partzyme oligonucleotides B1 and B2 hybridises to the assembly facilitator oligonucleotide, and the substrate arm of each of partzyme oligonucleotides B1 and B2 hybridises to the catalytic nucleic acid substrate A of a second PA polynucleotide, thereby forming catalytic nucleic acid B which cleaves the second PA polynucleotide to thereby release the catalytic nucleic acid A of the second PA polynucleotide which is consequently capable of movement through the permeable barrier; and
detecting the cleavage of any said PA and/or PB polynucleotide indicates the presence of the target in the sample,
or
(ii) the catalytic enzyme D with the sample, and using the selectively permeable barrier to separate the PB from the PA, wherein:
if the target is present in the sample, the target hybridises with a PA polynucleotide to form the nucleic acid duplex which comprises a recognition and cleavage site for the catalytic enzyme D,
the cleavage site for the catalytic enzyme D is not within the catalytic nucleic acid A, and
the catalytic enzyme D binds to and cleaves the nucleic acid duplex at the cleavage site releasing the catalytic nucleic acid A of the PA polynucleotide which is consequently capable of movement through the permeable barrier;
wherein:
the released catalytic nucleic acid A moves through the permeable barrier, hybridises to the catalytic nucleic acid substrate B of a PB polynucleotide, and cleaves the PB polynucleotide to thereby release the partzyme oligonucleotide B1 of the PB polynucleotide which is consequently capable of movement through the permeable barrier;
the released partzyme oligonucleotide B1 moves through the permeable barrier, the sensor arm of each of partzyme oligonucleotides B1 and B2 hybridises to the assembly facilitator oligonucleotide, and the substrate arm of each of partzyme oligonucleotides B1 and B2 hybridises to the catalytic nucleic acid substrate A of a second PA polynucleotide, thereby forming catalytic nucleic acid B which cleaves the second PA polynucleotide to thereby release the catalytic nucleic acid A of the second PA polynucleotide which is consequently capable of movement through the permeable barrier; and
detecting the cleavage of any said PA and/or PB polynucleotide indicates the presence of the target in the sample.

8. The method according to claim 7, wherein:
(i) each PC polynucleotide comprises a partzyme oligonucleotide B2 and a catalytic nucleic acid substrate B that is cleavable by catalytic nucleic acid A,
the released catalytic nucleic acid A cleaves the PC polynucleotide to thereby release the partzyme oligonucleotide B2 of the PC polynucleotide, and
detecting the cleavage of any PC polynucleotide indicates the presence of the target in the sample; or
(ii) each PC polynucleotide comprises a partzyme oligonucleotide B2 and a catalytic nucleic acid substrate A, and
if the target is present in the sample, the substrate arm of each of the partzyme oligonucleotides C1 and C2 hybridises to the catalytic nucleic acid substrate A of the PC polynucleotide and at least one of the partzyme oligonucleotides C1 and C2 hybridises to the target forming the catalytic nucleic acid C which cleaves the PC polynucleotide to thereby release the partzyme oligonucleotide B2 of the PC polynucleotide; or
(iii) if the target is present in the sample, the target hybridises with a PC polynucleotide to form the nucleic acid duplex which comprises a recognition and cleavage site for the catalytic enzyme D,
the cleavage site for the catalytic enzyme D is not within the partzyme oligonucleotide B2 of the PC polynucleotide, and
the catalytic enzyme D binds to and cleaves the nucleic acid duplex at the cleavage site to thereby release the partzyme oligonucleotide B2 of the PC polynucleotide.

9. The method according to claim 7 or claim 8, wherein:
the catalytic nucleic acid C is a multi component nucleic acid enzyme (MNAzyme), and
the two partzyme oligonucleotides C1 and C2 are capable of self-assembling to form the MNAzyme only in the presence of the target,
wherein the two partzyme oligonucleotides C1 and C2 each hybridise to the target during the self-assembly to form the MNAzyme.

10. A composition for the detection of a target in a sample, the composition comprising:
a population of polynucleotide A (PA) and a population of polynucleotide B (PB), wherein:
each PA polynucleotide comprises a catalytic nucleic acid A and a catalytic nucleic acid substrate A,
each PB polynucleotide comprises a catalytic nucleic acid B and a catalytic nucleic acid substrate B,
the catalytic nucleic acid A is capable of catalytically modifying the catalytic nucleic acid substrate B and is unable to catalytically modify the catalytic nucleic acid substrate A, and
the catalytic nucleic acid B is capable of catalytically modifying the catalytic nucleic acid substrate A and is unable to catalytically modify the catalytic nucleic acid substrate B,
the PA and/or PB are mobile in the composition;
a selectively permeable barrier capable of separating the PA from the PB;
and
(A) the catalytic nucleic acid A is a 10-23 DNAzyme and the catalytic nucleic acid B is an 8-17 DNAzyme; and
the catalytic nucleic acid substrate A is an 8-17 DNAzyme substrate and the catalytic nucleic acid substrate B is a 10-23 DNAzyme substrate; or
(B) the catalytic nucleic acid A is an 8-17 DNAzyme and the catalytic nucleic acid B is a 10-23 DNAzyme; and
the catalytic nucleic acid substrate A is a 10-23 DNAzyme substrate and the catalytic nucleic acid substrate B is an 8-17 DNAzyme substrate.

11. A composition comprising:
(i) a population of polynucleotide A (PA), a population of polynucleotide B (PB) and a population of polynucleotide C (PC), wherein
each PA polynucleotide comprises a catalytic nucleic acid A and a catalytic nucleic acid substrate A, and is mobile in the composition,
each PB polynucleotide comprises a partzyme oligonucleotide B1 and a catalytic nucleic acid substrate B,
each PC polynucleotide comprises a partzyme oligonucleotide B2,
wherein:
a substrate arm of the partzyme oligonucleotide B1 is capable of hybridising to the catalytic nucleic acid substrate A,
a substrate arm of the partzyme oligonucleotide B2 is capable of hybridising to the catalytic nucleic acid substrate A, and
the catalytic nucleic acid A is capable of catalytically modifying the catalytic nucleic acid substrate B and is unable to catalytically modify the catalytic nucleic acid substrate A;
(ii) an assembly facilitator oligonucleotide capable of hybridising to a sensor arm of partzyme oligonucleotide B1 and to a sensor arm of partzyme oligonucleotide B2;
(iii) a selectively permeable barrier capable of physically separating the PA from the PB, wherein the PA and PB are unable to permeate the selectively permeable barrier,
wherein hybridisation of the partzyme oligonucleotide B1 substrate arm and the partzyme oligonucleotide B2 substrate arm to the catalytic nucleic acid substrate A, and hybridisation of the partzyme oligonucleotide B1 sensor arm and the partzyme oligonucleotide B2 sensor arm to the assembly facilitator, forms a catalytic nucleic acid B capable of cleaving the catalytic nucleic acid substrate A;
wherein
(A) the catalytic nucleic acid A is a 10-23 DNAzyme and the catalytic nucleic acid B is an 8-17 DNAzyme; and
the catalytic nucleic acid substrate A is an 8-17 DNAzyme substrate and the catalytic nucleic acid substrate B is a 10-23 DNAzyme substrate; or
(B) the catalytic nucleic acid A is an 8-17 DNAzyme and the catalytic nucleic acid B is a 10-23 DNAzyme; and
the catalytic nucleic acid substrate A is a 10-23 DNAzyme substrate and the catalytic nucleic acid substrate B is an 8-17 DNAzyme substrate;
and wherein:
(i) each PC polynucleotide comprises a partzyme oligonucleotide B2 and said catalytic nucleic acid substrate B capable of cleavage by catalytic nucleic acid A; or
(ii) each PC polynucleotide comprises a partzyme oligonucleotide B2 and said catalytic nucleic acid substrate A.

## Patentansprüche

1. Verfahren zum Bestimmen einer Anwesenheit eines Nukleinsäure-Targets in einer Probe, wobei das Verfahren umfasst:
(a) Bereitstellen eines Reaktionsgemisches, umfassend:
eine Population von Polynukleotid A (PA) und eine Population von Polynukleotid B (PB), wobei
jedes PA-Polynukleotid eine katalytische Nukleinsäure A und ein Katalytische-Nukleinsäure-Substrat A umfasst,
jedes PB-Polynukleotid eine katalytische Nukleinsäure B und ein Katalytische-Nukleinsäure-Substrat B umfasst,
die katalytische Nukleinsäure A imstande ist, das Katalytische-Nukleinsäure-Substrat B katalytisch zu modifizieren, und nicht imstande ist, das Katalytische-Nukleinsäure-Substrat A katalytisch zu modifizieren, und
die katalytische Nukleinsäure B imstande ist, das Katalytische-Nukleinsäure-Substrat A katalytisch zu modifizieren, und nicht imstande ist, das Katalytische-Nukleinsäure-Substrat B katalytisch zu modifizieren;
eine selektiv permeable Barriere, die imstande ist, das PA physisch von dem PB zu trennen, wobei das PA und PB nicht imstande sind, die selektiv permeable Barriere zu durchdringen, und das PA und/oder PB in dem Reaktionsgemisch beweglich ist/sind; und
(i) zwei Oligonukleotide, von denen jedes imstande ist, spezifisch mit dem Katalytische-Nukleinsäure-Substrat A eines PA-Polynukleotids zu hybridisieren, wobei mindestens eines imstande ist, spezifisch mit dem Target zu hybridisieren, um dadurch eine katalytische Nukleinsäure C zu bilden, die imstande ist, das Katalytische-Nukleinsäure-Substrat A des PA-Polynukleotids zu spalten; oder
(ii) ein katalytisches Enzym D, das imstande ist, einen oder mehrere Stränge eines Nukleinsäure-Duplexes zu spalten, der durch Hybridisierung des Targets mit dem PA gebildet wird; und
(b) Kontaktieren:
(i) des PA, des PB und des/der Oligonukleotid(e) mit der Probe und Verwenden der selektiv permeablen Barriere, um das PA von dem PB zu trennen, wobei:
wenn das Target in der Probe anwesend ist, jedes der zwei Oligonukleotide mit dem Katalytische-Nukleinsäure-Substrat A eines PA-Polynukleotids hybridisiert und mindestens eines ferner mit dem Target hybridisiert, um dadurch die katalytische Nukleinsäure C zu bilden, welche das PA-Polynukleotid spaltet, um dadurch die katalytische Nukleinsäure A des PA-Polynukleotids freizusetzen, welche infolgedessen zu Bewegung durch die permeable Barriere imstande ist;
wobei die freigesetzte katalytische Nukleinsäure A sich durch die permeable Barriere bewegt, mit dem Katalytische-Nukleinsäure-Substrat B eines PB-Polynukleotids hybridisiert, und das PB-Polynukleotid spaltet, um dadurch die katalytische Nukleinsäure B des PB-Polynukleotids freizusetzen, welche infolgedessen zu Bewegung durch die permeable Barriere imstande ist;
wobei die freigesetzte katalytische Nukleinsäure B sich durch die permeable Barriere bewegt, mit dem Katalytische-Nukleinsäure-Substrat A eines zweiten PA-Polynukleotids hybridisiert, und das zweite PA-Polynukleotid spaltet, um dadurch die katalytische Nukleinsäure A des zweiten PA-Polynukleotids freizusetzen, welche infolgedessen zu Bewegung durch die permeable Barriere imstande ist;
wobei Detektieren der Spaltung von PA- und/oder PB-Polynukleotid die Anwesenheit des Targets in der Probe anzeigt; und
(A) die katalytische Nukleinsäure A ein 10-23-DNAzym ist und die katalytische Nukleinsäure B ein 8-17-DNAzym ist; und
das Katalytische-Nukleinsäure-Substrat A ein 8-17-DNAzym-Substrat ist und das Katalytische-Nukleinsäure-Substrat B ein 10-23-DNAzym-Substrat ist; oder
(B) die katalytische Nukleinsäure A ein 8-17-DNAzym ist und die katalytische Nukleinsäure B ein 10-23-DNAzym ist; und
das Katalytische-Nukleinsäure-Substrat A ein 10-23-DNAzym-Substrat ist und das Katalytische-Nukleinsäure-Substrat B ein 8-17-DNAzym-Substrat ist; oder
(ii) des PA, des PB und des katalytischen Enzyms D mit der Probe, und Verwenden der selektiv permeablen Barriere, um das PA von dem PB zu trennen, wobei:
wenn das Target in der Probe anwesend ist, das Target mit einem PA-Polynukleotid hybridisiert, um den Nukleinsäure-Duplex zu bilden, welcher eine Erkennungs- und Spaltstelle für das katalytische Enzym D umfasst,
die Spaltstelle für das katalytische Enzym D nicht innerhalb der katalytischen Nukleinsäure A liegt, und
das katalytische Enzym D den Nukleinsäure-Duplex bindet und an der Spaltstelle spaltet, wodurch die katalytische Nukleinsäure A des PA-Polynukleotids freigesetzt wird, welche infolgedessen zu Bewegung durch die permeable Barriere imstande ist,
wobei die freigesetzte katalytische Nukleinsäure A sich durch die permeable Barriere bewegt, mit dem Katalytische-Nukleinsäure-Substrat B eines PB-Polynukleotids hybridisiert, und das PB-Polynukleotid spaltet, um dadurch die katalytische Nukleinsäure B des PB-Polynukleotids freizusetzen, welche infolgedessen zu Bewegung durch die permeable Barriere imstande ist;
wobei die freigesetzte katalytische Nukleinsäure B sich durch die permeable Barriere bewegt, mit dem Katalytische-Nukleinsäure-Substrat A eines zweiten PA-Polynukleotids hybridisiert, und das zweite PA-Polynukleotid spaltet, um dadurch die katalytische Nukleinsäure A des zweiten PA-Polynukleotids freizusetzen, welche infolgedessen zu Bewegung durch die permeable Barriere imstande ist;
wobei Detektieren der Spaltung von PA- und/oder PB-Polynukleotid die Anwesenheit des Targets in der Probe anzeigt; und
(A) die katalytische Nukleinsäure A ein 10-23-DNAzym ist und die katalytische Nukleinsäure B ein 8-17-DNAzym ist; und
das Katalytische-Nukleinsäure-Substrat A ein 8-17-DNAzym-Substrat ist und das Katalytische-Nukleinsäure-Substrat B ein 10-23-DNAzym-Substrat ist; oder
(B) die katalytische Nukleinsäure A ein 8-17-DNAzym ist und die katalytische Nukleinsäure B ein 10-23-DNAzym ist; und
das Katalytische-Nukleinsäure-Substrat A ein 10-23-DNAzym-Substrat ist und das Katalytische-Nukleinsäure-Substrat B ein 8-17-DNAzym-Substrat ist.

2. Verfahren nach Anspruch 1, wobei die katalytische Nukleinsäure C nicht imstande ist, das Katalytische-Nukleinsäure-Substrat B des PB-Polynukleotids zu spalten.

3. Verfahren nach Anspruch 1 oder Anspruch 2, wobei ein beliebiges aus PA und/oder PB, die in dem Reaktionsgemisch bewegliche Polynukleotide sind, umfasst:
eine verbundene Komponente und/oder
einen Bereich der Selbstkomplementarität, der eine Sekundärstruktur bereitstellt, und/oder
einen geladenen Rest;
die/der Durchdringen des Polynukleotids durch die selektiv permeable Barriere verhindert;
und gegebenenfalls:
die Spaltung des Katalytische-Nukleinsäure-Substrats A die verbundene Komponente oder den Bereich der Selbstkomplementarität von der katalytischen Nukleinsäure A des PA-Polynukleotids freisetzt, welche infolgedessen zu Bewegung durch die permeable Barriere imstande ist, und/oder
die Spaltung des Katalytische-Nukleinsäure-Substrats B die verbundene Komponente oder den Bereich der Selbstkomplementarität von der katalytischen Nukleinsäure B des PB-Polynukleotids freisetzt, welche infolgedessen zu Bewegung durch die permeable Barriere imstande ist.

4. Verfahren nach einem der Ansprüche 1 bis 3, wobei:
die katalytische Nukleinsäure C ein Mehrkomponenten-Nukleinsäureenzym (MNAzym) ist, und
die zwei Oligonukleotide zwei Partzym-Oligonukleotide sind, die nur in der Anwesenheit des Targets imstande sind, sich selbst zu assemblieren, um das MNAzym zu bilden;
und wobei die zwei Partzym-Oligonukleotide während der Selbstassemblierung jeweils mit dem Target hybridisieren, um das MNAzym zu bilden.

5. Verfahren nach einem der Ansprüche 1 bis 4, wobei das katalytische Enzym D eine Endonuklease oder eine Exonuklease ist.

6. Verfahren nach einem der Ansprüche 1 bis 5, wobei die Detektion umfasst:
Isolieren der freigesetzten katalytischen Nukleinsäure A und/oder der freigesetzten katalytischen Nukleinsäure B aus dem Reaktionsgemisch;
Zuführen der isolierten katalytischen Nukleinsäure(n) zu einem zweiten Reaktionsgemisch, das ein Katalytische-Nukleinsäure-Substrat A und/oder ein Katalytische-Nukleinsäure-Substrat B unter Bedingungen umfasst, die geeignet sind für Spaltung der Katalytische-Nukleinsäure-Substrate durch die katalytischen Nukleinsäuren; und
Detektieren von Spaltung der Katalytische-Nukleinsäure-Substrate durch die katalytischen Nukleinsäuren.

7. Verfahren zum Bestimmen einer Anwesenheit eines Nukleinsäure-Targets in einer Probe, wobei das Verfahren umfasst:
(a) Bereitstellen eines Reaktionsgemischs umfassend:
(i) eine Population von Polynukleotid A (PA), eine Population von Polynukleotid B (PB) und eine Population von Polynukleotid C (PC), wobei
jedes PA-Polynukleotid eine katalytische Nukleinsäure A und ein Katalytische-Nukleinsäure-Substrat A umfasst,
jedes PB-Polynukleotid ein Partzym-Oligonukleotid B1 und ein Katalytische-Nukleinsäure-Substrat B umfasst,
jedes PC-Polynukleotid ein Partzym-Oligonukleotid B2 umfasst,
wobei:
ein Substratarm des Partzym-Oligonukleotids B1 imstande ist, mit dem Katalytische-Nukleinsäure-Substrat A zu hybridisieren,
ein Substratarm des Partzym-Oligonukleotids B2 imstande ist, mit dem Katalytische-Nukleinsäure-Substrat A zu hybridisieren,
die katalytische Nukleinsäure A imstande ist, das Katalytische-Nukleinsäure-Substrat B katalytisch zu modifizieren, und nicht imstande ist, das Katalytische-Nukleinsäure-Substrat A katalytisch zu modifizieren;
(ii) ein Assemblierungs-Unterstützer-Oligonukleotid, das imstande ist, mit einem Sensorarm des Partzym-Oligonukleotids B1 und mit einem Sensorarm des Partzym-Oligonukleotids B2 zu hybridisieren;
(iii) eine selektiv permeable Barriere, die imstande ist, das PA physisch von dem PB zu trennen, wobei das PA und PB nicht imstande sind, die selektiv permeable Barriere zu durchdringen, und das PA, PB und PC in dem Reaktionsgemisch beweglich sind; und eins aus entweder:
(iv) zwei Partzym-Oligonukleotide C1 und C2, von denen jedes imstande ist, spezifisch mit dem Katalytische-Nukleinsäure-Substrat A eines PA-Polynukleotids zu hybridisieren, und mindestens eines imstande ist, spezifisch mit dem Target zu hybridisieren, um dadurch eine katalytische Nukleinsäure C zu bilden, die imstande ist, das Katalytische-Nukleinsäure-Substrat A des PA-Polynukleotids zu spalten;
oder
(v) ein katalytisches Enzym D, das imstande ist, einen oder mehrere Stränge eines Nukleinsäure-Duplexes zu spalten, der durch Hybridisierung des Targets mit dem PA gebildet wird; und
wobei die PB- und/oder PC-Polynukleotide, die in dem Reaktionsgemisch beweglich sind, umfassen:
eine verbundene Komponente, gegebenenfalls ein Aptamer, das mit einem Liganden hybridisiert ist, ein Kügelchen, einen Mikropartikel oder einen Nanopartikel und/oder
einen Bereich der Selbstkomplementarität, der eine Sekundärstruktur bereitstellt, und/oder
einen geladenen Rest;
die/der Durchdringen des Polynukleotids durch die selektiv permeable Barriere verhindert;
und
(b) Kontaktieren:
(i) der Partzym-Oligonukleotide C1 und C2 mit der Probe, und Verwenden der selektiv permeablen Barriere, um das PB von dem PA zu trennen, wobei:
wenn das Target in der Probe anwesend ist, der Substratarm jedes der Partzym-Oligonukleotide C1 und C2 mit dem Katalytische-Nukleinsäure-Substrat A eines PA-Polynukleotids hybridisiert und mindestens eines der Partzym-Oligonukleotide C1 und C2 mit dem Target hybridisiert, wodurch die katalytische Nukleinsäure C gebildet wird, welche das PA-Polynukleotid spaltet, um dadurch die katalytische Nukleinsäure A des PA-Polynukleotids freizusetzen, welche infolgedessen zu Bewegung durch die permeable Barriere imstande ist;
wobei:
die freigesetzte katalytische Nukleinsäure A sich durch die permeable Barriere bewegt, mit dem Katalytische-Nukleinsäure-Substrat B eines PB-Polynukleotids hybridisiert und das PB-Polynukleotid spaltet, um dadurch das Partzym-Oligonukleotid B1 des PB-Polynukleotids freizusetzen, welches infolgedessen zu Bewegung durch die permeable Barriere imstande ist;
das freigesetzte Partzym-Oligonukleotid B1 sich durch die permeable Barriere bewegt, der Sensorarm jedes der Partzym-Oligonukleotide B1 und B2 mit dem Assemblierungs-Unterstützer-Oligonukleotid hybridisiert und der Substratarm jedes der Partzym-Oligonukleotide B1 und B2 mit dem Katalytische-Nukleinsäure-Substrat A eines zweiten PA-Polynukleotids hybridisiert, wodurch katalytische Nukleinsäure B gebildet wird, welche das zweite PA-Polynukleotid spaltet, um dadurch die katalytische Nukleinsäure A des zweiten PA-Polynukleotids freizusetzen, welche infolgedessen zu Bewegung durch die permeable Barriere imstande ist; und
Detektieren der Spaltung von PA- und/oder PB-Polynukleotid die Anwesenheit des Targets in der Probe anzeigt,
oder
(ii) des katalytischen Enzyms D mit der Probe, und Verwenden der selektiv permeablen Barriere, um das PB von dem PA zu trennen, wobei:
wenn das Target in der Probe anwesend ist, das Target mit einem PA-Polynukleotid hybridisiert, um den Nukleinsäure-Duplex zu bilden, welcher eine Erkennungs- und Spaltstelle für das katalytische Enzym D umfasst,
die Spaltstelle für das katalytische Enzym D nicht innerhalb der katalytischen Nukleinsäure A liegt, und
das katalytische Enzym D den Nukleinsäure-Duplex bindet und an der Spaltstelle spaltet, wodurch die katalytische Nukleinsäure A des PA-Polynukleotids freigesetzt wird, welche infolgedessen zu Bewegung durch die permeable Barriere imstande ist;
wobei:
die freigesetzte katalytische Nukleinsäure A sich durch die permeable Barriere bewegt, mit dem Katalytische-Nukleinsäure-Substrat B eines PB-Polynukleotids hybridisiert und das PB-Polynukleotid spaltet, um dadurch das Partzym-Oligonukleotid B1 des PB-Polynukleotids freizusetzen, welches infolgedessen zu Bewegung durch die permeable Barriere imstande ist;
das freigesetzte Partzym-Oligonukleotid B1 sich durch die permeable Barriere bewegt, der Sensorarm jedes der Partzym-Oligonukleotide B1 und B2 mit dem Assemblierungs-Unterstützer-Oligonukleotid hybridisiert und der Substratarm jedes der Partzym-Oligonukleotide B1 und B2 mit dem Katalytische-Nukleinsäure-Substrat A eines zweiten PA-Polynukleotids hybridisiert, wodurch katalytische Nukleinsäure B gebildet wird, welche das zweite PA-Polynukleotid spaltet, um dadurch die katalytische Nukleinsäure A des zweiten PA-Polynukleotids freizusetzen, welche infolgedessen zu Bewegung durch die permeable Barriere imstande ist; und
Detektieren der Spaltung von PA- und/oder PB-Polynukleotid die Anwesenheit des Targets in der Probe anzeigt.

8. Verfahren nach Anspruch 7, wobei
(i) jedes PC-Polynukleotid ein Partzym-Oligonukleotid B2 und ein Katalytische-Nukleinsäure-Substrat B umfasst, das durch katalytische Nukleinsäure A spaltbar ist,
die freigesetzte katalytische Nukleinsäure A das PC-Polynukleotid spaltet, um dadurch das Partzym-Oligonukleotid B2 des PC-Polynukleotids freizusetzen, und
Detektieren der Spaltung von PC-Polynukleotid die Anwesenheit des Targets in der Probe anzeigt; oder
(ii) jedes PC-Polynukleotid ein Partzym-Oligonukleotid B2 und ein Katalytische-Nukleinsäure-Substrat A umfasst, und
wenn das Target in der Probe anwesend ist, der Substratarm jedes der Partzym-Oligonukleotide C1 und C2 mit dem Katalytische-Nukleinsäure-Substrat A des PC-Polynukleotids hybridisiert und mindestens eines der Partzym-Oligonukleotide C1 und C2 mit dem Target hybridisiert, wodurch die katalytische Nukleinsäure C gebildet wird, welche das PC-Polynukleotid spaltet, um dadurch das Partzym-Oligonukleotid B2 des PC-Polynukleotids freizusetzen; oder
(iii) wenn das Target in der Probe anwesend ist, das Target mit einem PC-Polynukleotid hybridisiert, um den Nukleinsäure-Duplex zu bilden, welcher eine Erkennungs- und Spaltstelle für das katalytische Enzym D umfasst,
die Spaltstelle für das katalytische Enzym D nicht innerhalb des Partzym-Oligonukleotids B2 des PC-Polynukleotids liegt, und
das katalytische Enzym D den Nukleinsäure-Duplex bindet und an der Spaltstelle spaltet, um dadurch das Partzym-Oligonukleotid B2 des PC-Polynukleotids freizusetzen.

9. Verfahren nach Anspruch 7 oder Anspruch 8, wobei:
die katalytische Nukleinsäure C ein Mehrkomponenten-Nukleinsäureenzym (MNAzym) ist, und
die zwei Partzym-Oligonukleotide C1 und C2 nur in der Anwesenheit des Targets imstande sind, sich selbst zu assemblieren, um das MNAzym zu bilden,
wobei die zwei Partzym-Oligonukleotide C1 und C2 jeweils mit dem Target während der Selbstassemblierung hybridisieren, um das MNAzym zu bilden.

10. Zusammensetzung für die Detektion eines Targets in einer Probe, wobei die Zusammensetzung umfasst:
eine Population von Polynukleotid A (PA) und eine Population von Polynukleotid B (PB),
wobei:
jedes PA-Polynukleotid eine katalytische Nukleinsäure A und ein Katalytische-Nukleinsäure-Substrat A umfasst,
jedes PB-Polynukleotid eine katalytische Nukleinsäure B und ein Katalytische-Nukleinsäure-Substrat B umfasst,
die katalytische Nukleinsäure A imstande ist, das Katalytische-Nukleinsäure-Substrat B katalytisch zu modifizieren, und nicht imstande ist, das Katalytische-Nukleinsäure-Substrat A katalytisch zu modifizieren, und
die katalytische Nukleinsäure B imstande ist, das Katalytische-Nukleinsäure-Substrat A katalytisch zu modifizieren, und nicht imstande ist, das Katalytische-Nukleinsäure-Substrat B katalytisch zu modifizieren;
das PA und/oder PB in der Zusammensetzung beweglich sind;
eine selektiv permeable Barriere, die imstande ist, das PA von dem PB zu trennen,
und
(A) die katalytische Nukleinsäure A ein 10-23-DNAzym ist und die katalytische Nukleinsäure B ein 8-17-DNAzym ist; und
das Katalytische-Nukleinsäure-Substrat A ein 8-17-DNAzym-Substrat ist und das Katalytische-Nukleinsäure-Substrat B ein 10-23-DNAzym-Substrat ist; oder
(B) die katalytische Nukleinsäure A ein 8-17-DNAzym ist und die katalytische Nukleinsäure B ein 10-23-DNAzym ist; und
das Katalytische-Nukleinsäure-Substrat A ein 10-23-DNAzym-Substrat ist und
das Katalytische-Nukleinsäure-Substrat B ein 8-17-DNAzym-Substrat ist.

11. Zusammensetzung umfassend:
(i) eine Population von Polynukleotid A (PA), eine Population von Polynukleotid B (PB) und eine Population von Polynukleotid C (PC), wobei
jedes PA-Polynukleotid eine katalytische Nukleinsäure A und ein Katalytische-Nukleinsäure-Substrat A umfasst, und in der Zusammensetzung beweglich ist,
jedes PB-Polynukleotid ein Partzym-Oligonukleotid B1 und ein Katalytische-Nukleinsäure-Substrat B umfasst,
jedes PC-Polynukleotid ein Partzym-Oligonukleotid B2 umfasst,
wobei:
ein Substratarm des Partzym-Oligonukleotids B1 imstande ist, mit dem Katalytische-Nukleinsäure-Substrat A zu hybridisieren,
ein Substratarm des Partzym-Oligonukleotids B2 imstande ist, mit dem Katalytische-Nukleinsäure-Substrat A zu hybridisieren, und
die katalytische Nukleinsäure A imstande ist, das Katalytische-Nukleinsäure-Substrat B katalytisch zu modifizieren, und nicht imstande ist, das Katalytische-Nukleinsäure-Substrat A katalytisch zu modifizieren;
(ii) ein Assemblierungs-Unterstützer-Oligonukleotid, das imstande ist, mit einem Sensorarm des Partzym-Oligonukleotids B1 und mit einem Sensorarm des Partzym-Oligonukleotids B2 zu hybridisieren;
(iii) eine selektiv permeable Barriere, die imstande ist, das PA physisch von dem PB zu trennen, wobei das PA und PB nicht imstande sind, die selektiv permeable Barriere zu durchdringen,
wobei Hybridisierung des Partzym-Oligonukleotid-B1-Substratarms und des Partzym-Oligonukleotid-B2-Substratarms mit dem Katalytische-Nukleinsäure-Substrat A und Hybridisierung des Partzym-Oligonukleotid-B1-Sensorarms und des Partzym-Oligonukleotid-B2-Sensorarms mit dem Assemblierungs-Unterstützer eine katalytische Nukleinsäure B bildet, die imstande ist, das Katalytische-Nukleinsäure-Substrat A zu spalten;
wobei
(A) die katalytische Nukleinsäure A ein 10-23-DNAzym ist und die katalytische Nukleinsäure B ein 8-17-DNAzym ist; und
das Katalytische-Nukleinsäure-Substrat A ein 8-17-DNAzym-Substrat ist und das Katalytische-Nukleinsäure-Substrat B ein 10-23-DNAzym-Substrat ist; oder
(B) die katalytische Nukleinsäure A ein 8-17-DNAzym ist und die katalytische Nukleinsäure B ein 10-23-DNAzym ist; und
das Katalytische-Nukleinsäure-Substrat A ein 10-23-DNAzym-Substrat ist und das Katalytische-Nukleinsäure-Substrat B ein 8-17-DNAzym-Substrat ist;
und wobei:
(i) jedes PC-Polynukleotid ein Partzym-Oligonukleotid B2 und das Katalytische-Nukleinsäure-Substrat B umfasst, das zu Spaltung durch die katalytische Nukleinsäure A imstande ist; oder
(ii) jedes PC-Polynukleotid ein Partzym-Oligonukleotid B2 und das Katalytische-Nukleinsäure-Substrat A umfasst.

## Revendications

1. Procédé pour déterminer la présence d'une cible d'acide nucléique dans un échantillon, le procédé comprenant les étapes consistant à :
(a) mettre à disposition un mélange réactionnel comprenant :
une population de polynucléotide A (PA) et une population de polynucléotide B (PB), dans lesquelles
chaque polynucléotide PA comprend un acide nucléique catalytique A et un substrat d'acide nucléique catalytique A,
chaque polynucléotide PB comprend un acide nucléique catalytique B et un substrat d'acide nucléique catalytique B,
l'acide nucléique catalytique A est capable de modifier de manière catalytique le substrat d'acide nucléique catalytique B et est incapable de modifier de manière catalytique le substrat d'acide nucléique catalytique A, et
l'acide nucléique catalytique B est capable de modifier de manière catalytique le substrat d'acide nucléique catalytique A et est incapable de modifier de manière catalytique le substrat d'acide nucléique catalytique B ;
une barrière sélectivement perméable capable de physiquement séparer le PA du PB, où les PA et PB sont incapables de pénétrer la barrière sélectivement perméable, et le PA et/ou PB est/sont mobile(s) dans le mélange réactionnel ; et
(i) deux oligonucléotides, chacun étant capable de s'hybrider spécifiquement au substrat d'acide nucléique catalytique A d'un polynucléotide PA, au moins un étant capable de s'hybrider spécifiquement à la cible, pour ainsi former un acide nucléique catalytique C capable de cliver le substrat d'acide nucléique catalytique A du polynucléotide PA ; ou
(ii) une enzyme catalytique D capable de cliver un ou plusieurs brins d'un duplex d'acide nucléique formé par une hybridation de la cible au PA ; et
(b) mettre en contact :
(i) le PA, le PB, et le ou les oligonucléotides avec l'échantillon, et en utilisant la barrière sélectivement perméable pour séparer le PA du PB, où :
si la cible est présente dans l'échantillon, chacun des deux oligonucléotides s'hybride au substrat d'acide nucléique catalytique A d'un polynucléotide PA et au moins un s'hybride en outre à la cible pour ainsi former l'acide nucléique catalytique C qui clive le polynucléotide PA pour ainsi libérer l'acide nucléique catalytique A du polynucléotide PA qui est en conséquence capable de passer à travers la barrière perméable ;
où l'acide nucléique catalytique A libéré passe à travers la barrière perméable, s'hybride au substrat d'acide nucléique catalytique B d'un polynucléotide PB, et clive le polynucléotide PB pour ainsi libérer l'acide nucléique catalytique B du polynucléotide PB qui est en conséquence capable de passer à travers la barrière perméable ;
où l'acide nucléique catalytique B libéré passe à travers la barrière perméable, s'hybride au substrat d'acide nucléique catalytique A d'un deuxième polynucléotide PA, et clive le deuxième polynucléotide PA pour ainsi libérer l'acide nucléique catalytique A du deuxième polynucléotide PA qui est en conséquence capable de passer à travers la barrière perméable ;
où la détection du clivage de l'un quelconque dudit polynucléotide PA et/ou PB indique la présence de la cible dans l'échantillon ; et
(A) l'acide nucléique catalytique A est un ADNzyme 10-23 et l'acide nucléique catalytique B est un ADNzyme 8-17 ; et
le substrat d'acide nucléique catalytique A est un substrat d'ADNzyme 8-17 et le substrat d'acide nucléique catalytique B est un substrat d'ADNzyme 10-23 ; ou
(B) l'acide nucléique catalytique A est un ADNzyme 8-17 et l'acide nucléique catalytique B est un ADNzyme 10-23 ; et
le substrat d'acide nucléique catalytique A est un substrat d'ADNzyme 10-23 et le substrat d'acide nucléique catalytique B est un substrat d'ADNzyme 8-17 ; ou
(ii) le PA, le PB, et l'enzyme catalytique D avec l'échantillon, et en utilisant la barrière sélectivement perméable pour séparer le PA du PB, où :
si la cible est présente dans l'échantillon, la cible s'hybride à un polynucléotide PA pour former le duplex d'acide nucléique qui comprend un site de reconnaissance et de clivage pour l'enzyme catalytique D,
le site de clivage pour l'enzyme catalytique D n'est pas compris au sein de l'acide nucléique catalytique A, et
l'enzyme catalytique D se lie à et clive le duplex d'acide nucléique au niveau du site de clivage en libérant l'acide nucléique catalytique A du polynucléotide PA qui est en conséquence capable de passer à travers la barrière perméable,
où l'acide nucléique catalytique A libéré passe à travers la barrière perméable, s'hybride au substrat d'acide nucléique catalytique B d'un polynucléotide PB, et clive le polynucléotide PB pour ainsi libérer l'acide nucléique catalytique B du polynucléotide PB qui est en conséquence capable de passer à travers la barrière perméable ;
où l'acide nucléique catalytique B libéré passe à travers la barrière perméable, s'hybride au substrat d'acide nucléique catalytique A d'un deuxième polynucléotide PA, et clive le deuxième polynucléotide PA pour ainsi libérer l'acide nucléique catalytique A du deuxième polynucléotide PA qui est en conséquence capable de passer à travers la barrière perméable ;
où la détection du clivage de l'un quelconque dudit polynucléotide PA et/ou PB indique la présence de la cible dans l'échantillon ; et
(A) l'acide nucléique catalytique A est un ADNzyme 10-23 et l'acide nucléique catalytique B est un ADNzyme 8-17 ; et
le substrat d'acide nucléique catalytique A est un substrat d'ADNzyme 8-17 et le substrat d'acide nucléique catalytique B est un substrat d'ADNzyme 10-23 ; ou
(B) l'acide nucléique catalytique A est un ADNzyme 8-17 et l'acide nucléique catalytique B est un ADNzyme 10-23 ; et
le substrat d'acide nucléique catalytique A est un substrat d'ADNzyme 10-23 et le substrat d'acide nucléique catalytique B est un substrat d'ADNzyme 8-17.

2. Procédé selon la revendication 1, dans lequel l'acide nucléique catalytique C n'est pas capable de cliver le substrat d'acide nucléique catalytique B du polynucléotide PB.

3. Procédé selon la revendication 1 ou la revendication 2, dans lequel l'un quelconque du PA et/ou PB qui sont mobiles dans les polynucléotides du mélange réactionnel comprend :
un composant attaché et/ou,
une région d'auto-complémentarité fournissant une structure secondaire, et/ou
un fragment chargé ;
qui empêche la perméation du polynucléotide à travers la barrière sélectivement perméable ;
et facultativement :
le clivage du substrat d'acide nucléique catalytique A libère le composant attaché ou la région d'auto-complémentarité à partir de l'acide nucléique catalytique A du polynucléotide PA qui est en conséquence capable de passer à travers la barrière perméable, et/ou
le clivage du substrat d'acide nucléique catalytique B libère le composant attaché ou la région d'auto-complémentarité à partir de l'acide nucléique catalytique B du polynucléotide PB qui est en conséquence capable de passer à travers la barrière perméable.

4. Procédé selon l'une quelconque des revendications 1 à 3, dans lequel :
l'acide nucléique catalytique C est une enzyme d'acide nucléique à composants multiples (MNAzyme), et
les deux oligonucléotides sont deux oligonucléotides de partzyme capables de s'auto-assembler pour former la MNAzyme uniquement en présence de la cible ;
et dans lequel les deux oligonucléotides de partzyme s'hybrident chacun à la cible pendant l'auto-assemblage pour former la MNAzyme.

5. Procédé selon l'une quelconque des revendications 1 à 4, dans lequel l'enzyme catalytique D est une endonucléase ou une exonucléase.

6. Procédé selon l'une quelconque des revendications 1 à 5, dans lequel la détection comprend les étapes consistant à
isoler l'acide nucléique catalytique A libéré et/ou l'acide nucléique catalytique B libéré à partir du mélange réactionnel ;
appliquer le ou les acides nucléique catalytiques isolés à un deuxième mélange réactionnel comprenant le substrat d'acide nucléique catalytique A et/ou le substrat d'acide nucléique catalytique B dans des conditions appropriées pour permettre un clivage des substrats d'acide nucléique catalytique par les acides nucléiques catalytiques ; et
détecter le clivage des substrats d'acide nucléique catalytique par les acides nucléiques catalytiques.

7. Procédé pour déterminer la présence d'une cible d'acide nucléique dans un échantillon, le procédé comprenant les étapes consistant à :
(a) mettre à disposition un mélange réactionnel comprenant :
(i) une population de polynucléotide A (PA), une population de polynucléotide B (PB) et une population de polynucléotide C (PC), dans lesquelles
chaque polynucléotide PA comprend un acide nucléique catalytique A et un substrat d'acide nucléique catalytique A,
chaque polynucléotide PB comprend un oligonucléotide de partzyme B1 et un substrat d'acide nucléique catalytique B,
chaque polynucléotide PC comprend un oligonucléotide de partzyme B2,
dans lequel :
un bras substrat de l'oligonucléotide de partzyme B1 est capable de s'hybrider au substrat d'acide nucléique catalytique A,
un bras substrat de l'oligonucléotide de partzyme B2 est capable de s'hybrider au substrat d'acide nucléique catalytique A,
l'acide nucléique catalytique A est capable de modifier de manière catalytique le substrat d'acide nucléique catalytique B et est incapable de modifier de manière catalytique le substrat d'acide nucléique catalytique A ;
(ii) un oligonucléotide facilitateur d'assemblage capable de s'hybrider à un bras capteur de l'oligonucléotide de partzyme B1 et à un bras capteur de l'oligonucléotide de partzyme B2 ;
(iii) une barrière sélectivement perméable capable de physiquement séparer le PA du PB, où les PA et PB sont incapables de pénétrer la barrière sélectivement perméable, et les PA, PB et PC sont mobiles dans le mélange réactionnel ; et l'un quelconque de :
(iv) deux oligonucléotides de partzyme C1 et C2, chacun étant capable de s'hybrider spécifiquement au substrat d'acide nucléique catalytique A d'un polynucléotide PA et au moins un étant capable de s'hybrider spécifiquement à la cible, pour ainsi former un acide nucléique catalytique C capable de cliver le substrat d'acide nucléique catalytique A du polynucléotide PA ;
ou
(v) une enzyme catalytique D capable de cliver un ou plusieurs brins d'un duplex d'acide nucléique formé par une hybridation de la cible au PA ; et
dans lequel les polynucléotides PB et/ou PC qui sont mobiles dans le mélange réactionnel comprennent :
un composant attaché, facultativement un aptamère hybridé à un ligand, une bille, une microparticule, ou une nanoparticule et/ou,
une région d'auto-complémentarité fournissant une structure secondaire, et/ou
un fragment chargé ;
qui empêche la perméation du polynucléotide à travers la barrière sélectivement perméable ;
et
(b) mettre en contact :
(i) les oligonucléotides de partzyme C1 et C2 avec l'échantillon, et en utilisant la barrière sélectivement perméable pour séparer le PB du PA, dans lequel :
si la cible est présente dans l'échantillon, le bras substrat de chacun des oligonucléotides de partzyme C1 et C2 s'hybride au substrat d'acide nucléique catalytique A d'un polynucléotide PA et au moins un des oligonucléotides de partzyme C1 et C2 s'hybride à la cible en formant l'acide nucléique catalytique C qui clive le polynucléotide PA pour ainsi libérer l'acide nucléique catalytique A du polynucléotide PA qui est en conséquence capable de passer à travers la barrière perméable ;
dans lequel :
l'acide nucléique catalytique A libéré passe à travers la barrière perméable, s'hybride au substrat d'acide nucléique catalytique B d'un polynucléotide PB, et clive le polynucléotide PB pour ainsi libérer l'oligonucléotide de partzyme B1 du polynucléotide PB qui est en conséquence capable de passer à travers la barrière perméable ;
l'oligonucléotide de partzyme B1 libéré passe à travers la barrière perméable, le bras capteur de chacun des oligonucléotides de partzyme B1 et B2 s'hybride à l'oligonucléotide facilitateur d'assemblage, et le bras substrat de chacun des oligonucléotides de partzyme B1 et B2 s'hybride au substrat d'acide nucléique catalytique A d'un deuxième polynucléotide PA, en formant ainsi un acide nucléique catalytique B qui clive le deuxième polynucléotide PA pour ainsi libérer l'acide nucléique catalytique A du deuxième polynucléotide PA qui est en conséquence capable de passer à travers la barrière perméable ; et
la détection du clivage de l'un quelconque dudit polynucléotide PA et/ou PB indique la présence de la cible dans l'échantillon,
ou
(ii) l'enzyme catalytique D avec l'échantillon, et en utilisant la barrière sélectivement perméable pour séparer le PB du PA, dans lequel :
si la cible est présente dans l'échantillon, la cible s'hybride à un polynucléotide PA pour former le duplex d'acide nucléique qui comprend un site de reconnaissance et de clivage pour l'enzyme catalytique D,
le site de clivage pour l'enzyme catalytique D n'est pas compris au sein de l'acide nucléique catalytique A, et
l'enzyme catalytique D se lie à et clive le duplex d'acide nucléique au niveau du site de clivage en libérant l'acide nucléique catalytique A du polynucléotide PA qui est en conséquence capable de passer à travers la barrière perméable ;
dans lequel :
l'acide nucléique catalytique A libéré passe à travers la barrière perméable, s'hybride au substrat d'acide nucléique catalytique B d'un polynucléotide PB, et clive le polynucléotide PB pour ainsi libérer l'oligonucléotide de partzyme B1 du polynucléotide PB qui est en conséquence capable de passer à travers la barrière perméable ;
l'oligonucléotide de partzyme B1 libéré passe à travers la barrière perméable, le bras capteur de chacun des oligonucléotides de partzyme B1 et B2 s'hybride à l'oligonucléotide facilitateur d'assemblage, et le bras substrat de chacun des oligonucléotides de partzyme B1 et B2 s'hybride au substrat d'acide nucléique catalytique A d'un deuxième polynucléotide PA, en formant ainsi un acide nucléique catalytique B qui clive le deuxième polynucléotide PA pour ainsi libérer l'acide nucléique catalytique A du deuxième polynucléotide PA qui est en conséquence capable de passer à travers la barrière perméable ; et
la détection du clivage de l'un quelconque dudit polynucléotide PA et/ou PB indique la présence de la cible dans l'échantillon.

8. Procédé selon la revendication 7, dans lequel :
(i) chaque polynucléotide PC comprend un oligonucléotide de partzyme B2 et un substrat d'acide nucléique catalytique B qui peut être clivé par l'acide nucléique catalytique A,
l'acide nucléique catalytique A libéré clive le polynucléotide PC pour ainsi libérer l'oligonucléotide de partzyme B2 du polynucléotide PC, et
la détection du clivage d'un quelconque polynucléotide PC indique la présence de la cible dans l'échantillon ; ou
(ii) chaque polynucléotide PC comprend un oligonucléotide de partzyme B2 et un substrat d'acide nucléique catalytique A, et
si la cible est présente dans l'échantillon, le bras substrat de chacun des oligonucléotides de partzyme C1 et C2 s'hybride au substrat d'acide nucléique catalytique A du polynucléotide PC et au moins un des oligonucléotides de partzyme C1 et C2 s'hybride à la cible en formant l'acide nucléique catalytique C qui clive le polynucléotide PC pour ainsi libérer l'oligonucléotide de partzyme B2 du polynucléotide PC ; ou
(iii) si la cible est présente dans l'échantillon, la cible s'hybride à un polynucléotide PC pour former le duplex d'acide nucléique qui comprend un site de reconnaissance et de clivage pour l'enzyme catalytique D,
le site de clivage pour l'enzyme catalytique D n'est pas compris au sein de l'oligonucléotide de partzyme B2 du polynucléotide PC, et
l'enzyme catalytique D se lie à et clive le duplex d'acide nucléique au niveau du site de clivage pour ainsi libérer l'oligonucléotide de partzyme B2 du polynucléotide PC.

9. Procédé selon la revendication 7 ou la revendication 8, dans lequel :
l'acide nucléique catalytique C est une enzyme d'acide nucléique à composants multiples (MNAzyme), et
les deux oligonucléotides de partzyme C1 et C2 sont capables de s'auto-assembler pour former la MNAzyme uniquement en présence de la cible,
dans lequel les deux oligonucléotides de partzyme C1 et C2 s'hybrident chacun à la cible pendant l'auto-assemblage pour former la MNAzyme.

10. Composition pour la détection d'une cible dans un échantillon, la composition comprenant :
une population de polynucléotide A (PA) et une population de polynucléotide B (PB), dans lesquelles :
chaque polynucléotide PA comprend un acide nucléique catalytique A et un substrat d'acide nucléique catalytique A,
chaque polynucléotide PB comprend un acide nucléique catalytique B et un substrat d'acide nucléique catalytique B,
l'acide nucléique catalytique A est capable de modifier de manière catalytique le substrat d'acide nucléique catalytique B et est incapable de modifier de manière catalytique le substrat d'acide nucléique catalytique A, et
l'acide nucléique catalytique B est capable de modifier de manière catalytique le substrat d'acide nucléique catalytique A et est incapable de modifier de manière catalytique le substrat d'acide nucléique catalytique B, le PA et/ou le PB sont mobiles dans la composition ;
une barrière sélectivement perméable capable de physiquement séparer le PA du PB ;
et
(A) l'acide nucléique catalytique A est un ADNzyme 10-23 et l'acide nucléique catalytique B est un ADNzyme 8-17 ; et
le substrat d'acide nucléique catalytique A est un substrat d'ADNzyme 8-17 et le substrat d'acide nucléique catalytique B est un substrat d'ADNzyme 10-23 ; ou
(B) l'acide nucléique catalytique A est un ADNzyme 8-17 et l'acide nucléique catalytique B est un ADNzyme 10-23 ; et
le substrat d'acide nucléique catalytique A est un substrat d'ADNzyme 10-23 et le substrat d'acide nucléique catalytique B est un substrat d'ADNzyme 8-17.

11. Composition comprenant :
(i) une population de polynucléotide A (PA), une population de polynucléotide B (PB) et une population de polynucléotide C (PC), dans lesquelles
chaque polynucléotide PA comprend un acide nucléique catalytique A et un substrat d'acide nucléique catalytique A, et est mobile dans la composition,
chaque polynucléotide PB comprend un oligonucléotide de partzyme B1 et un substrat d'acide nucléique catalytique B,
chaque polynucléotide PC comprend un oligonucléotide de partzyme B2,
dans laquelle :
un bras substrat de l'oligonucléotide de partzyme B1 est capable de s'hybrider au substrat d'acide nucléique catalytique A,
un bras substrat de l'oligonucléotide de partzyme B2 est capable de s'hybrider au substrat d'acide nucléique catalytique A, et
l'acide nucléique catalytique A est capable de modifier de manière catalytique le substrat d'acide nucléique catalytique B et est incapable de modifier de manière catalytique le substrat d'acide nucléique catalytique A ;
(ii) un oligonucléotide facilitateur d'assemblage capable de s'hybrider à un bras capteur de l'oligonucléotide de partzyme B1 et à un bras capteur de l'oligonucléotide de partzyme B2 ;
(iii) une barrière sélectivement perméable capable de physiquement séparer le PA du PB, où les PA et PB sont incapables de pénétrer la barrière sélectivement perméable,
dans laquelle une hybridation du bras substrat de l'oligonucléotide de partzyme B1 et du bras substrat de l'oligonucléotide de partzyme B2 au substrat d'acide nucléique catalytique A, et une hybridation du bras capteur de l'oligonucléotide de partzyme B1 et du bras capteur de l'oligonucléotide de partzyme B2 au facilitateur d'assemblage, forme un acide nucléique catalytique B capable de cliver le substrat d'acide nucléique catalytique A ;
dans laquelle
(A) l'acide nucléique catalytique A est un ADNzyme 10-23 et l'acide nucléique catalytique B est un ADNzyme 8-17 ; et
le substrat d'acide nucléique catalytique A est un substrat d'ADNzyme 8-17 et le substrat d'acide nucléique catalytique B est un substrat d'ADNzyme 10-23 ; ou
(B) l'acide nucléique catalytique A est un ADNzyme 8-17 et l'acide nucléique catalytique B est un ADNzyme 10-23 ; et
le substrat d'acide nucléique catalytique A est un substrat d'ADNzyme 10-23 et le substrat d'acide nucléique catalytique B est un substrat d'ADNzyme 8-17 ;
et dans laquelle :
(i) chaque polynucléotide PC comprend un oligonucléotide de partzyme B2 et ledit substrat d'acide nucléique catalytique B capable d'être clivé par l'acide nucléique catalytique A ; ou
(ii) chaque polynucléotide PC comprend un oligonucléotide de partzyme B2 et ledit substrat d'acide nucléique catalytique A.
